# FASCICULE DE BREVET EUROPEEN

(11) **EP 1 246 837 B1**
(45) Date de publication et mention de la délivrance du brevet: **26.10.2005**
(21) Numéro de dépôt: 01903924.7
(22) Date de dépôt: 11.01.2001
(51) Int. Cl.: C07K 7/02, C07K 14/575, C08L 77/10, C08L 77/02

(54) **OLIGOMERES DE MIMES CONTRAINTS NON PEPTIDIQUES DE DIPEPTIDES OU DE TRIPEPTIDES, ET LEUR UTILISATION POUR LA SYNTHESE DE PROTEINES ET POLYPEPTIDES ARTIFICIELS**
OLIGOMERE VON KONFORMATIONSBESCHRÄNKTEN, NICHT-PEPTIDISCHEN DI- ODER TRIPEPTID-MIMETIKA, UND IHRE VERWENDUNG ZUR SYNTHESE VON KÜNSTLICHEN POLYPEPTIDEN UND PROTEINEN
OLIGOMERS OF NONPEPTIDE RESTRICTED MIMETICS OF DIPEPTIDES OR TRIPEPTIDES AND THE USE THEREOF IN THE SYNTHESIS OF SYNTHETIC PROTEINS AND POLYPEPTIDES

(30) Priorité: 11.01.2000 FR 0000288
(43) Date de publication de la demande: 09.10.2002
(73) Titulaire: CENTRE NATIONAL DE LA RECHERCHE SCIENTIFIQUE (CNRS), 75016 Paris (FR)
(72) Inventeur: AMBLARD, Muriel, F-34430 Saint Jean de Vedas (FR); MARTINEZ, Jean, F-34720 Caux (FR); BERGE, Gilbert, F-34090 Montpellier (FR)
(74) Mandataire: Sueur, Yvette
(86) Numéro de dépôt international: PCT/FR2001/000088
(87) Numéro de publication internationale: WO 2001/051506

(56) Documents cités:
- WO-A-97/30975
- WO-A-99/48913
- DE-A- 3 716 629
- STIGERS KIMBERLY D ET AL: "Designed molecules that fold to mimic protein secondary structures." CURRENT OPINION IN CHEMICAL BIOLOGY, vol. 3, no. 6, décembre 1999 (1999-12), pages 714-723, XP002148554 ISSN: 1367-5931
- GELLMAN, SAMUEL H.: "FOLDAMERS A MANIFESTO" ACC CHEM RES (1998) 31(4) 173-180, XP002148555
- SOTH MICHAEL J ET AL: "Unnatural oligomers and unnatural oligomer libraries." CURRENT OPINION IN CHEMICAL BIOLOGY, vol. 1, no. 1, juin 1997 (1997-06), pages 120-129, XP002173277 ISSN: 1367-5931
- SEEBACH, DIETER ET AL.: "BETA - PEPTIDES A SURPRISE AT EVERY TURN" CHEM COMMUN (CAMBRIDGE) (1997) (21) 2015-2022, XP002148556
- AMBLARD MURIEL ET AL: "Synthesis and characterization of bradykinin B2 receptor agonists containing constrained dipeptide mimics." JOURNAL OF MEDICINAL CHEMISTRY, vol. 42, no. 20, 7 octobre 1999 (1999-10-07), pages 4193-4201, XP002148557 ISSN: 0022-2623 cité dans la demande
- NAGAI U ET AL: "BICYCLIC TURNED DIPEPTIDE (BTD) AS A BETA-TURN MIMETIC;ITS DESIGN SYNTHESIS AND INCORPORATION INTO BIOACTIVE PEPTIDES" TETRAHEDRON,NL,ELSEVIER SCIENCE PUBLISHERS, AMSTERDAM, vol. 49, no. 17, 23 avril 1993 (1993-04-23), pages 3577-3592, XP000576111 ISSN: 0040-4020
- PITT N ET AL: "Synthesis and Structure of a Small Macrocyclic Hexapeptide Model for Antiparallel beta-Sheet Containing Two Restrained 2-(3'-Aminopropynyl)-aniline Reverse-Turn Mimetics" TETRAHEDRON LETTERS,NL,ELSEVIER SCIENCE PUBLISHERS, AMSTERDAM, vol. 40, no. 19, 7 mai 1999 (1999-05-07), pages 3811-3814, XP004163839 ISSN: 0040-4039

## Description

La présente invention a pour objet des oligomères d'acides aminés, un procédé pour leur préparation, leur utilisation pour la synthèse de polypeptides et de protéines artificiels, et les polypeptides et protéines artificiels obtenus.

Les protéines et les polypeptides sont des polymères obtenus par couplage d'un certain nombre d'acides α-aminés identiques ou différents dans un ordre donné. Ils présentent de nombreuses propriétés fort intéressantes, mais leur structure les rend fragiles et ils sont facilement dégradés.

On a cherché à augmenter la stabilité en remplaçant tout ou partie des acides α-aminés dans la séquence d'une protéine ou d'un polypeptide naturel par des acides β-aminés (K. Gademan, et al, Helvetica Chimica Acta Vol. 82 (1999), pp. 1-11). Cette substitution a effectivement permis d'améliorer la stabilité, l'activité et la structuration des produits obtenus. Les β-aminoacides ne sont cependant pas des entités contraintes et sont proches des structures des α-aminoacides.

Des polyamides obtenus par polymérisation d'acides aminés possédant un noyau pyrrole ou un noyau imidazole sont décrits dans WO 97/30975. Des polymères obtenus par polymérisation de divers β-aminoacides sont décrits notamment par Stigers Kimberley et al., ["Designed molécules that fold to mimic protein secondary structures", Current Opinion in Chemical Biology, Vol. 3, n° 6, déc. 1999 (714-723)], par Gellman, Samuel H, ["Foldamers A Manifesto", Acc Chem. Res. (1998) 31(4) 173-180], ou par Seebach, Dieter, et al ["Beta-peptides A surprise at every turn", Chem. Commun. (Cambridge) (1997) (21) 2015-2222]. WO 99/48913 décrit des groupements présentant des propriétés de mimes de peptides. Il est également connu que divers groupements présentent des propriétés de mimes contraints de dipeptides ou de tripeptides. De tels groupements sont décrits notamment par Obrecht, et al (Advances in Medicinal Chemistry, vol. 4, p. 1-68, 1999), et par Nagai et al. (Tetrahedron, Vol 49, n°17, p.3577-3592, 1993). Les composés contenant de tels groupements sont décrits comme étant utiles pour orienter les chaînes peptidiques et mimer les conformations contraintes en coude β dans les peptides.

Le but de l'invention est l'obtention de polypeptides artificiels et de protéines artificielles analogues de polypeptides naturels et de protéines naturelles, dans lesquels les parties peptidiques structurées, en particulier en hélice α, sont remplacées par des oligomères non peptidiques. Lesdits polypeptides artificiels et protéines artificielles sont plus stables que leurs analogues naturels dont ils diffèrent par la structure, notamment par la taille.

L'invention a ainsi pour objet des oligomères d'amino-acides dont les unités récurrentes sont des mimes contraints non peptidiques de dipeptides ou de tripeptides, un procédé pour leur obtention, leur utilisation pour l'élaboration de polypeptides artificels et de protéines artificielles, et les polypeptides et protéines obtenus.

Un oligomère de la présente invention est représenté par l'une des formules générales :

R₁₋(NR'-A-CO)ₘ-OR₂ (I) ou R₁-(NR'-A-CO)ₘ-NR'₂R"₂ (I')

dans lesquelles :
- l'unité -NR'-A-CO- représente un mime contraint non peptidique d'un fragment dipeptide ou tripeptide, inducteur de coude β, étant entendu que A représente un groupe hétérocyclique aromatique ou non, monocyclique ou polycyclique condensé ou non, ou que le groupe -NR'-A-CO- représente un groupe hétérocyclique aromatique ou non, monocyclique ou polycyclique condensé ou non ;
- m est compris entre 2 et 40 ;
- R¹ représente un groupe acyle R₃-CO- ou un groupe R₃-O-CO- dans lesquels R₃ représente un groupe benzyle, un groupe tert-butyle ou un groupe 9-fluorénylméthyle ;
- R₂ représente H, un groupe alkyle, (de préférence un méthyle ou un éthyle) ou un groupe benzyle ;
- R'₂ et R"₂ représentent indépendamment l'un de l'autre H, un groupe alkyle, (de préférence un méthyle ou un éthyle) ou un groupe benzyle ;
- R' représente un atome d'hydrogène ou bien
- R' forme un groupe monocyclique ou un groupe polycyclique avec l'atome N et le groupe A, ledit groupe polycyclique étant un groupe condensé ou non.

Le groupe -NR'-A-CO- peut comprendre un centre asymétrique, qui peut être de configuration R ou de configuration S.

Un oligomère de l'invention peut être constitué d'unités récurrentes -NR'-A-CO- qui sont toutes identiques. Il peut également être constitué par des unités différentes qui répondent à la définition ci-dessus.

Comme exemples de groupements -NR'-A-CO- qui présentent des propriétés de mimes contraints non peptidiques de dipeptide ou de tripeptide et qui sont inducteurs de coude β, on peut citer les groupes suivants, dans lesquels :
- R, et le cas échéant R₄, sont choisis indépendamment l'un de l'autre parmi les groupes constituants les chaînes latérales des α-aminoacides, par exemple H, CH₃-, (CH₃)₂CH-, CH₃-(CH₂)₃- ou C₆H₅-CH₂- ;
- R₅ et R₆ représentent indépendamment l'un de l'autre H, CH₃-, ou C₆H₅-CH₂- ;
- R₇ représente H ou un phényle ;
- R₈ représente H, CH₃-, C₂H₅- ou C₆H₅-CH₂- ;
- les substituants X et Z sont définis de manière spécifique pour chaque composé qui les contient, et
- Me représente un groupe méthyle ; et
- n représente le nombre d'atomes de carbone, et est égal à 0, 1, 2 ou 3.

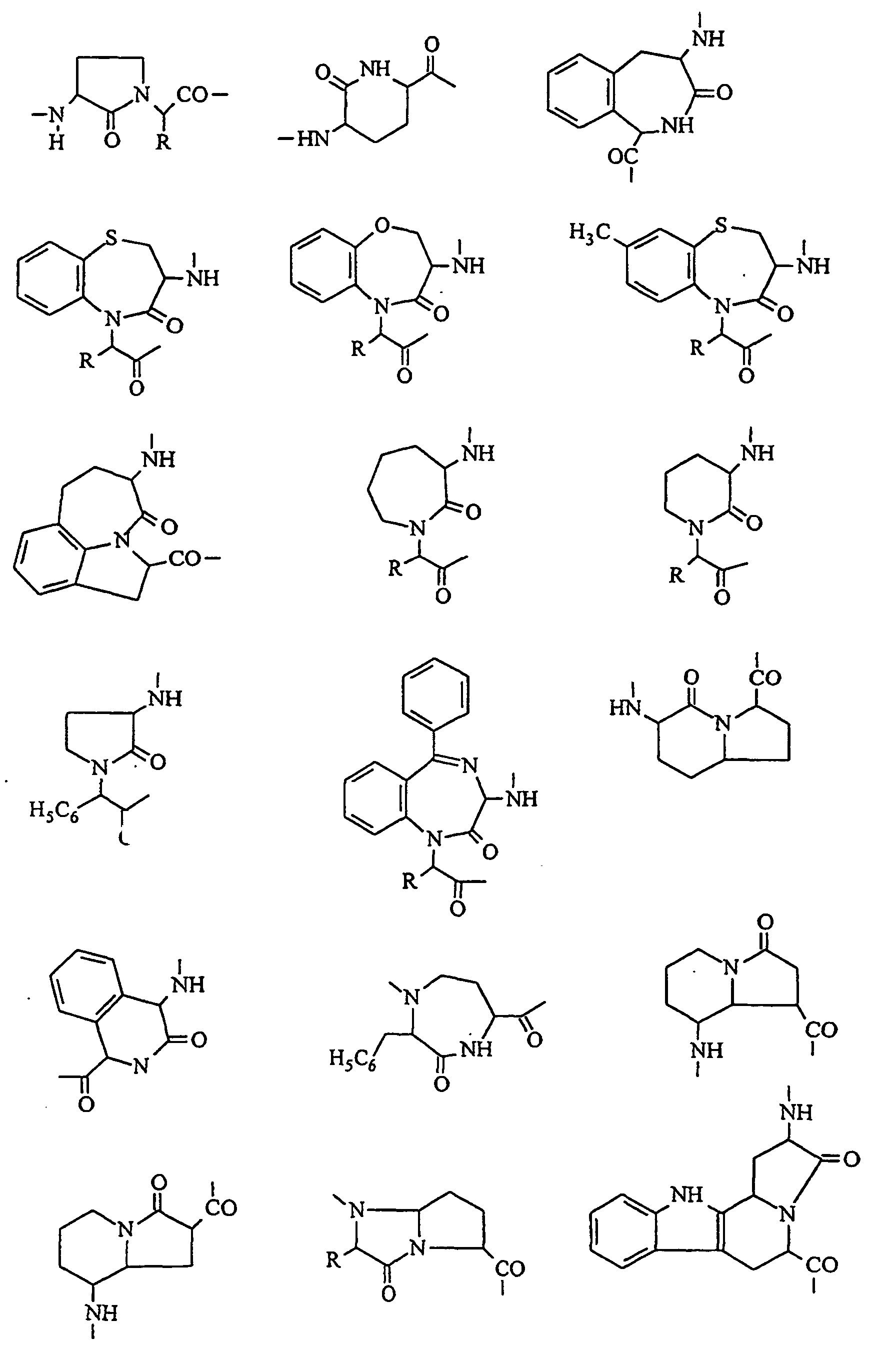

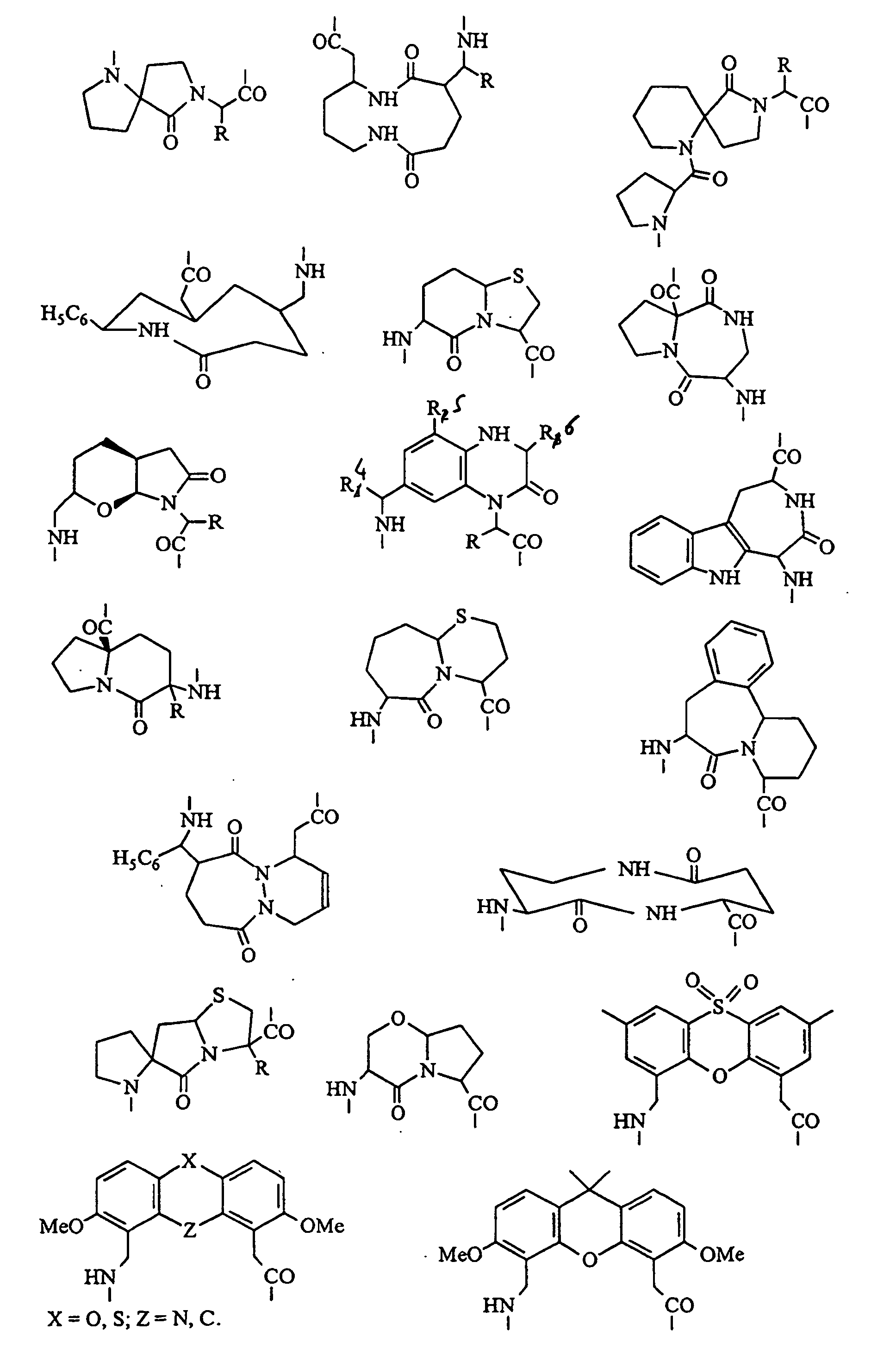

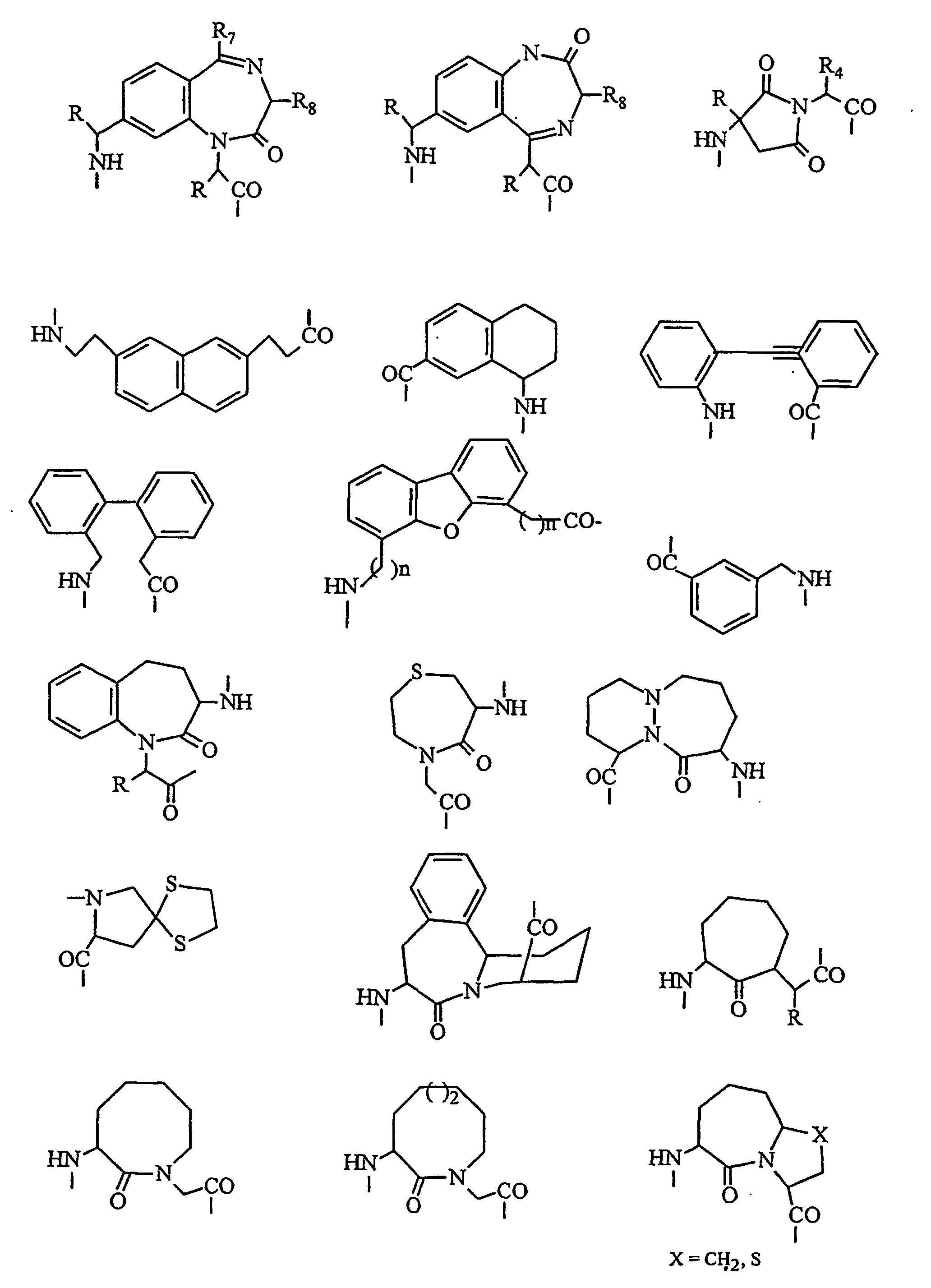

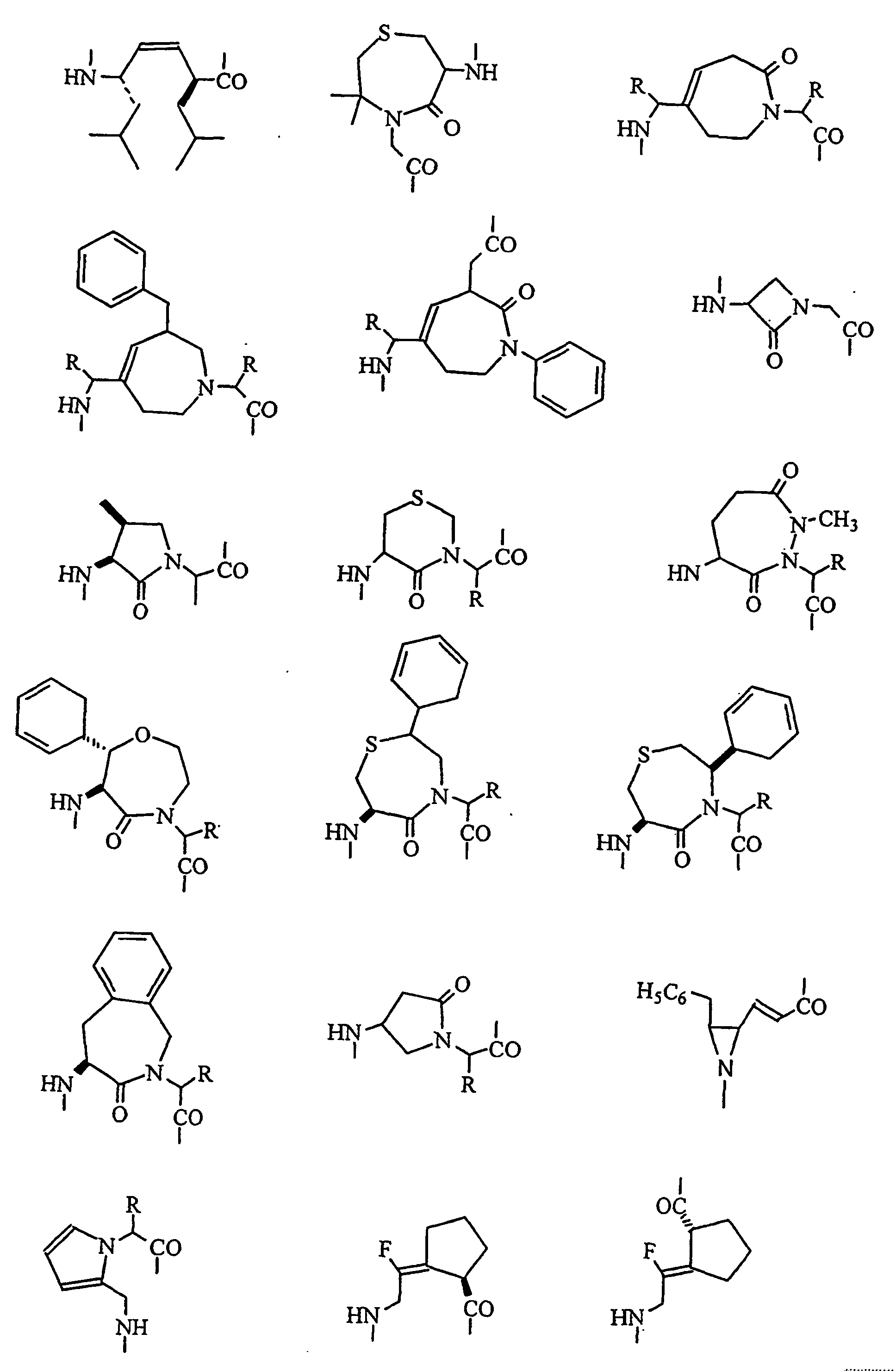

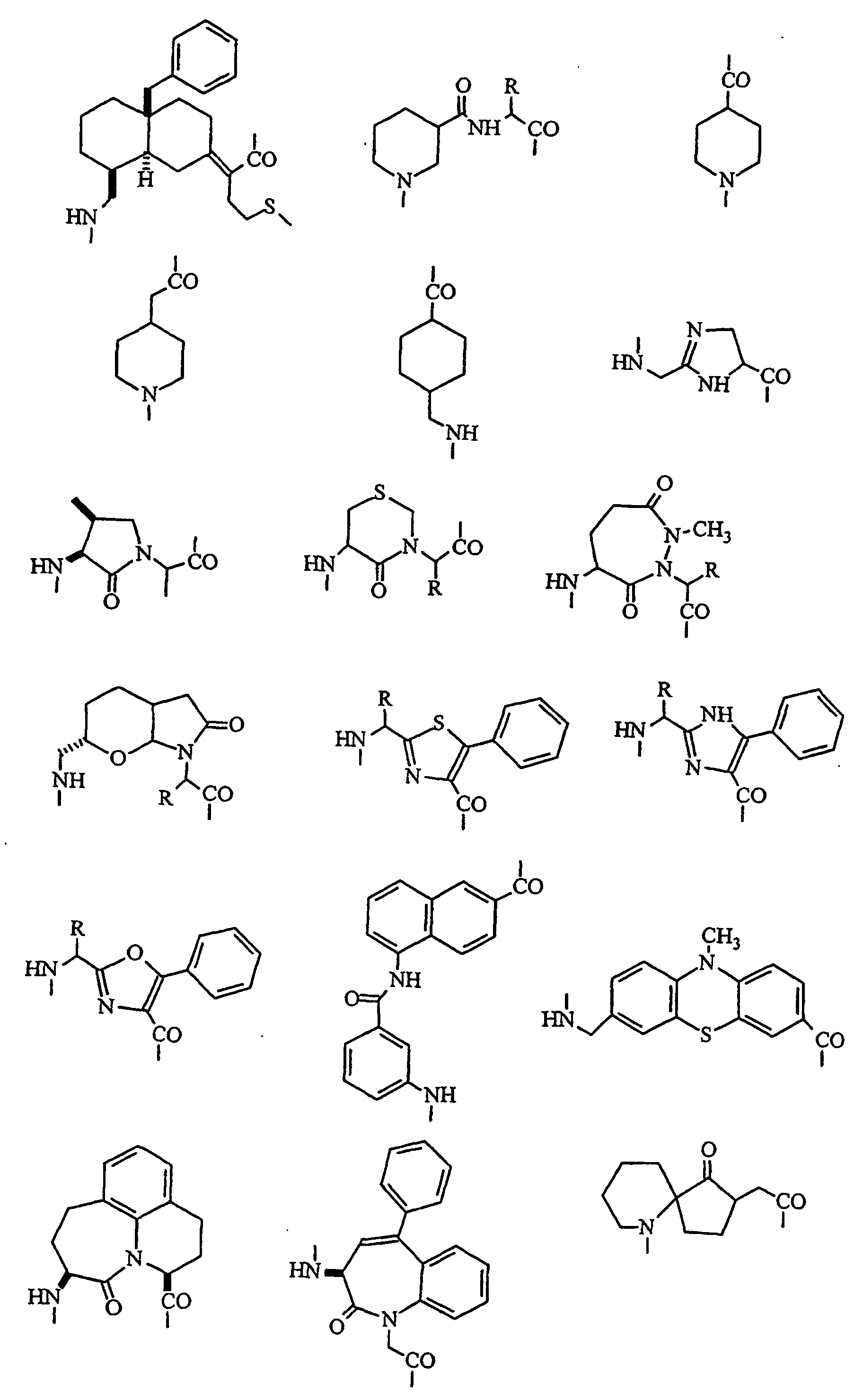

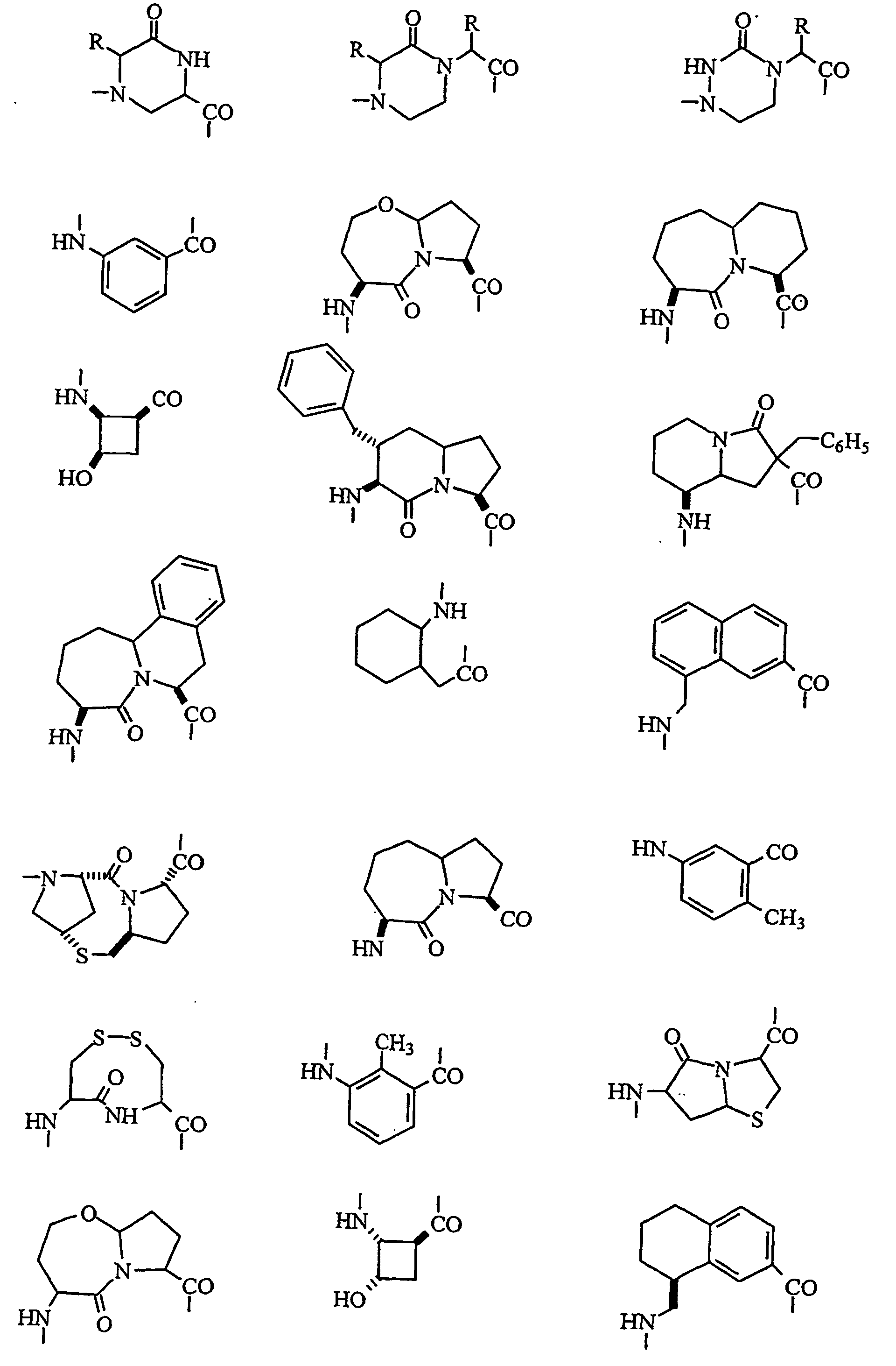

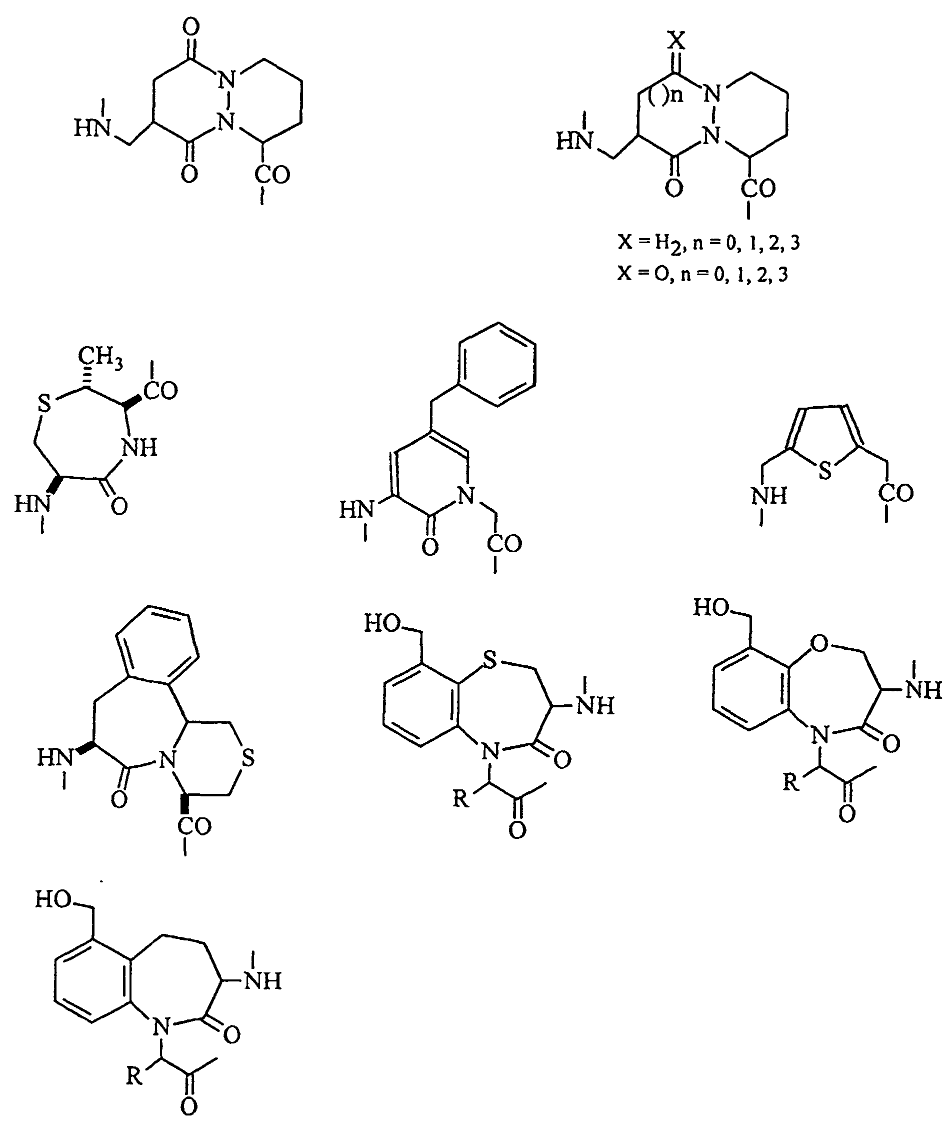

Dans les composés ci-dessus comprenant des centres asymétriques, lesdits centres asymétriques peuvent être de configuration R ou S.

Un oligomère (I) ou (I') est obtenu par polymérisation d'au moins un acide aminé qui constitue un mime contraint non peptidique d'un dipeptide ou d'un tripeptide, qui est inducteur de coude β, et qui répond à la formule NHR'-A-CO-OH (II) dans laquelle les groupes R' et A ont la même signification que dans l'oligomère (I) ou (I'). A titre d'exemples de composés (II), on peut citer les composés ci-dessous :

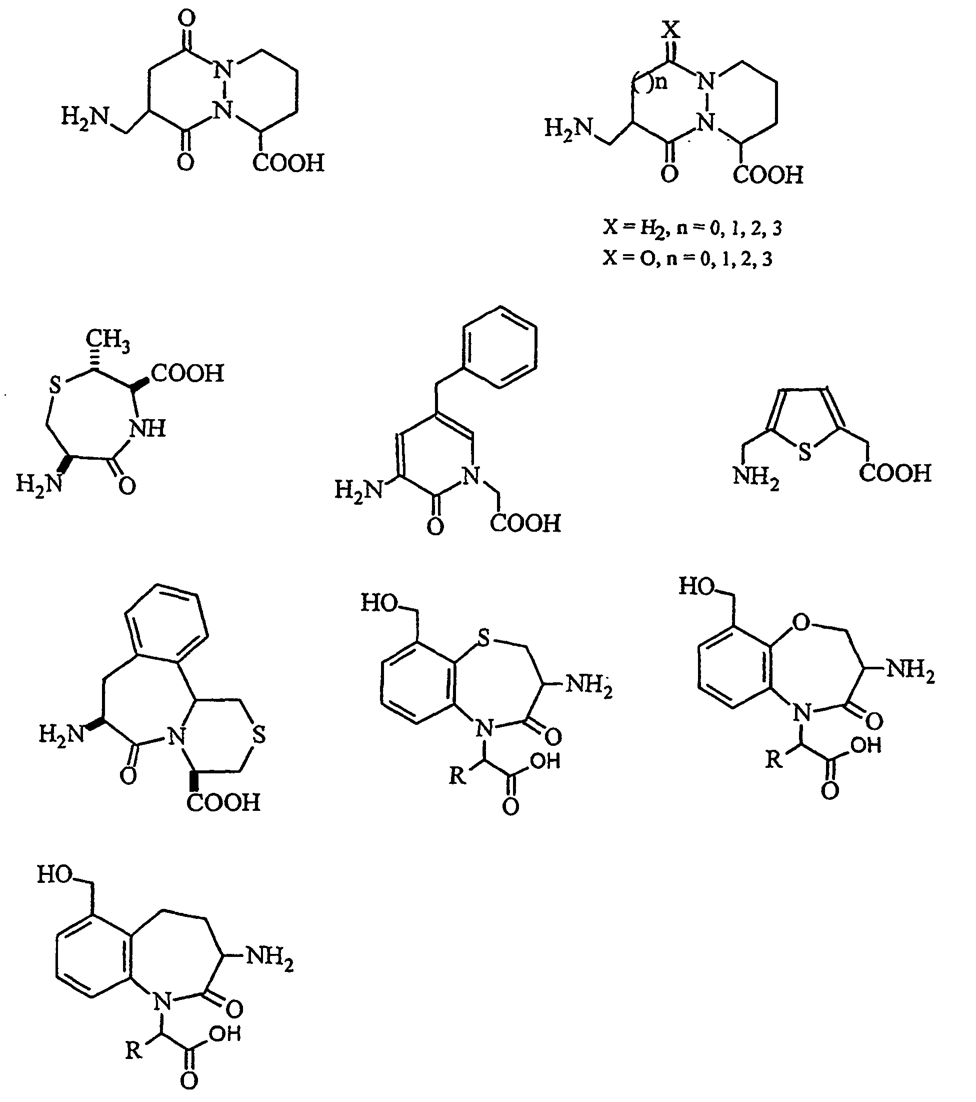

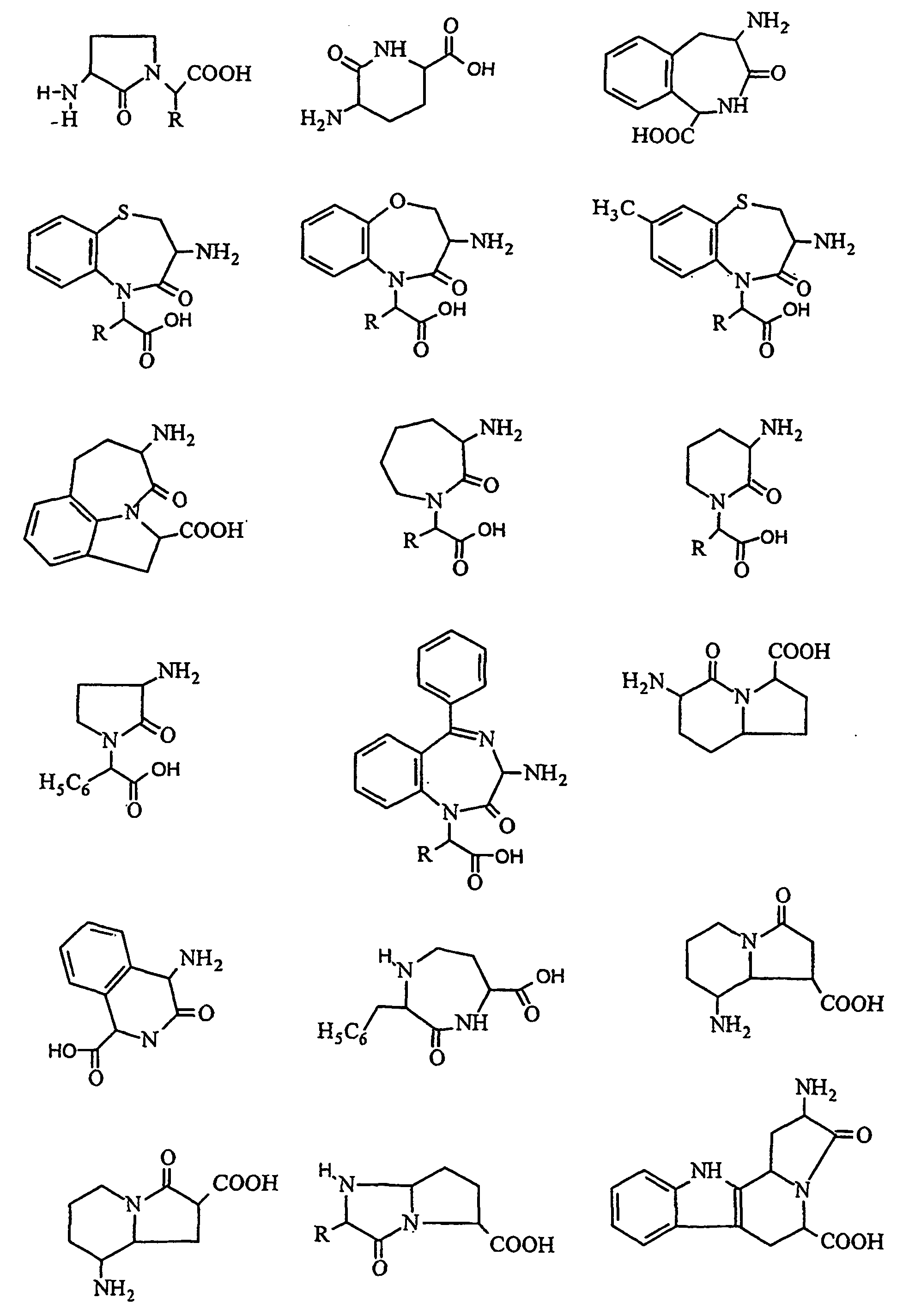

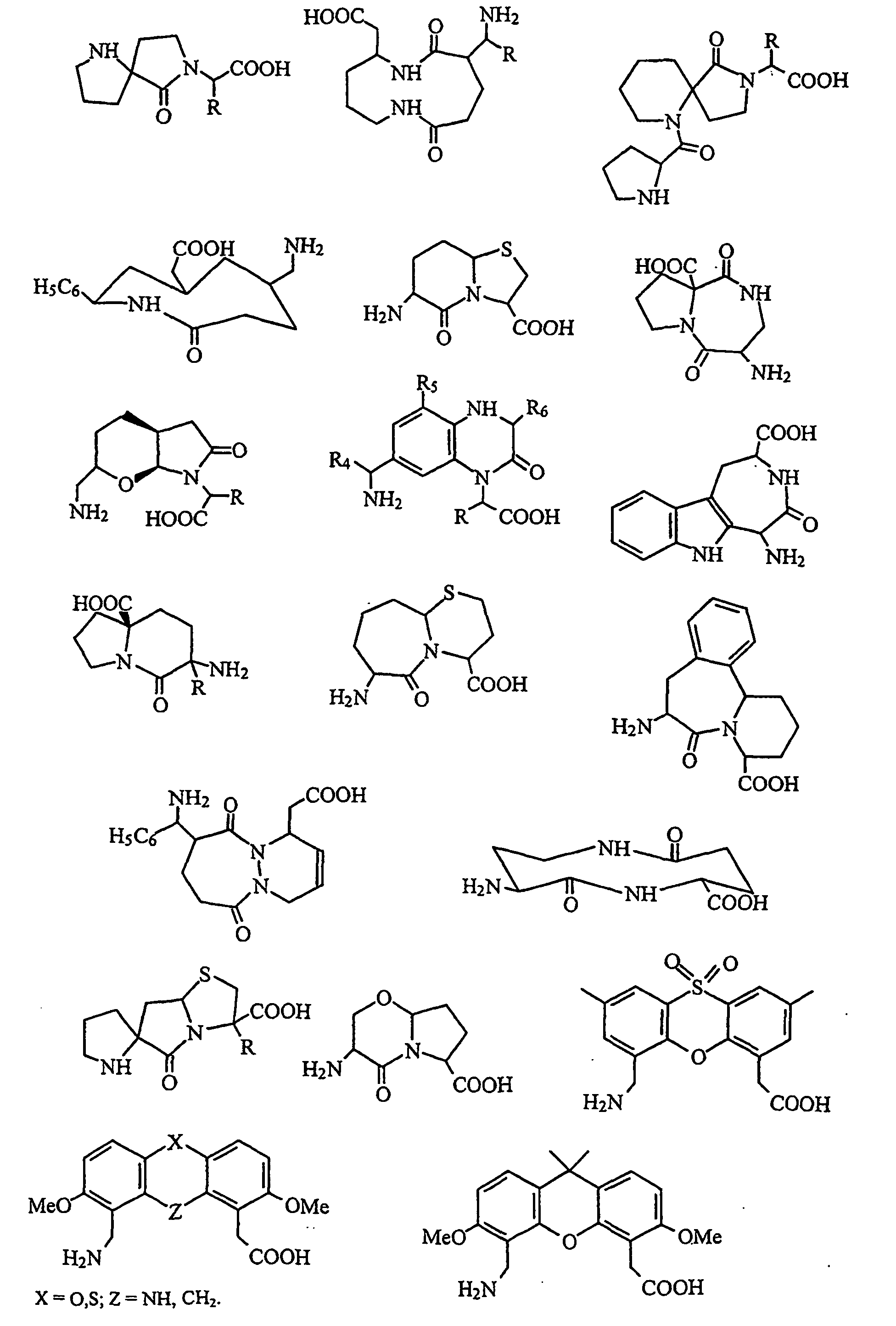

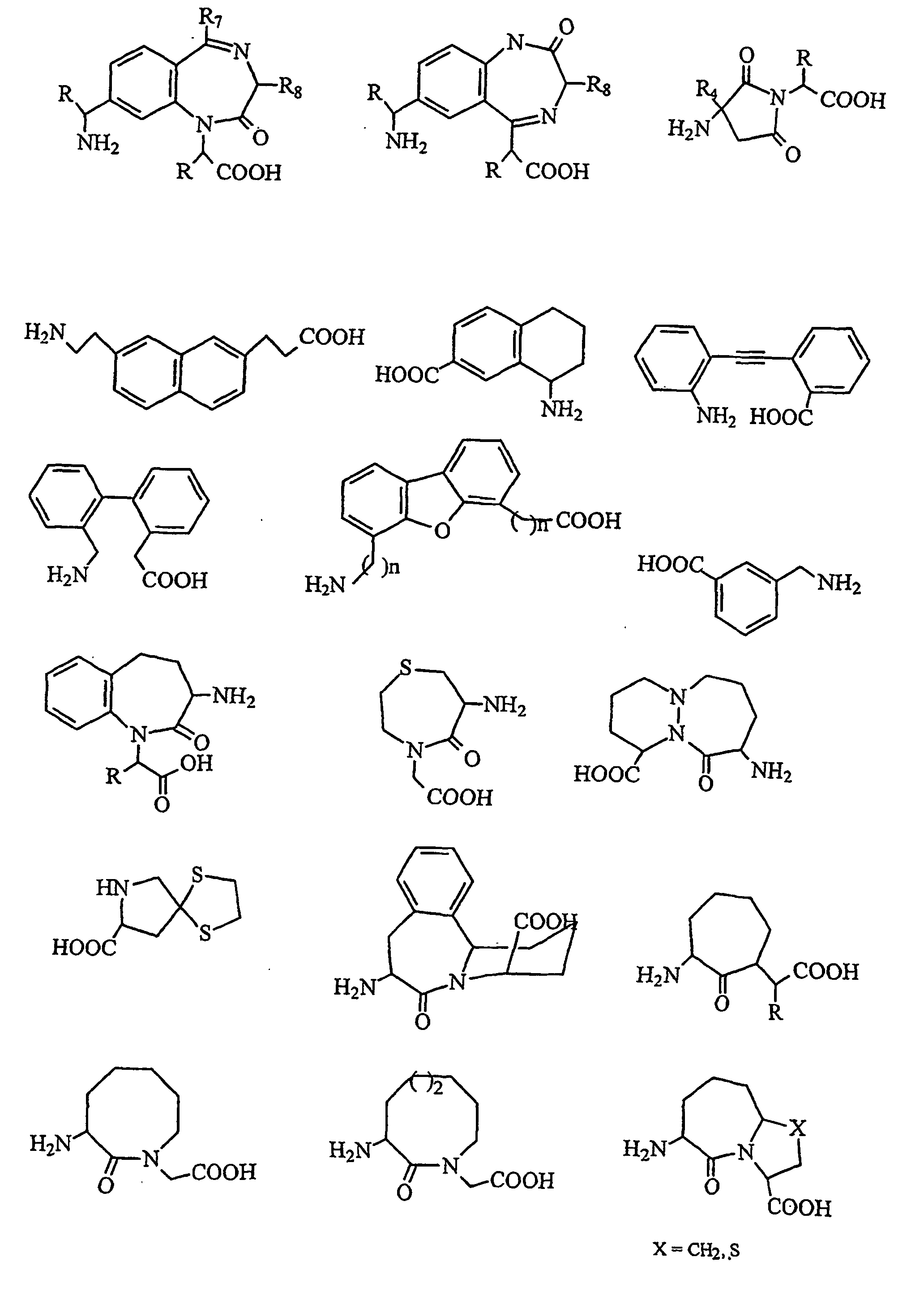

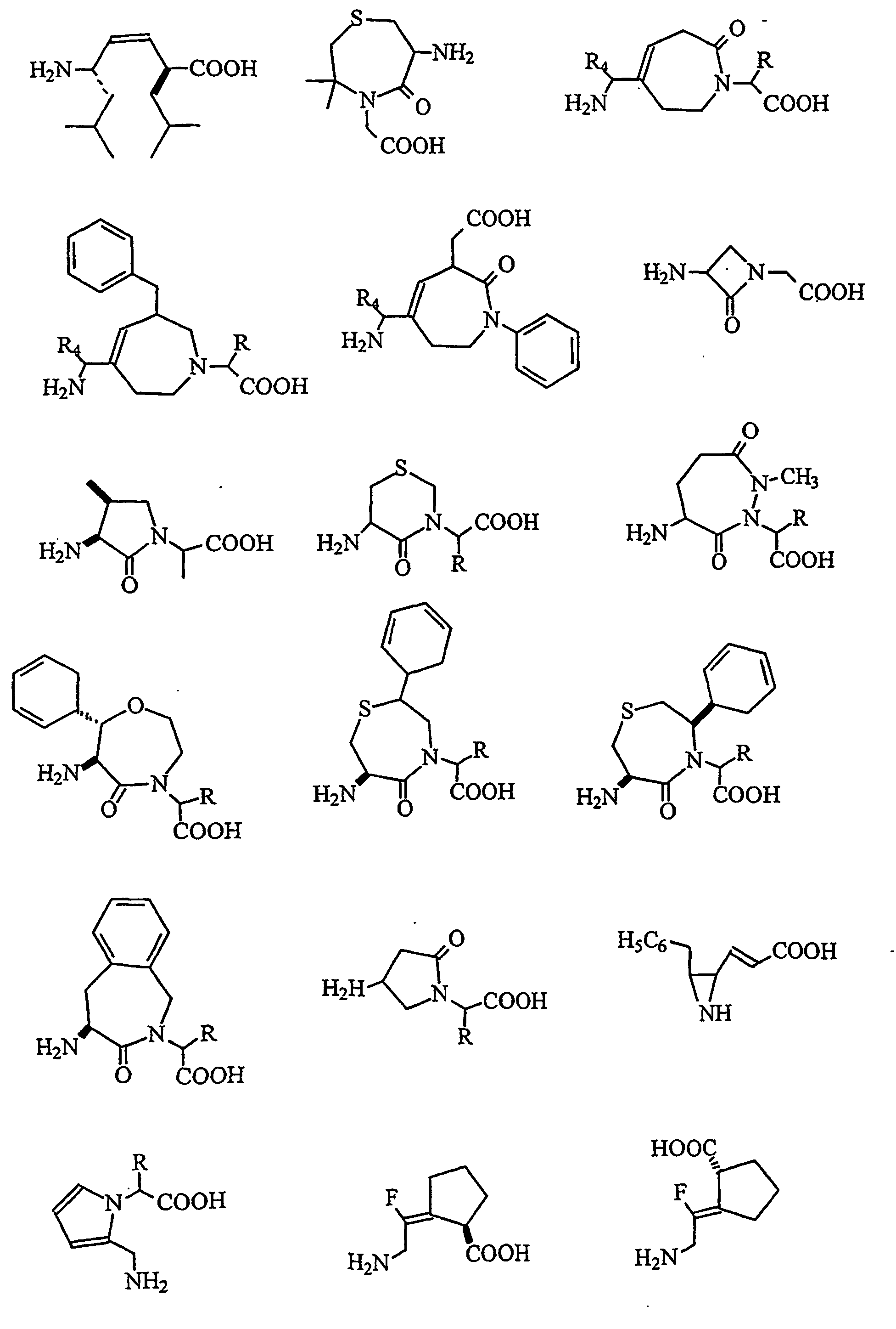

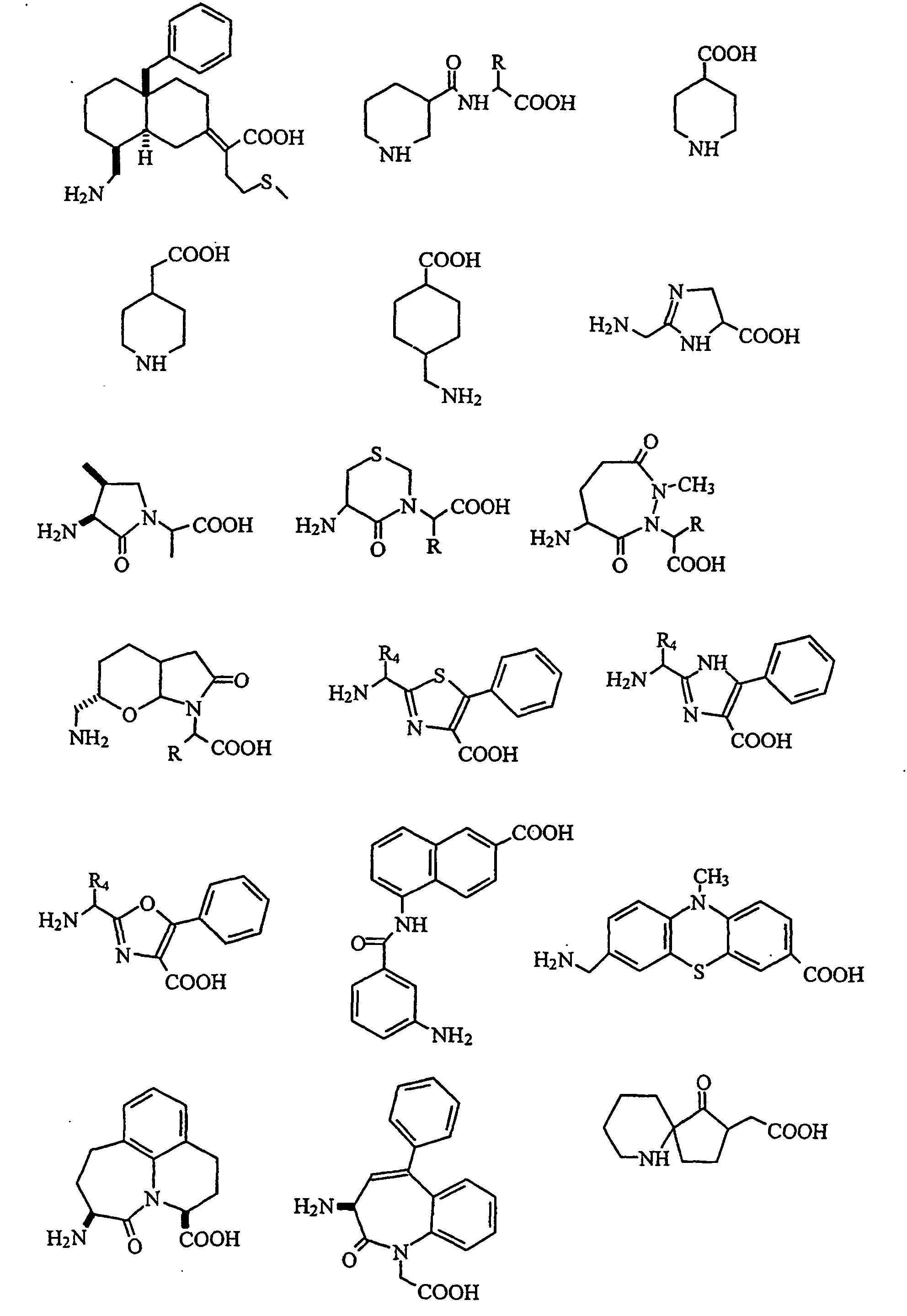

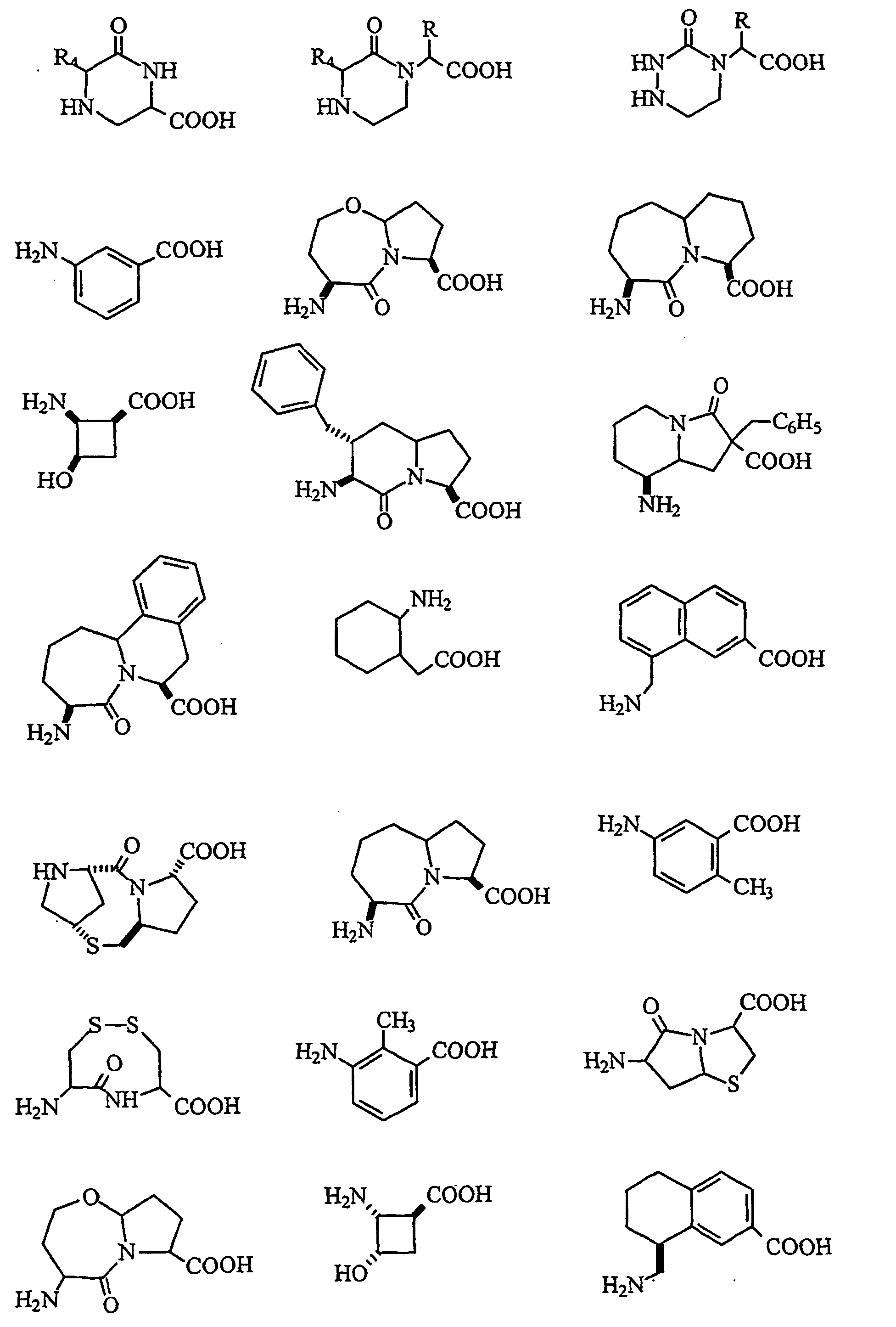

Dans les composés ci-dessus, les substituants R, R₄ à R₈, X et Z ont la signification indiquée ci-dessus.

Dans les composés ci-dessus comprenant des centres asymétriques, lesdits centres asymétriques peuvent être de configuration R ou S.

Ces monomères peuvent être synthétisés par des procédés décrits dans l'art antérieur. Par exemple, Amblard, et al., J. Med. Chem., 1999, 42, 4193-4201, décrivent la synthèse de la (3S)[amino]-5-(carbonylméthyl)-2,3-dihydro-1,5-benzothiazépin-4(5H)-one et d'autres monomères. D'autres procédés sont décrits dans Obrecht, précité.

La polymérisation des monomères (II) peut être effectuée en solution selon un mode opératoire classique pour les synthèses peptidiques. On met en solution un monomère (II) protégé sur sa fonction acide, un monomère (II) identique ou non au premier cité et protégé sur sa fonction amine et un agent de couplage, ce qui provoque la formation d'une liaison amide entre les deux monomères. Ensuite, on déprotège sélectivement la fonction N-terminale de l'oligomère obtenu et on condense un autre monomère (II) N-protégé avec cet oligomère pour obtenir un oligomère protégé constitué de trois unités monomères. La synthèse est ainsi poursuivie par des étapes successives de déprotection sélective de la fonction N-terminale de l'oligomère, suivie d'un couplage avec un monomère N-protégé, jusqu'à, l'obtention d'un oligomère de la taille désirée. L'oligomère final peut être déprotégé in fine soit sélectivement sur la fonction carboxylique, soit sur la fonction amine, soit totalement.

Dans un oligomère (I) ou (I'), le substituant terminal R₁ peut être obtenu en utilisant pour la dernière étape de couplage, un monomère N-protégé par un groupe R₁, ou en effectuant une réaction d'acylation avec le réactif approprié.

Lorsque l'un au moins des substituants R'₂ ou R"₂ est différent de H, le groupe terminal -NR'₂R"₂ d'un oligomère (I') est obtenu en choisissant comme premier monomère N-protégé libre sur sa fonction acide, un composé résultant de la réaction d'un composé (II) avec une amide HNR'₂R"₂, répondant (avant protection de la fonction amine) à la formule NHR'-A-CO-HNR'₂R"₂.

Lorsque le substituant R₂ d'un oligomère (I) est différent de H, le groupe terminal OR₂ est obtenu par le choix du groupe protecteur de la fonction acide dans le premier monomère.

Un oligomère (I) ou (I') peut également être obtenu par un procédé de polymérisation en phase solide, selon une stratégie classique de synthèse peptidique : une résine portant des substituants amine est fonctionnalisée à l'aide d'un acide aminé, puis le fragment ainsi fixé sur la résine est allongé depuis le côté C-terminal vers le côté N-terminal, le fragment final étant protégé sur son extrémité N-terminale avant d'être séparé de la résine. Un tel procédé en phase solide permet d'obtenir les oligomères (I) et (I') dans lesquels les substituants respectifs R₂, R'₂ et R"₂ sont H.

De manière plus précise, un procédé de polymérisation en phase solide pour l'obtention d'un oligomère de l'invention comprend les étapes suivantes :
a) on fonctionnalise une résine support portant des substituants amine par un composé H-NR'-A-CO-OH (II') qui répond à la définition donnée pour (II) et dans lequel le groupe amino a été protégé au préalable ;
b) on allonge le fragment ainsi fixé sur la résine support à partir du côté C-terminal vers la côté N-terminal par (n-2) réactions successives de couplage de monomère (II), ledit monomère (II) étant utilisé en excès, différents monomères (II) pouvant être utilisés dans les étapes de couplage successives ;
c) on effectue une dernière réaction de couplage d'un monomère protégé sur sa fonction N-terminale par un groupe R₁ stable dans les conditions dans lesquelles l'oligomère doit être séparé du support ;
d) on sépare l'oligomère de la résine support.

Dans le procédé de polymérisation en phase solide, chaque étape de couplage d'un composé (II) [sur la résine support initiale dans l'étape a) ou sur la résine portant un fragment issu d'un composé (II) dans les étapes b) et c)] comprend la protection du groupe amino du composé (II), la réaction de couplage proprement dite en présence d'un agent de couplage, le lavage du produit obtenu après le couplage, puis la déprotection du groupe amino de l'unité -NR'-A-CO- fixée.

La protection du groupe amino de l'acide aminé peut être effectuée par exemple par un groupe *tert*-butyloxycarbonyle (désigné ci-après par Boc-) ou un groupe 9-fluorénylméthyloxycarbonyle (désigné ci-après par Fmoc) représenté par la formule 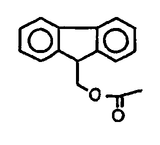 La protection est effectuée selon les procédés connus de l'art antérieur. Par exemple, la protection par le groupe Boc- peut être obtenue en faisant réagir l'acide aminé avec le di-tert-butylpyrocarbonate (BoC₂O).

Pour l'étape a), on peut utiliser comme résine support toute résine utilisée de manière classique dans les synthèses peptidiques. A titre d'exemple, on peut citer une résine 4-méthylbenzydrylamine.HCl (désignée ci-après par MBHA.HCl), ou une résine dite Merrifield, qui est un copolymère de styrène et de divinylbenzène fonctionnalisée par le chlorobenzyle. Les deux résines sont des résines commerciales distribuées notamment par les sociétés Novabiochem et Bachem. On peut également citer les résines PAL-PEG-PS, qui sont des copolymères 5-(4-aminométhyl-3,5-diméthoxyphénoxy)valéric acid - polyéthylène glycol - polystyrène.

L'agent de couplage peut être choisi parmi les agents de couplage classiquement utilisés en synthèse peptidique. On peut citer par exemple :

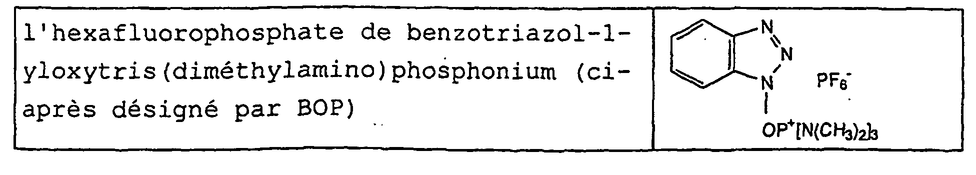

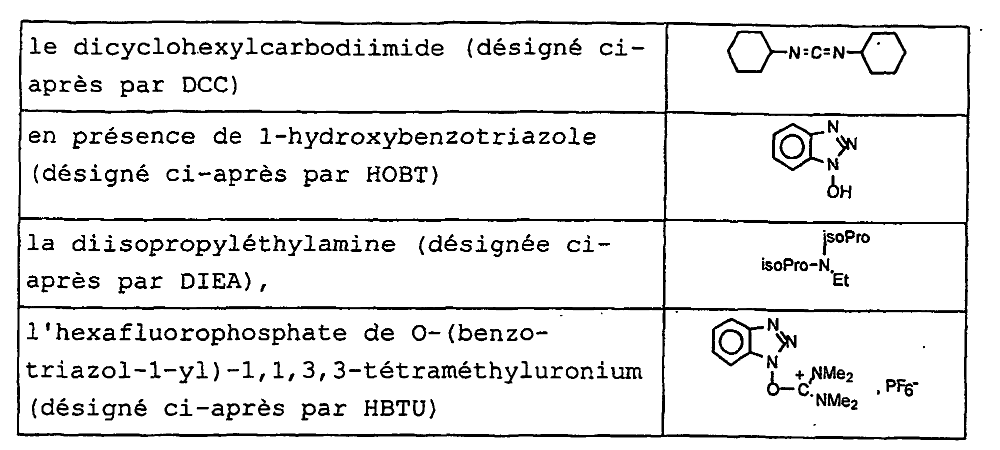

On peut également utiliser pour le couplage, les anhydrides symétriques préformés à partir d'un monomère (II) N-protégé et de la DCC.

Le lavage des produits obtenus après chaque phase de couplage peut être effectué à l'aide des solvants utilisés classiquement dans les synthèses peptidiques en phase solide. A titre d'exemple, on peut citer la diméthylformamide (DMF)-, le méthanol et le dichlorométhane (DCM).

Le réactif utilisé pour la déprotection du groupe amine après une étape de couplage dépend de l'agent de protection utilisé. Par exemple, si la protection est effectuée par un groupe Boc, la déprotection est effectuée avantageusement à l'aide d'une solution d'acide trifluoroacétique (TFA). Si la protection est effectuée par un groupe Fmoc-, la déprotection peut être effectuée par la pipéridine. De manière générale, les groupes protecteurs et les réactifs de déprotection utilisés de manière connue lors des synthèses peptidiques en phase solide peuvent être utilisés lors de la synthèse des oligomères de la présente invention.

L'oligomère peut avantageusement être séparé de la résine par traitement par un acide. A titre d'exemple, on peut citer l'acide trifluoroacétique ou l'acide fluorhydrique en présence d'anisole, suivant le type de résine utilisée. Dans ces conditions, la protection du groupe amino avant séparation de la résine est effectuée par un groupe uréthane stable dans les conditions acide de clivage ou par un groupe acyle stable dans les mêmes conditions. Lorsque la séparation est effectuée par l'acide trifluoroacétique, le groupe amino est protégé par exemple par un groupe Fmoc défini précédemment.

La préparation des oligomères en phase solide est réalisée de manière avantageuse dans un synthétiseur automatique utilisé de manière classique pour la synthèse des peptides sur support solide. Dans ce type d'appareil, la succession des différentes opérations de couplage, de lavage, de déprotection, est pilotée par un ordinateur.

Les inventeurs ont trouvé qu'un oligomère (I) ou (I') de l'invention, qui est obtenu par couplage de plusieurs molécules du même monomère acide aminé (II) ou par couplage de monomères (II) différents, forme une structure organisée et rigide, par exemple une structure en hélice, qui peut remplacer avantageusement le(s) fragment(s) structurant(s) en hélice α d'une protéine naturelle ou d'un polypeptide naturel.

Un objet de la présente invention est donc également constitué par une protéine artifielle ou un polypeptide artificiel analogue d'une protéine naturelle ou d'un polypeptide naturel, et par un procédé pour leur préparation.

Le procédé de préparation d'une protéine artificielle ou d'un polypeptide artificiel analogue d'une protéine naturelle ou d'un polypeptide naturel consiste à effectuer des réactions de couplage de synthèse peptidique en phase solide. Il est caractérisé en ce que, dans la succession des réactions de couplage des acides α-aminés constituant la protéine ou le polypeptide naturel, une ou plusieurs séquences d'acides α-aminés sont remplacées par un oligomère (I) ou (I') ayant une longueur équivalente à celle de la séquence d'acides α-aminés remplacée. Dans un cas particulier, la séquence d'acides α-aminés constituant l'hélice α de la protéine ou du peptide naturel est remplacée par un oligomère (I) ou (I').

L'oligomère est préparé au préalable selon le procédé de polymérisation en phase solide tel que décrit ci-dessus et il est utilisé sous une forme dans laquelle la fonction amine N-terminale est protégée par un groupe protecteur résistant aux conditions dans lesquelles l'oligomère sera séparé de la résine support. A titre d'exemple d'un tel groupe protecteur, on peut citer le groupe 9-fluorénylméthoxycarbonyle (Fmoc).

Une protéine ou un polypeptide artificiel analogue d'une protéine ou d'un polypeptide naturel donné comprend un ou plusieurs fragments structurants, par exemple en hélice, et un ou plusieurs fragments peptidiques. Il est caractérisé en ce que le ou les fragments peptidiques sont identiques à ceux de la protéine ou du polypeptide naturel correspondant, et en ce que le ou les fragments structurants sont constitués par un fragment d'un oligomère (I) ou (I') qui a une longueur sensiblement identique à celle la partie structurante en hélice α de la protéine ou du polypeptide naturel.

A titre d'exemple, on peut citer une protéine artificielle analogue du CRF humain ou hCRF ("human corticotropin releasing factor" c'est-à-dire "facteur humain de libération de la corticotropine"). Une telle protéine artificielle a une structure analogue à celle du CRF, dans laquelle la partie centrale formant l'hélice α, qui est une partie structurante sans activité biologique, a été remplacée par une séquence -(NR'-A-CO)ₘ-, dans laquelle m est 8 ou 9 et -NR'-A-CO- est un fragment -DBT- dérivé de la (3S)[amino]-5-(carbonylméthyl)-2,3-dihydro-1,5-benzothiazepin-4(5H)-one et représenté par la formule

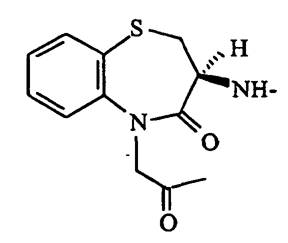

On peut également citer une autre protéine artificielle analogue du hCRF dans lequel la partie centrale formant l'hélice α a été remplacée par une séquence -(NR'-A₁-CO)₂₀-, dans laquelle -NR'-A₁-CO- est un fragment représenté par la formule

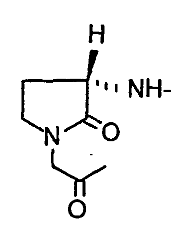

Ces protéines artificielles, analogues du CRF humain, présentent une certaine affinité pour les récepteurs du CRF humain.

La présente invention est décrite plus en détail à l'aide des exemples suivants, qui sont donnés à titre d'illustration et auxquels l'invention n'est pas limitée.

### Exemple 1

### Préparation d'une résine Boc-(DBT)₅- MBHA

La polymérisation a été effectuée en phase solide à l'aide d'un synthétiseur automatique Applied 433 en stratégie Boc en utilisant :
- comme acide aminé (II) : la (35) [amino]-5-(carbonylméthyl)-2,3-dihydro-1,5-benzothiaepin-4(5H)-one, désignée ci-après par H-DBT-OH, dans laquelle la fonction amine a été protégée par un groupe Boc défini précédemment,
- comme résine support : 2 g de résine MBHA.HCl substituée à 0,8 mmol/g par des fonctions amine (commercialisée par Novabiochem).

A partir de la résine MBHA substituée, on a effectué 5 fois la séquence d'opérations suivantes :
- protection de la fonction amine de 1,25 éq. de H-DBT-OH par réaction avec le di-tert-butylpyrocarbonate Boc₂O, pour obtenir 704 mg (1,25 équivalent) de Boc-DBT-OH ;
- couplage dans le diméthylformamide (DMF), à l'aide de HBTU (758 mg) comme agent de couplage en présence de DIEA (0,62 ml), à pH 8-9 ;
- lavage du produit obtenu 1 fois dans le DMF, puis 2 fois dans le méthanol et 2 fois dans le dichlorométhane (DCM) ;
- déprotection par traitement à l'aide d'un mélange d'acide trifluoroacétique (TFA), de dichlorométhane (DCM) et d'éthanedithiol (EDT) dans les proportions suivantes : TFA/DCM/EDT (40/60/2) pendant 2 min, puis filtration, puis second traitement pendant 28 min à l'aide du même mélange TFA/DCM/EDT, la déprotection étant omise après la dernière étape de couplage.

L'on a ainsi récupéré une résine MBHA portant des fragments Boc-(DBT)₅-, désignée ci-après par résine Boc-(DBT)₅-MBHA.

### Exemple 2

### Préparation d'un oligomère Ac-(DBT)₅-NH₂

250 mg d'une résine Boc-(DBT)₅-MBHA obtenue selon le mode opératoire décrit dans l'exemple 1 ont été placés dans un réacteur. Le groupement Boc- a été éliminé à l'aide d'une solution constituée par un mélange TFA/DCM/EDT = 40/60/2, en deux étapes d'une durée respective de 2 min et de 28 min séparées par une filtration. 5 ml d'une solution 50/50 d'anhydride acétique Ac₂O dans le DCM ont ensuite été ajoutés et le mélange a été agité à température ambiante pendant 1 heure. La résine Ac-(DBT)₅-MBHA obtenue a été lavée 1 fois dans le DMF, puis 2 fois dans le méthanol et 2 fois dans le dichlorométhane (DCM), puis séchée sous vide. La résine séchée a ensuite été placée sous vide.

Pour obtenir l'oligomère libre, on a introduit la résine séchée Ac-(DBT)₅-MBHA dans un réacteur en téflon contenant de l'anisole (1 ml/g de résine) et l'on a introduit de l'acide fluorhydrique anhydre par distillation dans le réacteur (10 ml de HF/g de résine). Le milieu réactionnel a été agité à 0°C pendant 1 heure, puis on a éliminé HF par distillation, on a introduit de l'éther anhydre, et on a filtré, cette séquence d'opérations étant renouvelée plusieurs fois.

Le produit brut a été extrait par le diméthylsulfoxyde (DMSO), additionné d'eau, puis lyophilisé, et l'on a obtenu un produit blanc floconneux. 100 mg de ce produit ont été purifiés en HPLC (chromatographie liquide haute pression) préparative et le produit purifié a été analysé par spectrométrie de masse [m/z 616.17 (dichargé, M+H⁺)].

Le temps de rétention (Rt) HPLC est de 15,3 min (détection UV 214 nm, gradient : 0% (A) à 100% (B) en 25 min) ce qui confirme la pureté du produit obtenu.

### Exemple 3

### Préparation d'oligomères Ac-(DBT)ₙ-NH₂, n>5

Une résine obtenue selon le mode opératoire décrit dans l'exemple 1 a été soumise à deux séquences supplémentaires protection/couplage/lavage/déprotection telles que décrites dans l'exemple 1, et l'on a obtenu une résine portant des fragments Boc-(DBT)₇-. 250 mg de cette résine ont été traités selon le mode opératoire de l'exemple 2, pour obtenir finalement l'oligomère Ac-(DBT)₇-NH₂.

De la même manière, on a préparé l'oligomère Ac-(DBT)₉-NH₂ et l'oligomère Ac-(DBT)₁₁-NH₂ en soumettant une résine obtenue selon le mode opératoire décrit dans l'exemple A1, respectivement à 4 et 6 séquences supplémentaires protection/couplage/lavage/déprotection, puis en traitant chacune des résines obtenues selon le mode opératoire de l'exemple 2.

Les différents oligomères ont été analysés de la même manière que l'oligomère Ac-(DBT)₅-NH₂. Les résultats sont les suivants :
- Ac-(DBT)₇-NH₂: m/z 849,9 (dichargé, M+H⁺)
Rt HPLC = 28,4 min (détection UV 214 nm,
gradient: 0% (A) à 100% (B) en 50 min)
- Ac-(DBT)₉-NH₂: m/z 1084, 5 (dichargé, M+H⁺)
Rt HPLC = 32,2 min (détection UV 214 nm,
gradient: 0% (A) à 100% (B) en 50 min)
- Ac-(DBT)₁₁-NH₂: Rt HPLC = 33,3 min (détection UV 214 nm,
gradient: 0% (A) à 100% (B) en 50 min)

### Exemple 4

### Synthèse d'oligomères H-(DBT)ₙ-OH

Divers oligomères H-(DBT)ₙ-OH ont été préparés pour n = 2, 3, 5, 7 et 9. On a mis en oeuvre un procédé analogue à celui des exemples 1 et 2, en utilisant :
- une résine de Merrifield portant 0,66 mmol de DBT par g de résine,
- 2,5 équivalents de Boc-DBT-OH par étape de couplage
- le BOP comme agent de couplage.

La résine de Merrifield portant 0,66 mmol de DBT par g de résine a été obtenue de la manière suivante. On a dissous 2,5 g (7,1 mmol) de Boc-DBT-OH dans 20 ml d'éthanol à 95%, on a ajouté de l'eau jusqu'à la limite de précipitation, puis 1,17 g (3,55 mmol) de Cs₂CO₃. Le pH a été maintenu à 7 et la solution agitée pendant 30 min à température ambiante. Après évaporation de l'éthanol, on a lyophilisé le sel de césium Boc-DBT-O⁻Cs⁺ obtenu. On a ajouté 3,32 g (6, 80 mmol) de ce sel à 3 g d'une résine de Merrifield fonctionnalisée par le chlorobenzyle de 1 à 2 mmol/g (commercialisée par la société Novabiochem) dans 20 ml de DMF. On a agité le milieu réactionnel à 60°C pendant 48 h, puis on a séparé par filtration la résine obtenue, on l'a lavée par DMF, DCM, MeOH, l'eau et à nouveau par DCM, puis séchée sous vide. Le taux de substitution de la résine par des groupes Boc-DBT-O-est de 0,66 mmol/g de résine.

Pour un oligomère contenant n unités DBT, on a effectué (n-1) fois la réaction de couplage sur la résine de Merrifield fonctionnalisée par DBT. A la fin de la polymérisation, les groupes Boc ont été supprimés et la résine clivée de la même manière que dans l'exemple 2. Les oligomères ont été purifiés par HPLC sur une colonne C18, leur pureté a été contrôlée par HPLC sur une colonne C18 Waters, 150 x 4,6 mm, 100 Å, 5 µm. La caractérisation a été effectuée par spectrométrie de masse. Les résultats sont les suivants :
- H-(DBT)₅-NH₂: Rt HPLC = 18,2 min (détection UV 214 nm,
gradient: 30% (A) à 60% (B) en 20 min)
- H-(DBT)₃-NH₂: Rt HPLC = 11,6 min (détection UV 214 nm,
gradient: 30% (A) à 60%(B) en 15 min)
- H-(DBT)₂-NH₂: Rt HPLC = 5,4 min (détection UV 214 nm,
gradient: 30% (A) à 40% (B) en 10 min)

Les produits obtenus ont en outre été caractérisés par 1H RMN du proton à 300 MHz.

### Exemple 5

### Synthèse d'une protéine artificielle analogue du hCRF H-Ser-Glu-Glu-Pro-Pro-(DBT)₈-Lys-Leu-Met-Glu-Ile-Ile-NH₂

Le hCRF (corticotropin releasing factor (human)) est un peptide amidé de 41 amino acides. La partie en hélice α du hCRF (constituée par 30 acides aminés) a été remplacée par un fragment d'oligomère -(DBT)₈- pour obtenir la protéine artificielle du présent exemple.

Au cours d'une première étape, on a préparé l'oligomère Fmoc-(DBT)₈-OH par un procédé de polymérisation en phase solide sur une résine Merrifield fonctionnalisée à 0,66 mmol de DBT par g de résine obtenue selon le procédé décrit dans l'exemple 4. On a placé 0,76 g de cette résine fonctionnalisée dans le réacteur d'un synthétiseur automatique et on a effectué 6 couplages successifs dans des conditions analogues à celles décrite dans l'exemple 1, en utilisant à chaque couplage 4 équivalents (0,528 g, 2 mmol) de Boc-DBT-OH et 20 ml d'une solution de DCC et de HOBT 1M dans le DMF comme agent de couplage. Après chaque étape de couplage, la fonction amine du fragment fixé sur la résine a été déprotégée par une solution de TFA. Une septième réaction de couplage a été effectuée dans les mêmes conditions en utilisant Fmoc-DBT-OH au lieu de Boc-DBT-OH. Ensuite, la résine obtenue a été séchée sous vide, puis traitée par HF en présence d'anisole selon le mode opératoire décrit dans l'exemple 2. Après élimination de HF par distillation, le résidu a été lavé plusieurs fois à l'éther, et filtré en présence de la résine. Le produit brut récupéré a été dissous dans le DMSO et précipité par l'eau, lavé à l'éther et séché sous vide sur P₂O₅, ce qui a permis d'obtenir 400 mg de produit brut. 100 mg de ce produit brut ont été purifiés en HPLC préparative et le produit purifié Fmoc-(DBT)₈-OH obtenu a été analysé par spectrométrie de masse. Rt HPLC = 34,0 min (détection UV 214 nm, gradient: 0% (A) à 100% (B) en 50 min).

On a ensuite synthétisé la protéine artificielle dans un synthétiseur automatique en stratégie Boc, à partir d'une résine MBHA permettant de générer la fonction amide C-terminale, selon le schéma de synthèse suivant, dans lequel le fragment DBT représente le fragment de l'acide aminé impliqué dans la réaction de couplage. Les acides aminés successivement couplés sont indiqués dans le tableau ci-dessous.

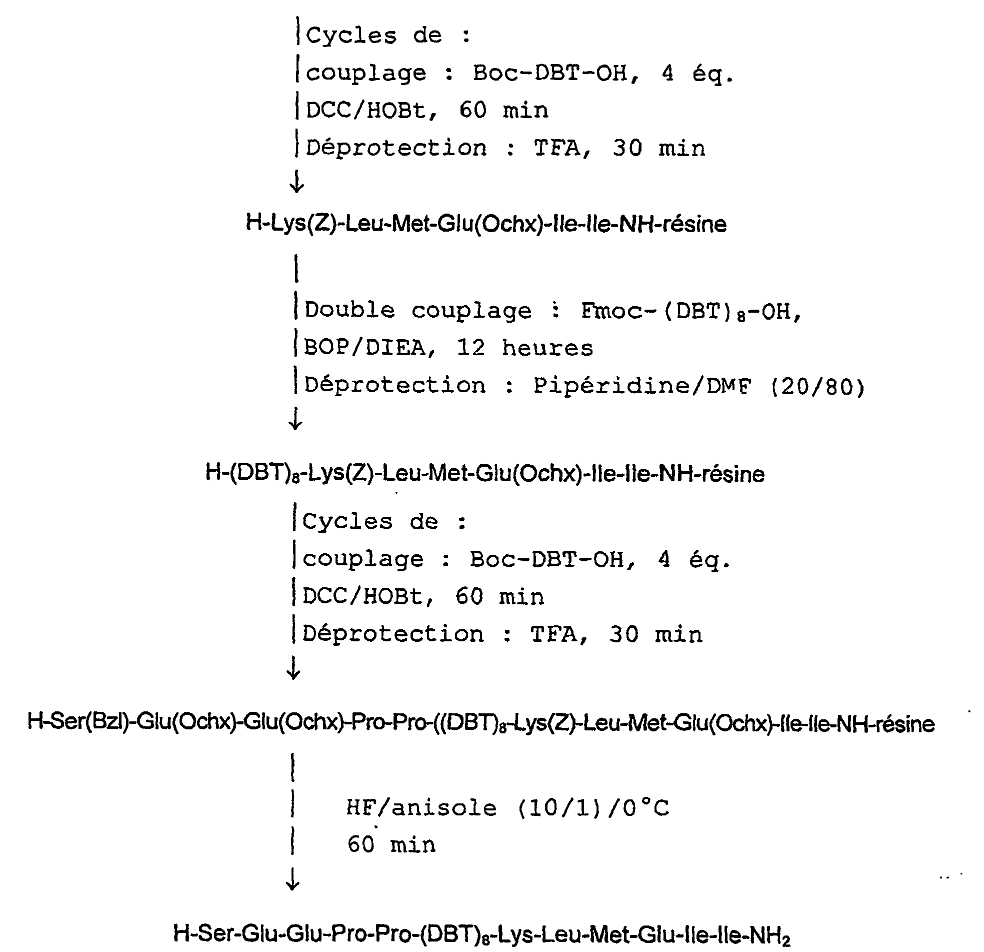

On a placé dans la colonne du réacteur d'un synthétiseur automatique fonctionnant en stratégie Boc, 0,625 g de résine MBHA.HCl substituée à 0,8 mmol et 4 équivalents de chacun des amino-acides requis, correspondant aux quantités suivantes :

| Aminoacide | Quantité (mg) |
|---|---|
| Boc-Ile-OH | 463 |
| Boc-Ile-OH | 463 |
| Boc-Glu(Ochx)-OH | 659 |
| Boc-Met-OH | 498 |
| Boc-Leu-OH.H₂O | 499 |
| Boc-Lys(Z)-OH | 761 |
| Fmoc-(DBT)₈-OH | 1056 |
| Boc-Pro-OH | 430 |
| Boc-Pro-OH | 430 |
| Boc- Glu(Ochx)-OH | 659 |
| Boc- Glu(Ochx)-OH | 659 |
| Boc-Ser(Bzl)-OH | 591 |

Dans le tableau ci-dessus, "chx" signifie "cyclohexyle", "Z" signifie "benzyloxycarbonyle" et "Bzl" signifie "benzyle".

Au cours d'une première étape, on a effectué la synthèse automatique du peptide-résine H-Lys(Z)-Leu-Met-Glu(Ochx)-Ile-Ile-NH-résine en effectuant 6 étapes de couplage successives d'une durée de 60 min, en utilisant le DCC dans HOBt comme agent de couplage, chaque acide aminé étant protégé par un groupe Boc, chaque réaction de couplage étant suivie d'une étape de déprotection par TFA pendant 30 min.

Au cours d'une deuxième étape, le couplage du Fmoc-(DBT)₈-OH a été effectué selon le processus suivant. 1056 mg (1 mmol) d'oligomère H-(DBT)₈-OH ont été couplés au peptide résine H-Lys(Z)-Leu-Met-Glu(OChx)-Ile-Ile-MBHA résine en utilisant un mélange BOP/DIEA comme agent de couplage, pendant 12 heures. Le produit réactionnel a ensuite été traité par une solution à 20% de pipéridine dans le DMF pour éliminer le groupement protecteur Fmoc.

Au cours d'une troisième étape, l'allongement par les acides aminés N-protégés a été poursuivi en mode automatique pour obtenir le composé Boc-Ser(Bzl)-Glu(Ochx)-Glu(Ochx)-Pro-Pro-(DBT)₈-Lys(Z)-Leu-Met-Glu(OChx)-Ile-Ile-MBHA résine.

Après la fin de la synthèse, on a séché le composé porté par la résine sous vide, on l'a traité par une solution TFA/DCM/EDT (50/50/2) pendant 2 min, puis pendant 28 min. On a ensuite traité la résine portant le composé par HF en présence d'anisole suivant le mode opératoire décrit précédemment. On a purifié par HPLC le composé obtenu, et on l'a analysé par spectrométrie de masse comme indiqué dans l'exemple 2. Les résultats obtenus sont les suivants : Rt HPLC = 25,0 min (détection UV 214 nm, gradient: 0% (A) à 100% (B) en 50 min).

### Exemple 6

### Synthèse d'une protéine artificielle analogue du hCRFH-Ser-Glu-Glu-Pro-Pro-(DBT)₉-Leu-Met-Glu-Ile-Ile-NH₂

Dans le présent exemple, on a cherché à remplacer la partie en hélice α par un fragment d'oligomère -(DBT)₉-.

La protéine artificelle a été synthétisé par un procédé analogue à celui de l'exemple 5, mais en préparant au préalable un oligomère Fmoc-(DBT)₉-OH et en utilisant les acides aminés du tableau de l'exemple 5, à l'exception du Boc-Lys(Z)-OH.

### Exemple 7

### Synthèse d'un oligomère Fmoc-(A₁)₅-OH

Au cours d'une première étape, on a fonctionnalisé une résine Merrifield par Boc-A₁-OH pour obtenir Boc-A₁-O-Merrifield. La fonctionnalisation a été effectuée par estérification d'une résine Merrifield chlorométhylée substituée de 1 à 2 mmol/g.

Le groupe A₁ répond à la formule ci-dessous :

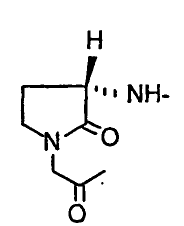

L'acide aminé (II) utilisé est la 3-(S)-amino-1-carbonylméthyl-pyrrolidin-2-one. La résine Merrifield est une résine commercialisée par la société Novabiochem.

Le fragment a ensuite été allongé pas à pas de l'extrémité C-terminale vers l'extrémité N-terminale par des couplages par du BOP et de la DIEA et des déprotections successives. Le dernier mime est introduit sous forme de Fmoc-A₁-OH, puis le polymère total est clivé du support sous forme de Fmoc-(A₁)₅-OH, à l'aide de HF à 0°C en présence d'anisole. Ces conditions de clivage permettent de conserver la protection N-terminale Fmoc.

La synthèse a été réalisée selon le schéma suivant :

| | |
|---|---|
| Couplages 1, 2, 3 | Boc-A₁-OH (2 eq.), 1,48 g |
| | BOP, 2,55 g |
| | DIEA 1,98 mL |
| | DCM |
| Couplage 4 | Fmoc-A₁-OH (2 eq.), 2,19 g |
| | BOP, 2,55 g |
| | DIEA 1,98 mL |
| | DMF |
| Lavages | 1 x DCM |
| | 2 x MeOH |
| | 2 x DCM |
| Déprotection | TFA/DCM (50/50) 2 min |
| | essorage |
| | TFA/DCM (50/50) 28 min |

Pour effectuer le clivage, la résine (1,1 g) a été placée dans un réacteur en téflon contenant 1,1 ml d'anisole, Après distillation de HF (11 ml) dans le réacteur, le mélange a été agité à 0°C pendant 1 h. Le HF a été éliminé par distillation et le peptide attendu a été précipité par de l'éther, puis filtré en présence de la résine déprotégée. Le produit brut a été élué par un mélange CH₃CN/H₂O/TFA (50/50/0,1) puis lyophilisé pour conduire à un composé floconneux blanc. L'opération a été répétée 3 fois pour conduire à 1,84 g de produit, qui a été analysé par spectrométrie de masse (ESI), m/z 941.

### Exemple 8

### Préparation de protéine artificielle H-Ser-Glu-Glu-Pro-Pro-(A₁)₂₀-Arg-Lys-Leu-Met-Glu-Ile-Ile-NH₂

La synthèse de la protéine artificielle H-Ser-Glu-Glu-Pro-Pro-(A₁)₂₀-Arg-Lys-Leu-Met-Glu-Ile-Ile-NH₂ analogue du hCRF a été réalisée à l'aide d'un synthétiseur automatique PerSeptive sur une résine PAL-PEG-PS obtenue auprès de la société PerSeptive Biosystems. Les mimes ont été introduits par blocs de 5 (4 x Fmoc-(A₁)₅-OH). Les couplages ont été réalisés en présence de HBTU et de DIEA.

Les divers acides aminés utilisés sont commercialisés par les sociétés Bachem, Propetide et Novabiochem.

On a placé 0,6 g de résine Emoc-PAL-PEG-PS substituée à 0,19 mmol/g dans le réacteur du synthétiseur. 5 équivalents de chaque amino acide ont été utilisés pour le couplage (double couplage effectué), correspondant aux quantités indiquées dans le tableau suivant :

| Amino acide | quantité (mg) |
|---|---|
| Fmoc-Ile-OH | 201 |
| Fmoc -Ile-OH | 201 |
| Fmoc-Glu(OtBu)-OH | 248 |
| Fmoc-Met-OH | 212 |
| Fmoc-Leu-OH.H₂O | 201 |
| Fmoc-Lys(Boc)-OH | 267 |
| Fmoc-Arg(Pbf)-OH | 370 |
| Fmoc-(A₁)₅-OH x 4 | 214 x 4 |
| Fmoc-Pro-OH | 154 |
| Fmoc-Pro-OH | 154 |
| Fmoc-Glu(OtBu)-OH | 194 |
| Fmoc-Glu(OtBu)-OH | 194 |
| Fmoc-Ser(tBu)-OH | 175 |

Dans le tableau ci-dessus, "OtBu" signifie tertiobutylester ; "Pbf" signifie 2,2,4,6,7-pentaméthyldihydrobenzofuran-5-sulfonyle ; "tBu" signifie "tertiobutyle".

HBTU et DIEA ont été utilisés sous forme de solutions à 0,5 M dans le diméthylformamide. Le groupement Fmoc a été éliminé par action d'une solution de pipéridine dans le DMF (20/80). 2 équivalents de Fmoc-(A₁)₅-OH (214 mg) ont introduits à l'aide d'un réacteur manuel en présence de BOP (100 mg) et de DIEA (40 µl) dans le DMF. Le groupement Fmoc a été éliminé par 5 ml d'une solution de pipéridine dans le DMF. La déprotection a été effectuée en 2 fois : 3 min, puis 7 min. Le résine a été lavée 1 fois par DMF, 2 fois par MeOH et 2 fois par DCM. Le dernier fragment n'a pas étédéprotégé et la résine a été replacée dans le synthétiseur automatique. Le groupement Fmoc du dernier amino acide (avant clivage et déprotection finale) a été éliminé.

A la fin de la synthèse, la résine a été séchée sous vide, puis traitée par une solution TFA/thioanisole pendant deux heures.

Le schéma de l'ensemble des réactions est résumé ci-après :

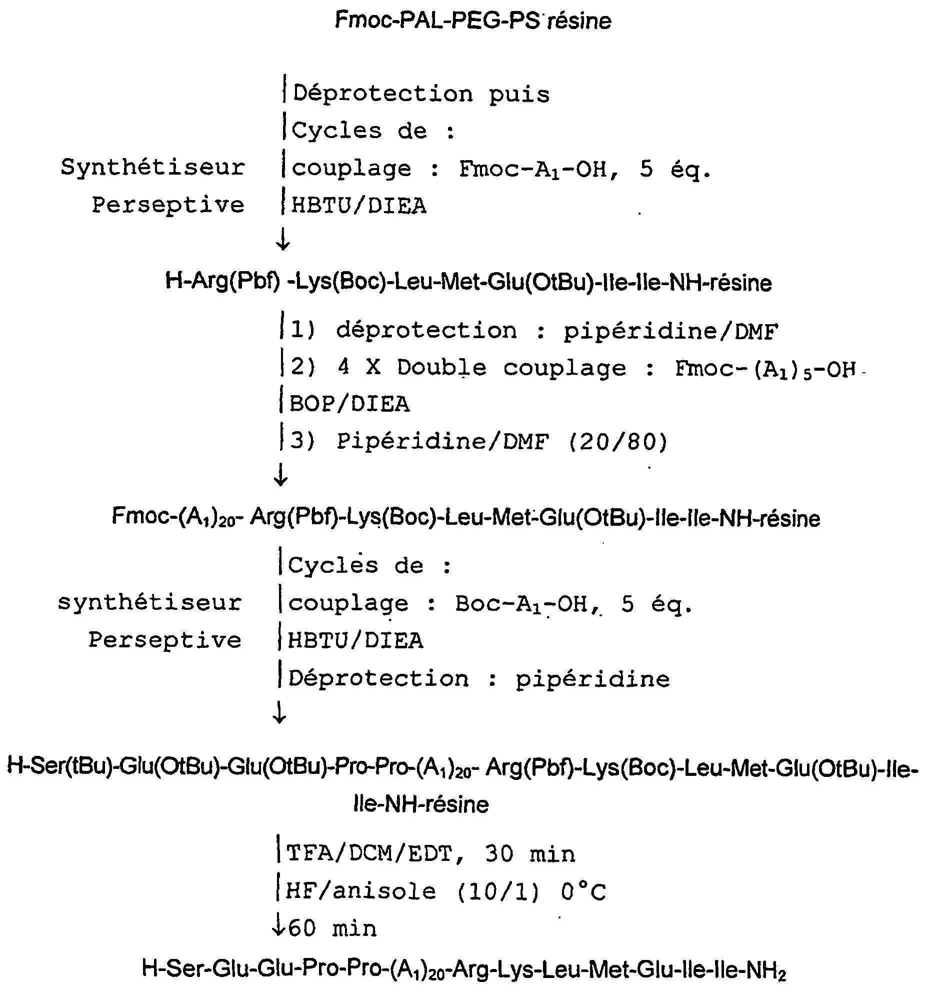

## Revendications

1. Oligomère représenté par l'une des formules générales
R₁-(NR'-A-CO)ₘ-OR₂ (I)
ou
R₁-(NR'-A-CO)ₘ-NR'₂R''₂ (I')
dans lesquelles :
- l'unité -NR'-A-CO- représente un mime contraint non peptidique inducteur de coude β, d'un fragment dipeptide ou tripeptide, étant entendu que A représente un groupe hétérocyclique aromatique ou non, monocyclique ou polycyclique éventuellement condensé, ou que le groupe -NR'-A-CO représente un groupe hétérocyclique aromatique ou non, monocyclique ou polycyclique éventuellement condensé ;
- R₁ représente un groupe acyle R₃-CO- ou un groupe R₃-O-CO- dans lesquels R₃ représente un groupe benzyle, un groupe tert-butyle ou un groupe 9-fluorénylméthyle ;
- R₂ représente H, un groupe alkyle ou un groupe benzyle ;
- R'₂ et R''₂ représentent indépendamment l'un de l'autre H, un groupe alkyle, ou un groupe benzyle ;
- R' représente un atome d'hydrogène ou bien R' forme un groupe monocyclique ou un groupe polycyclique éventuellement condensé avec l'atome N et le groupe A ;
- m est compris entre 2 et 40.

2. Oligomère selon la revendication 1, **caractérisé en ce que** le groupe -NR'-A-CO- comprend un centre asymétrique, qui peut être de configuration R ou de configuration S.

3. Oligomère selon la revendication 1, **caractérisé en ce que** les unités récurrentes -NR'-A-CO- sont toutes identiques.

4. Oligomère selon la revendication 1, **caractérisé en ce que** les unités récurrentes -NR'-A-CO- sont différentes.

5. Oligomère selon la revendication 1, **caractérisé en ce que** les unités récurrentes sont choisies parmi les groupes suivants : 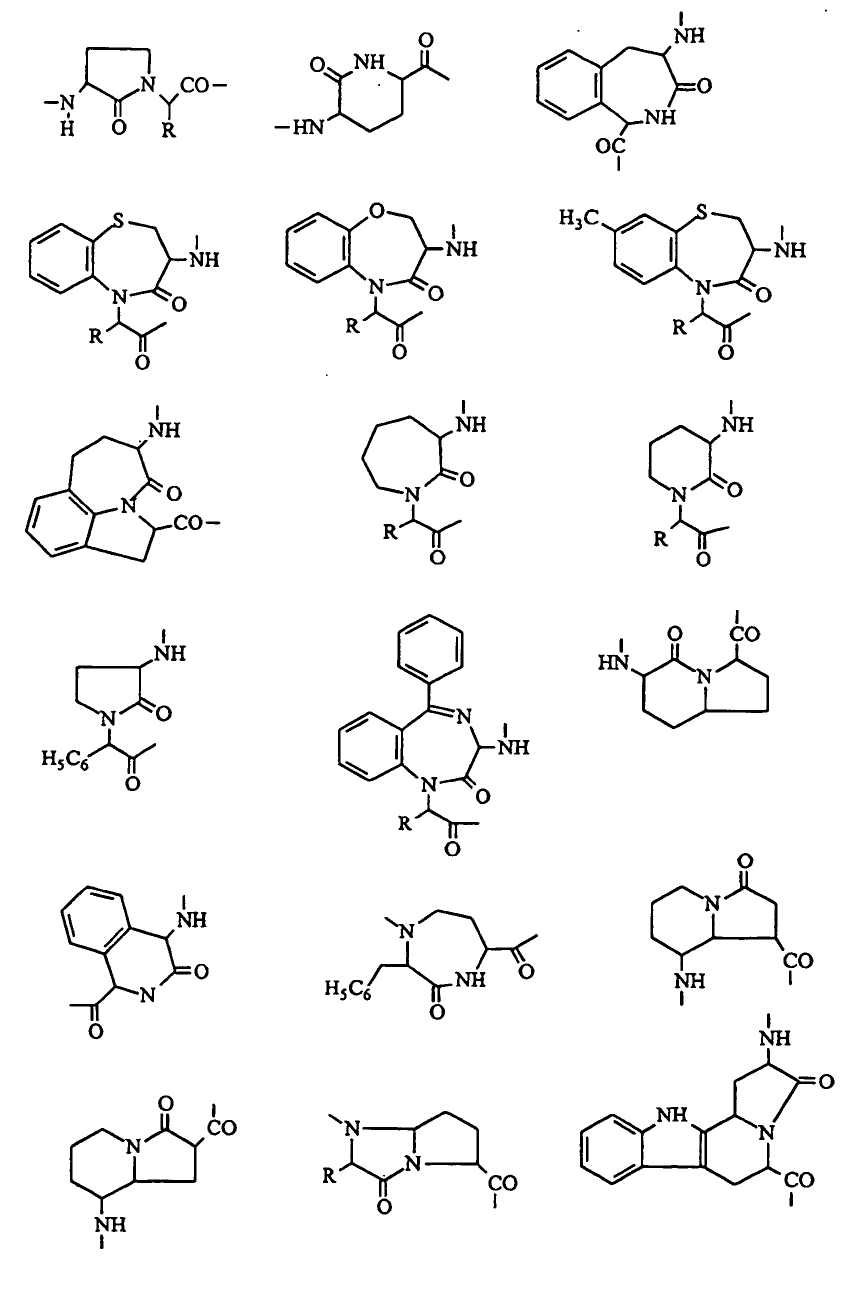 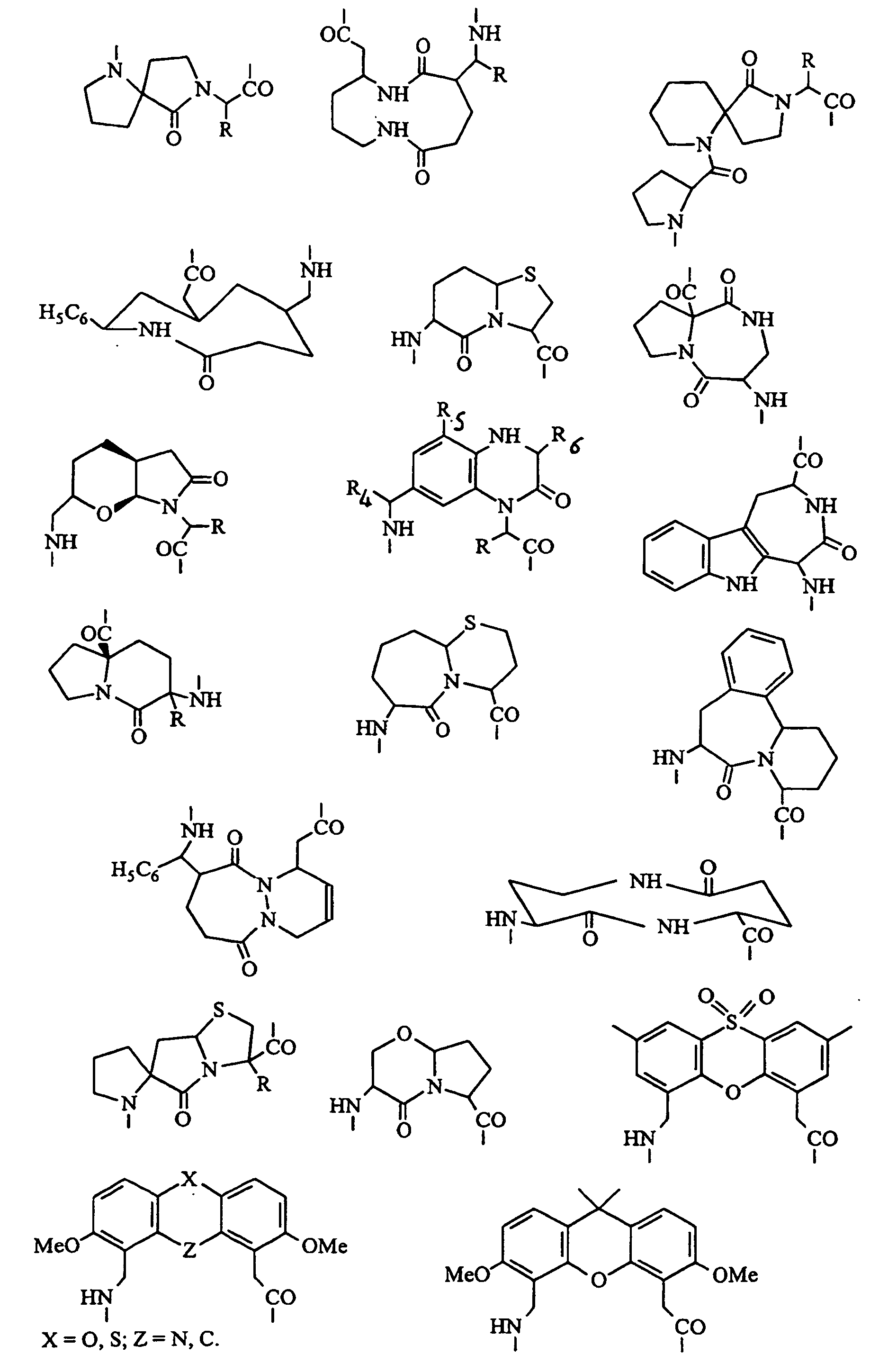 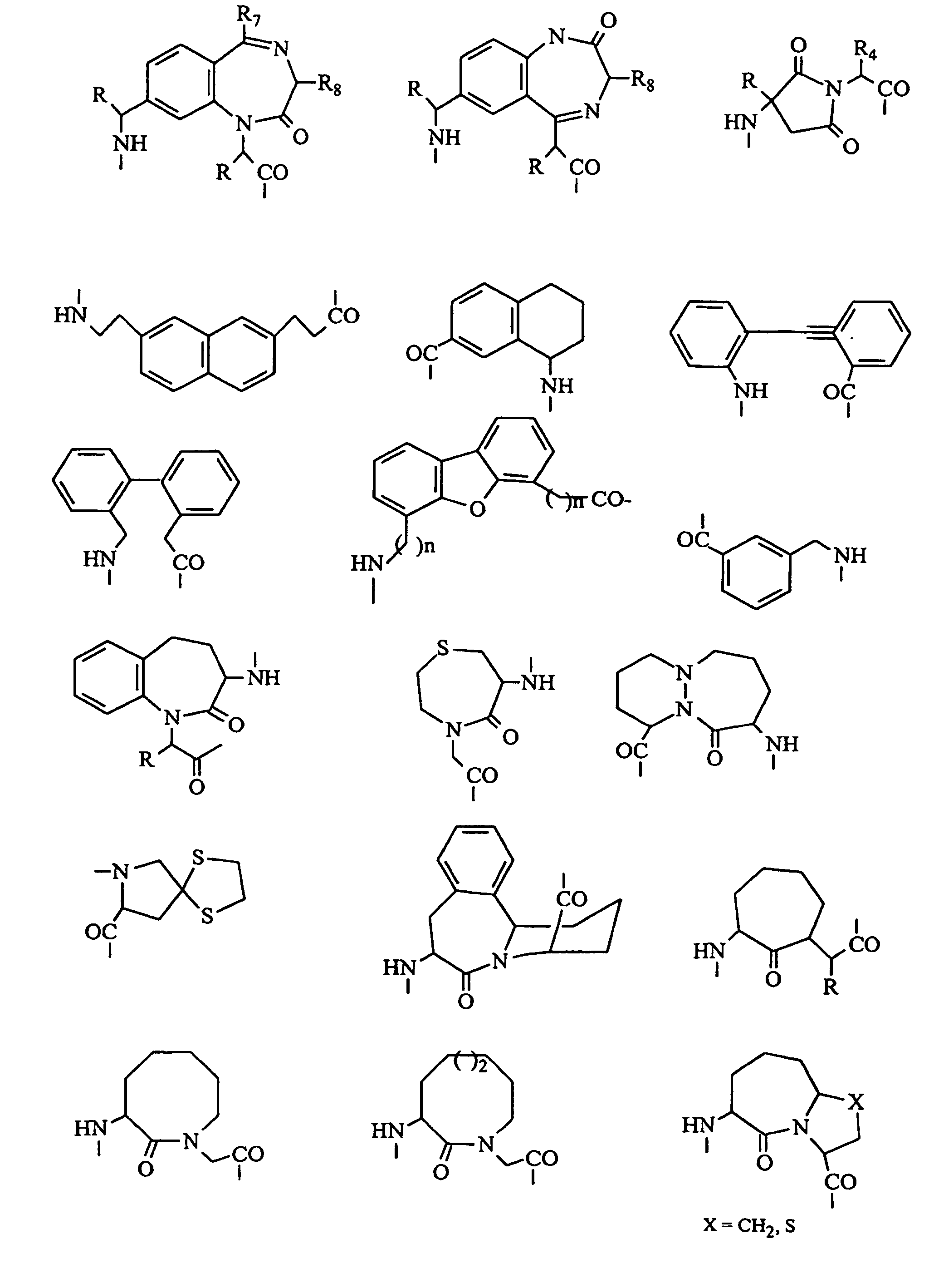 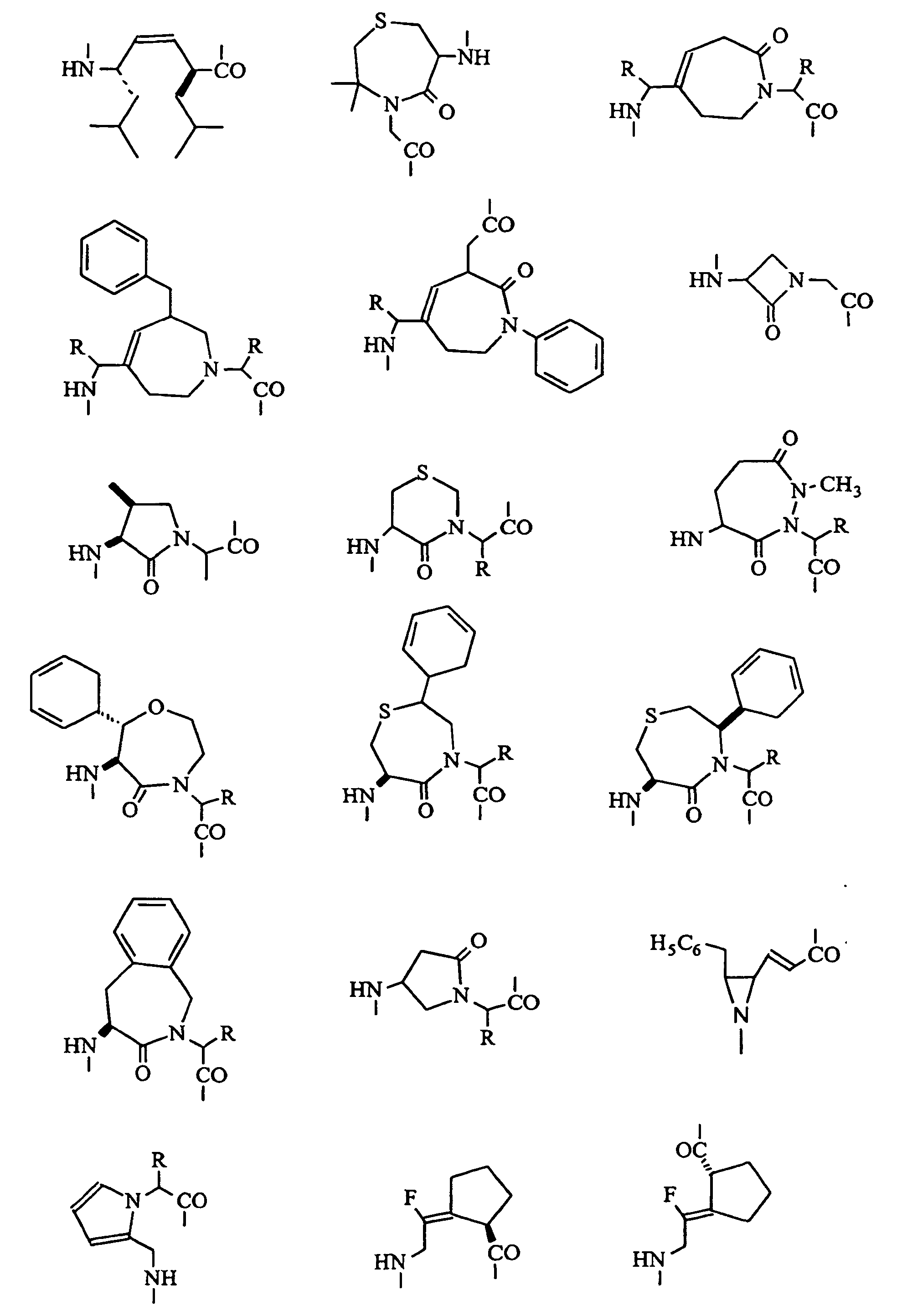 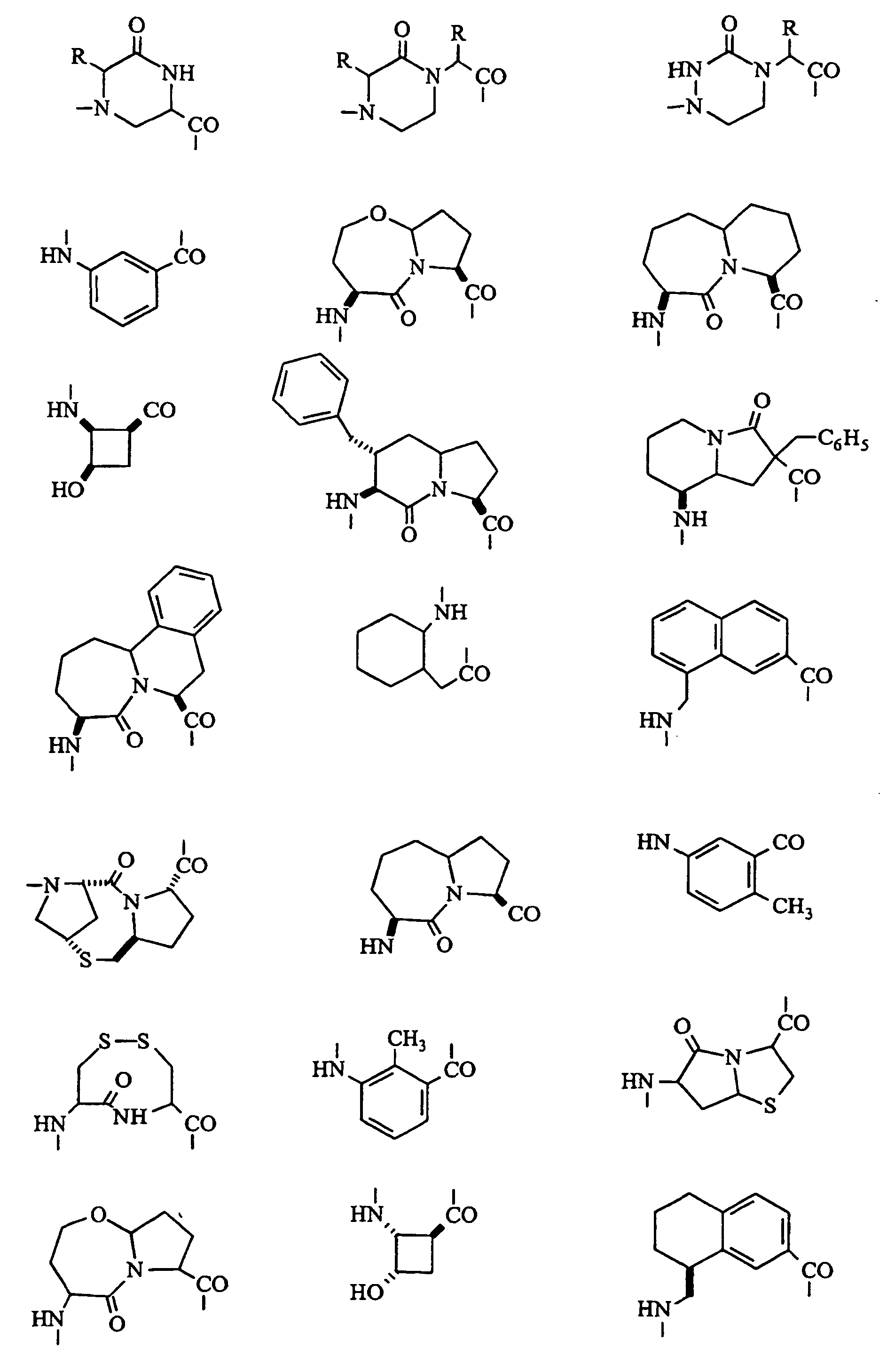 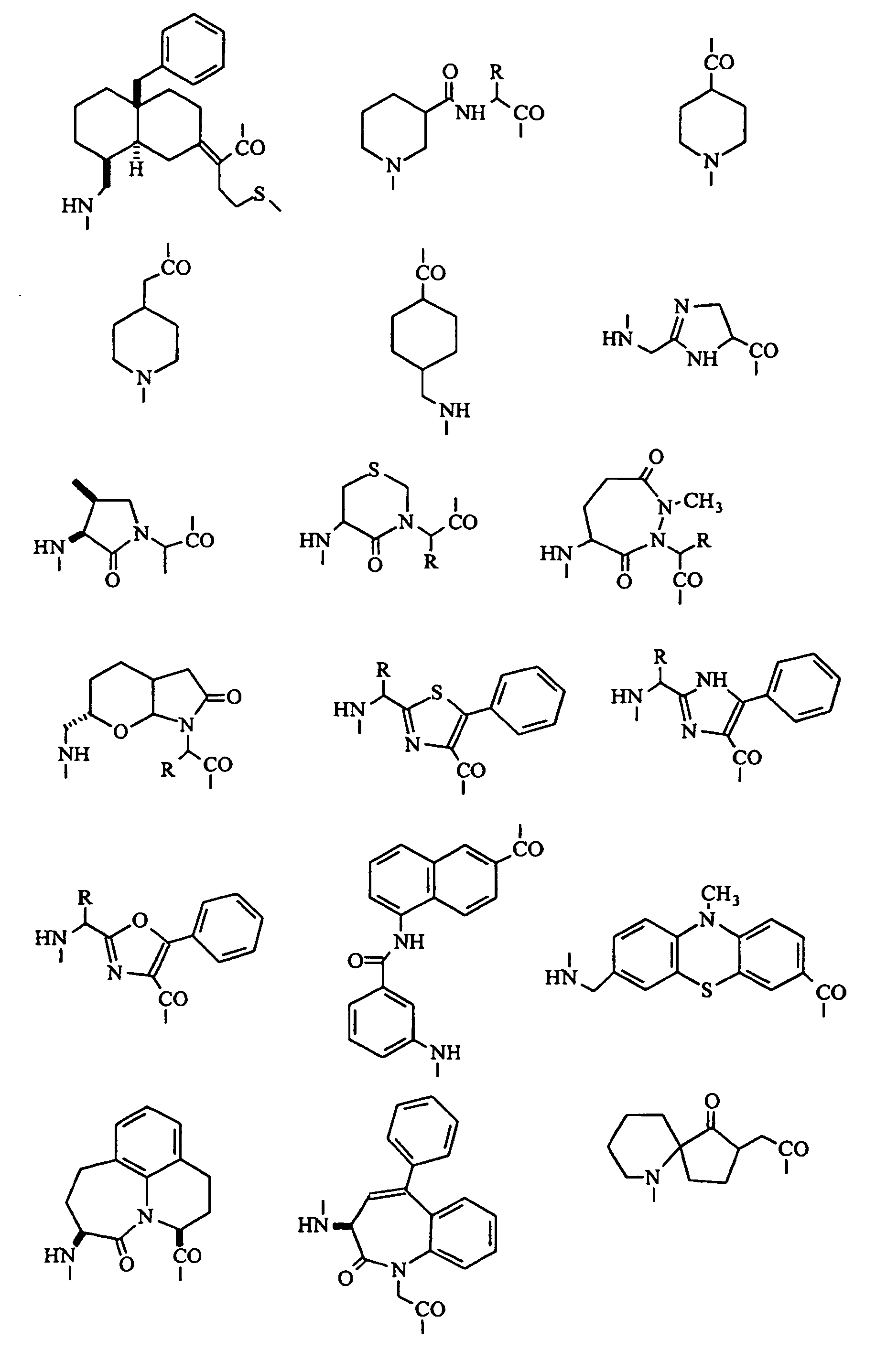 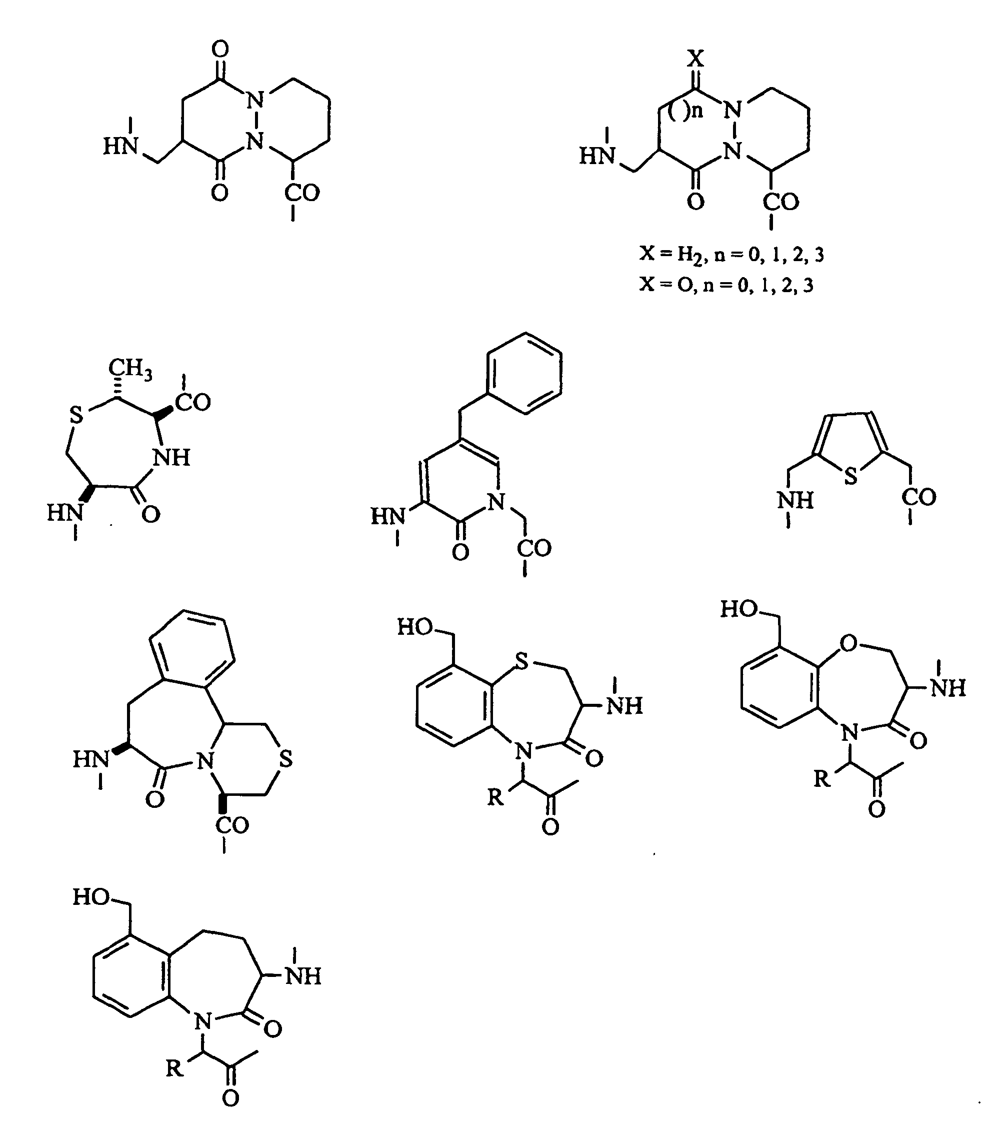 dans lesquels :
- R, et le cas échéant R₄, sont choisis indépendamment l'un de l'autre parmi les groupes constituant les chaînes latérales des α-aminoacides ;
- R₅ et R₆ représentent indépendamment l'un de l'autre H, CH₃-, ou C₆H₅-CH₂- ;
- R₇ représente H ou un phényle ;
- R₈ représente H, CH₃-, C₂H₅- ou C₆H₅-CH₂- ;
- les substituants X et Z sont définis de manière spécifique pour chaque composé qui les contient, et
- Me représente un groupe méthyle ; et
- n représente le nombre d'atomes de carbone, et est égal à 0, 1, 2 ou 3.

6. Oligomère selon la revendication 5, **caractérisé en ce que** R, et le cas échéant R₄ sont choisis indépendamment l'un de l'autre parmi H, CH₃-, (CH₃)₂CH-, CH₃-(CH₂)₃- ou C₆H₅-CH₂-.

7. Procédé de préparation d'un oligomère selon la revendication 1, **caractérisé en ce qu**'il consiste à effectuer une polymérisation d'au moins un acide aminé constituant un mime contraint non peptidique inducteur de coude β, d'un dipeptide ou d'un tripeptide et répondant à la formule NHR'-A-CO-OH (II), dans laquelle R' représente un atome d'hydrogène ou bien R' forme un groupe monocyclique ou un groupe polycyclique éventuellement condensé avec l'atome N et le groupe A.

8. Procédé selon la revendication 7, **caractérisé en ce que** A représente un groupe hétérocyclique aromatique ou non, monocyclique ou polycyclique éventuellement condensé.

9. Procédé selon la revendication 7, **caractérisé en ce que** le groupe -NR'-A-CO- représente un groupe hétérocyclique aromatique ou non, monocyclique ou polycyclique éventuellement condensé.

10. Procédé selon la revendication 7, **caractérisé en ce que** le groupe -NR'-A-CO- comprend un centre asymétrique.

11. Procédé selon la revendication 7, **caractérisé en ce que** la polymérisation est effectuée en solution.

12. Procédé selon la revendication 7, **caractérisé en ce que** la polymérisation est effectuée en phase solide, selon une stratégie de synthèse peptidique.

13. Procédé selon la revendication 7, **caractérisé en ce que** le composé (II) est choisi parmi les composés suivants, dans lesquels :
- R, et le cas échéant R₄, sont choisis indépendamment l'un de l'autre parmi les groupes constituant les chaînes latérales des α-aminoacides ;
- R₅ et R₆ représentent indépendamment l'un de l'autre H, CH₃-, ou C₆H₅-CH₂- ;
- R₇ représente H ou un phényle ;
- R₈ représente H, CH₃-, C₂H₅- ou C₆H₅-CH₂- ;
- les substituants X et Z sont définis de manière spécifique pour chaque composé qui les contient, et
- Me représente un groupe méthyle ; et
- n représente le nombre d'atomes de carbone, et est égal à 0, 1, 2 ou 3 ;

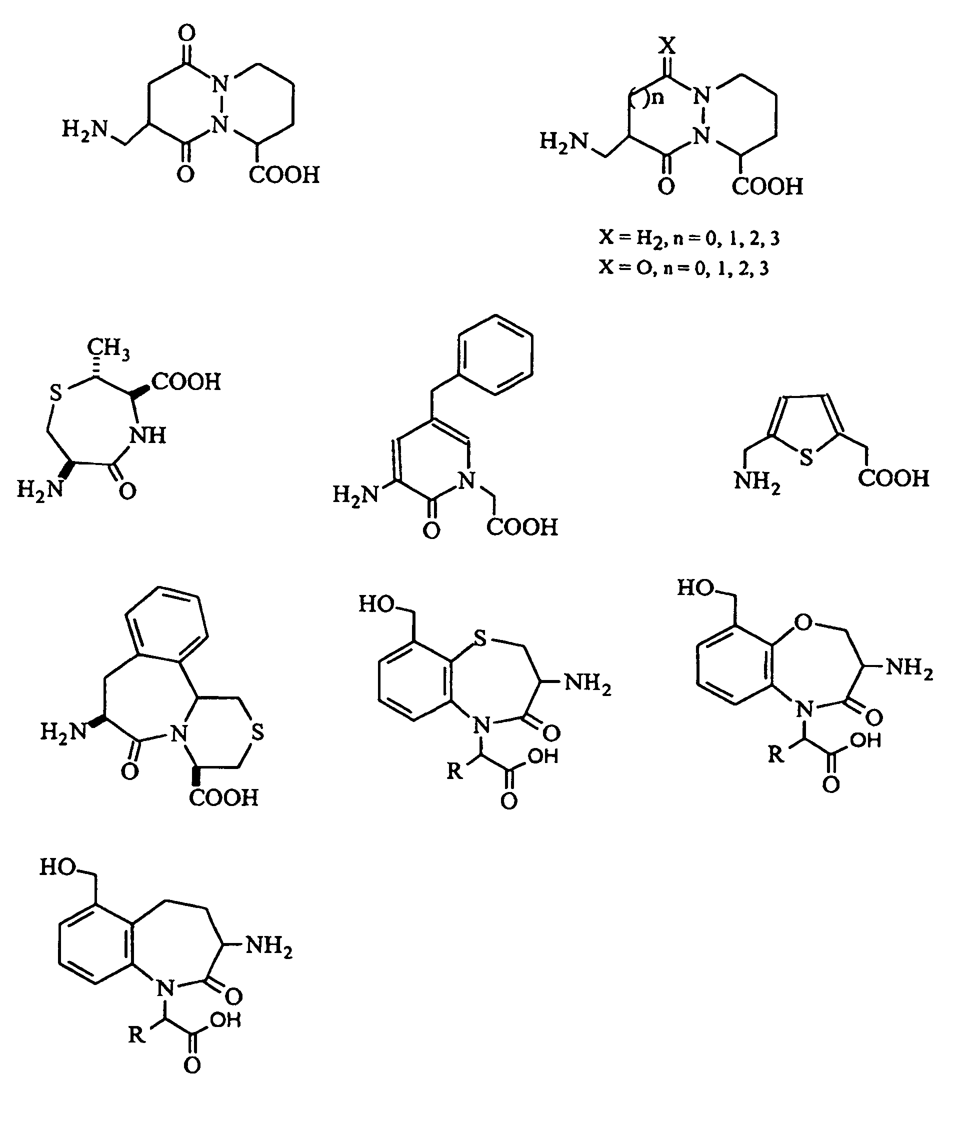

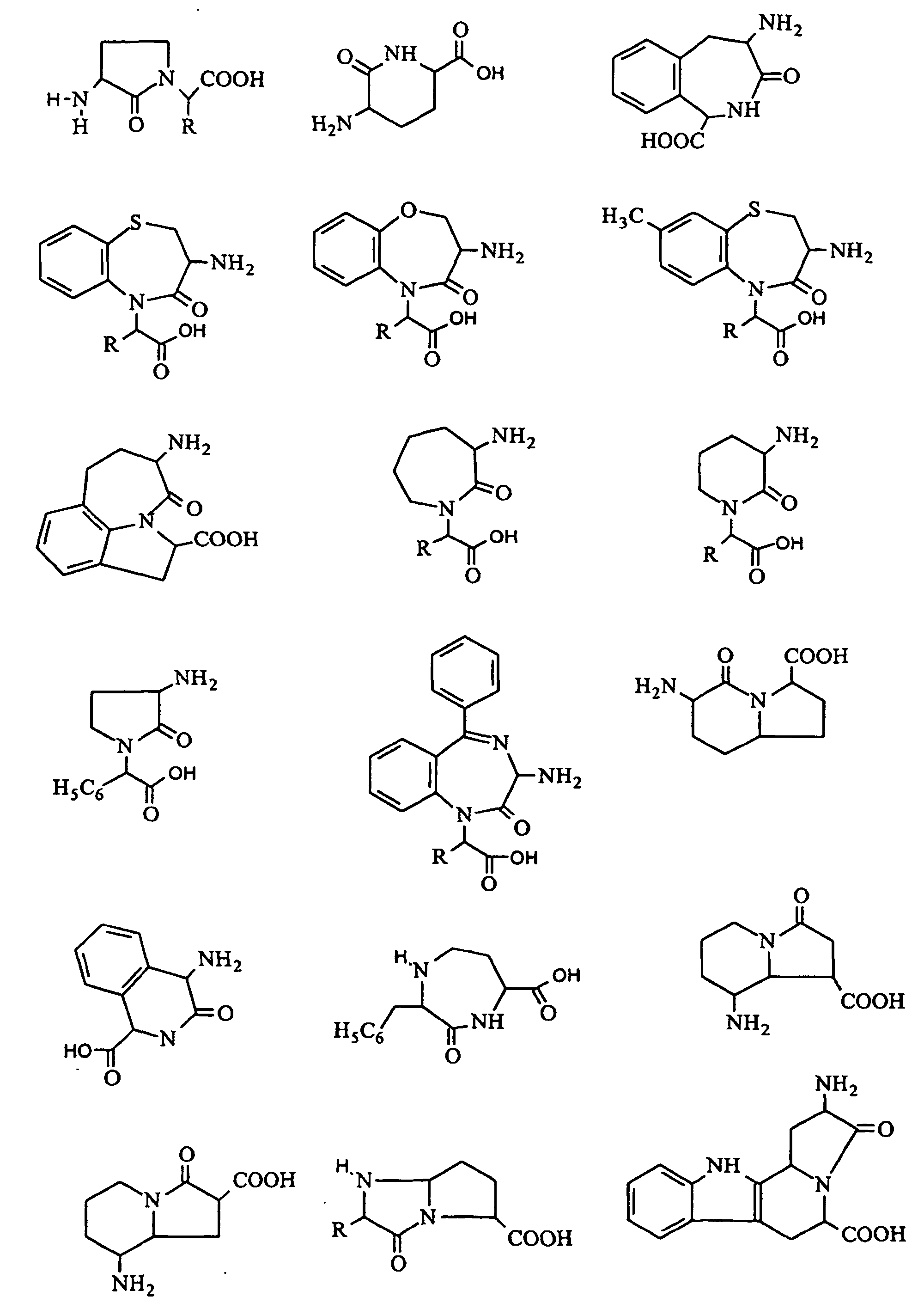

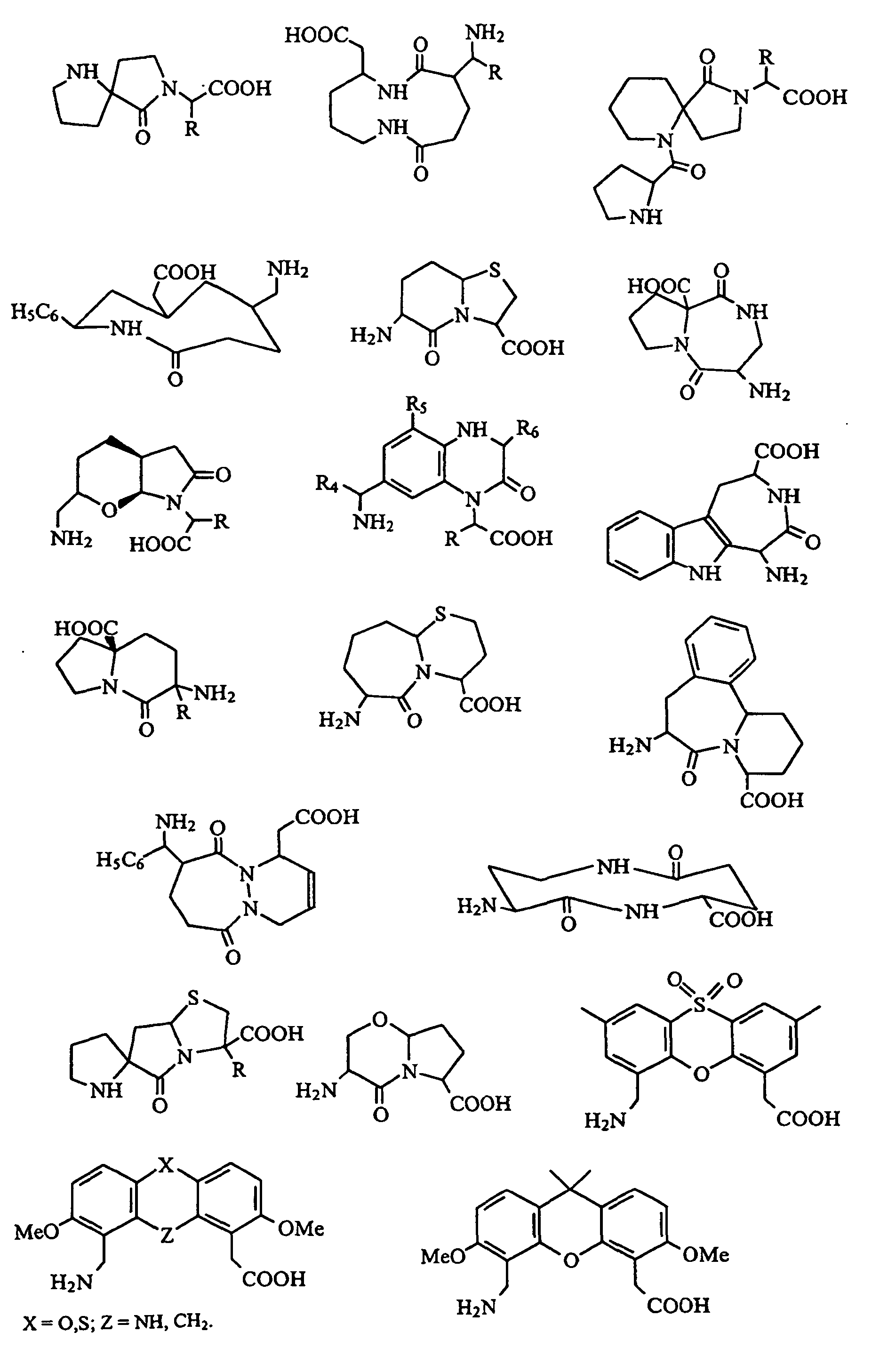

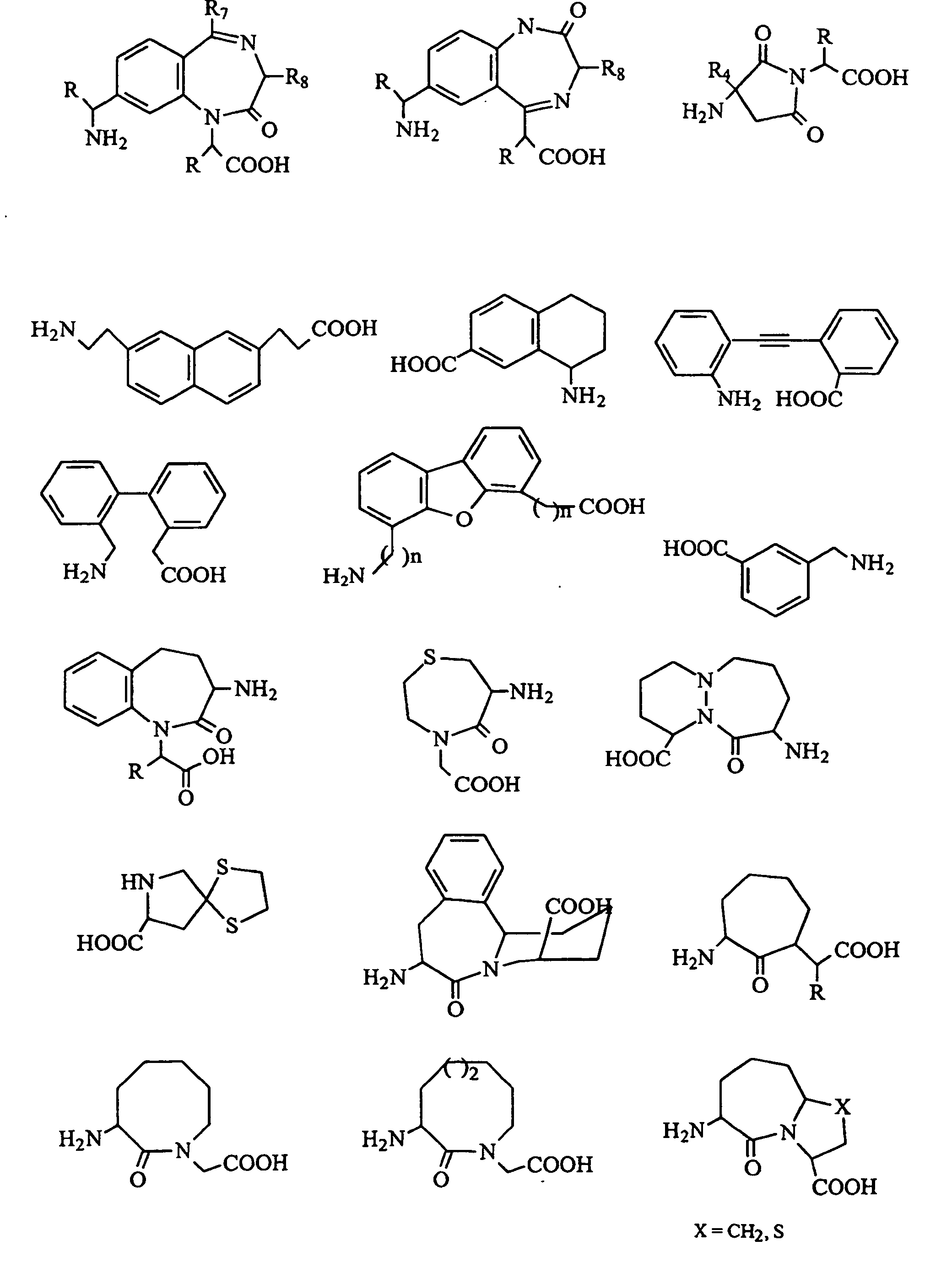

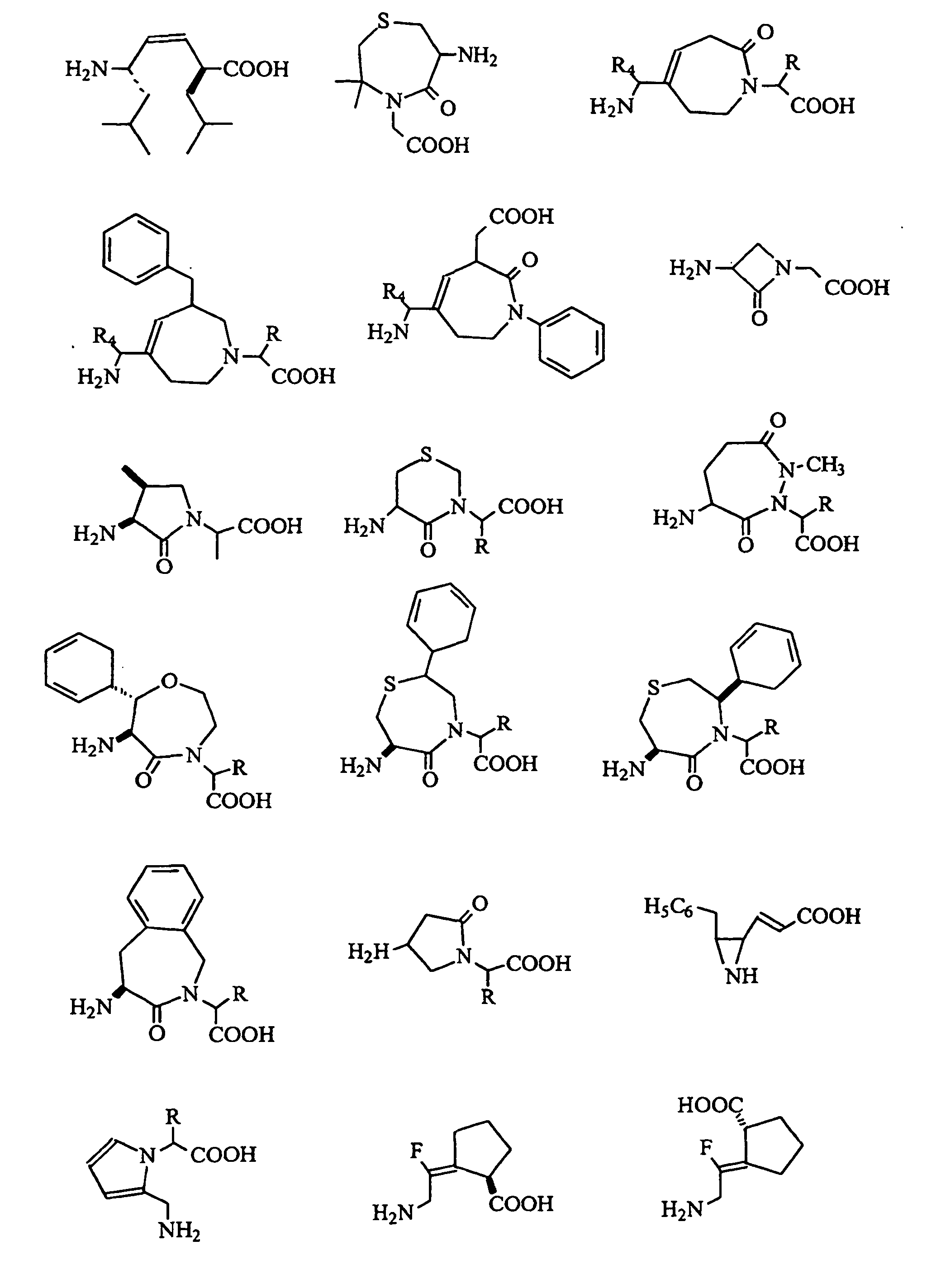

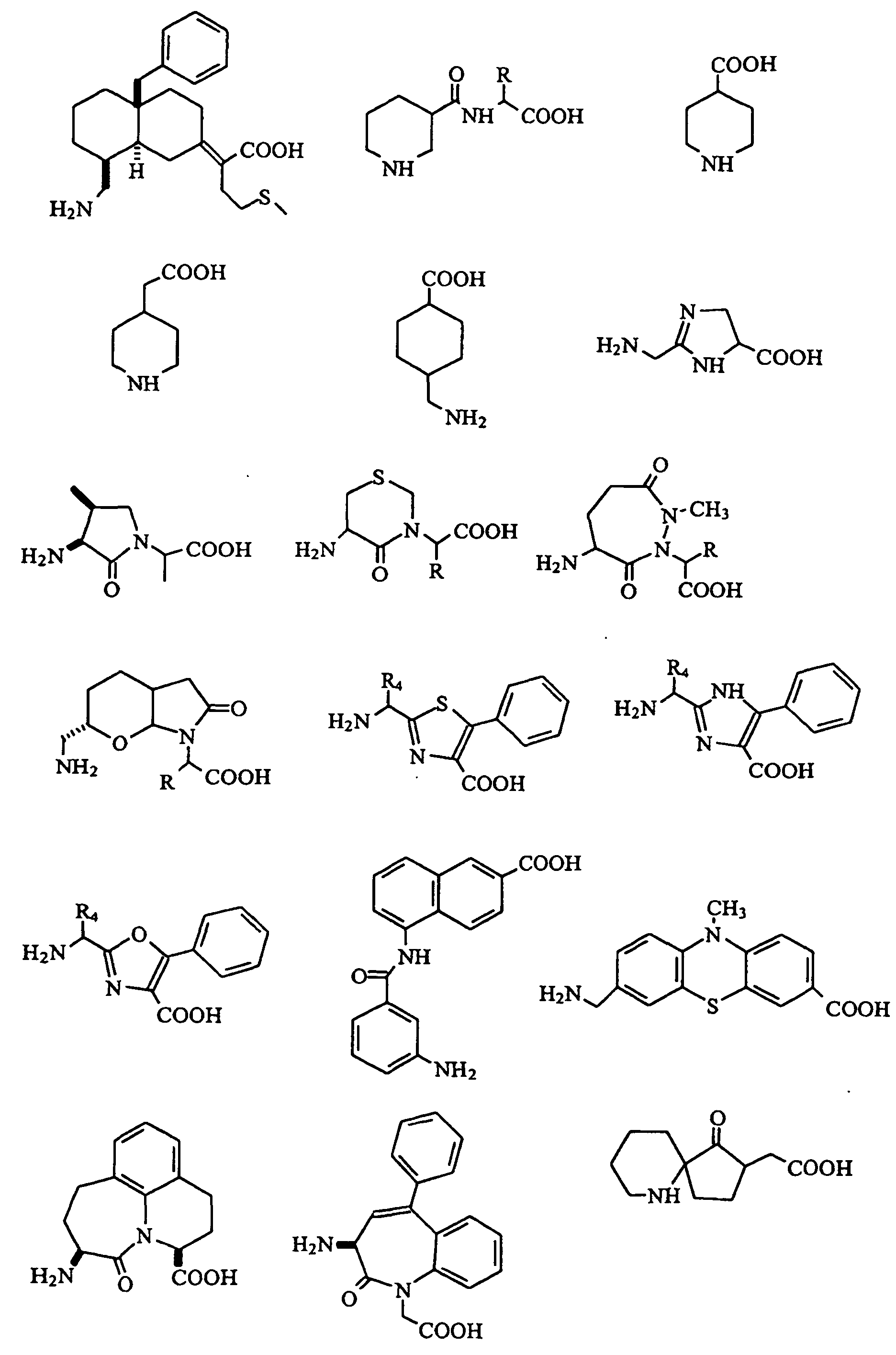

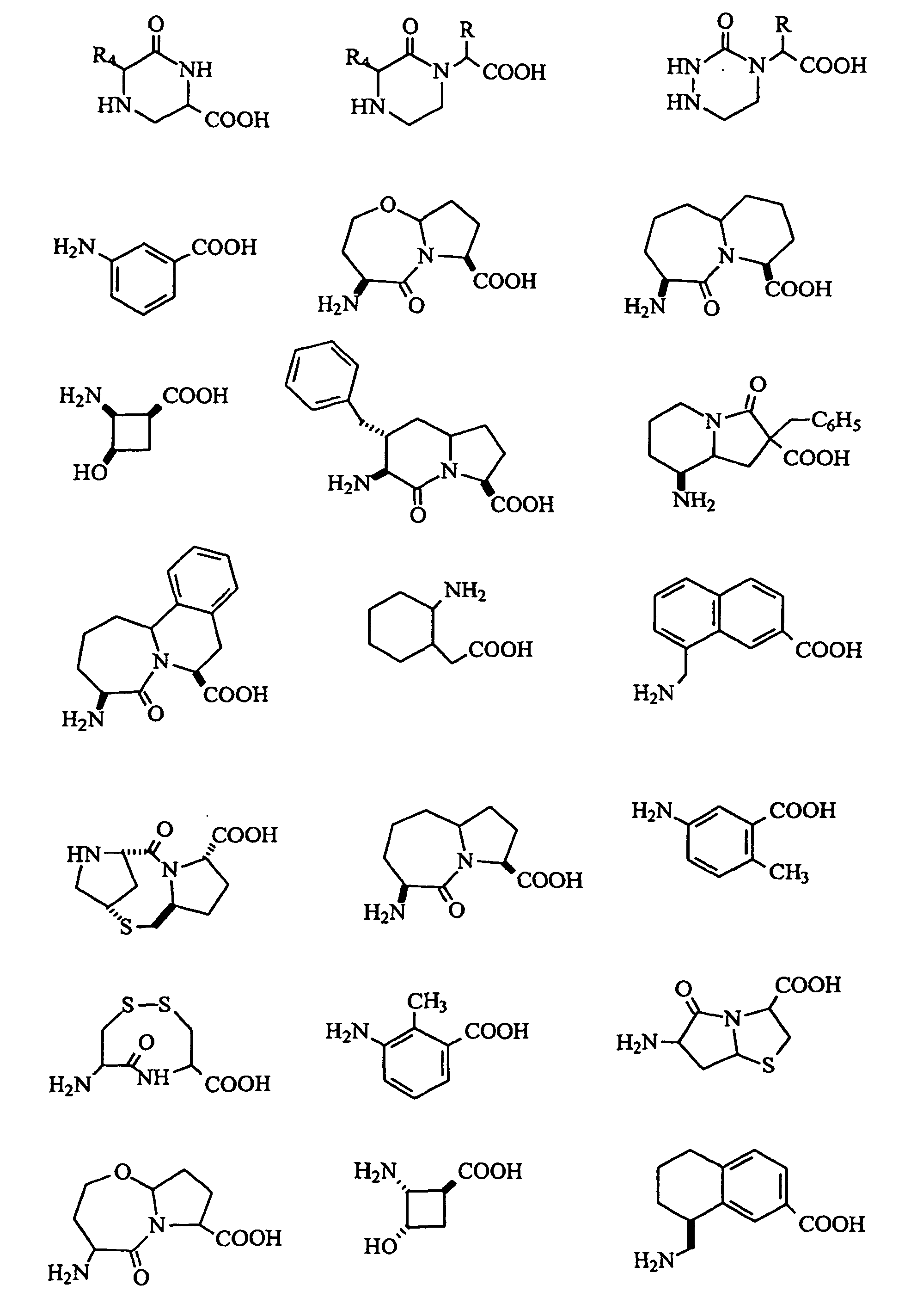

14. Procédé selon la revendication 12, **caractérisé en ce que** :
a) on fonctionnalise une résine support portant des substituants amine par un composé H-NR'-A-CO-OH (II') qui répond à la définition donnée pour (II) et dans lequel le groupe amino a été protégé au préalable ;
b) on allonge le fragment ainsi fixé sur la résine support à partir du côté C-terminal vers le côté N-terminal par (n-2) réactions successives de couplage de monomère (II), ledit monomère (II) étant utilisé en excès, différents monomères (II) pouvant être utilisés dans les étapes de couplage successives ;
c) on effectue une dernière réaction de couplage d'un monomère protégé sur sa fonction N-terminale par un groupe R₁ stable dans les conditions dans lesqulles l'oligomère doit être séparé du support ;
d) on sépare l'oligomère de la résine support.

15. Procédé de préparation d'une protéine artificielle ou d'un polypeptide artificiel analogue d'une protéine naturelle ou d'un polypeptide naturel, consistant à effectuer des réactions de couplage de synthèse peptidique en phase solide, **caractérisé en ce que**, dans la succession des réactions de couplage des acides α-aminés constituant la protéine naturelle ou le polypeptide naturel, une ou plusieurs séquences d'acides α-aminés sont remplacés par un oligomère (I) ou (I') ayant une longueur équivalente à celle de la séquence d'acides α-aminés remplacée.

16. Protéine artificielle ou polypeptide artificiel analoque d'une protéine naturelle ou d'un polypeptide naturel donné, comprenant un ou plusieurs fragments structurants et un ou plusieurs fragments peptidiques, **caractérisé en ce que** le ou les fragments peptidiques sont identiques à ceux de la protéine naturelle ou du polypeptide naturel correspondant, et **en ce que** le ou les fragments structurants sont constitués par un fragment d'un oligomère (I) ou (I') qui a une longueur sensiblement identique à celle de la partie structurante en hélice α de la protéine naturelle ou du polypeptide naturel.

17. Protéine analogue du hcRF (facteur humain de libération de la corticotropine), **caractérisé en ce qu'**elle répond à l'une des formules suivantes :
H-Ser-Glu-Glu-Pro-Pro-(DBT)₈-Lys-Leu-Met-Glu-Ile-NH₂
ou
H-Ser-Glu-Pro-Pro--(DBT)₉-Leu-Met-Glu-Ile-Ile-NH₂
dans lequelles DBT est un fragment dérivé de la (3S)-[amino]-5-(carbonylméthyl)-2-3-dihydro-1,5-benzothiazepin-4(5H)one.

18. Protéine analogue du hCRF (facteur humain de libération de la corticothérapie), **caractérisé en ce qu'**elle répond à la formule suivante
H-Ser-Glu-Pro-Pro-(A₁)-Arg-Lys-Leu-Met-Glu-Ile-Ile-NH₂
dans laquelle A1 est un groupe dérivé de la 3-(*S*)-amino-1-carbonylméthyl-pyrrolidin-2-one et représenté par la formule

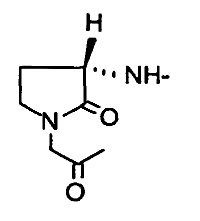

## Patentansprüche

1. Oligomer, das durch eine der allegemeinen Formeln
R₁-(NR'-A-CO)ₘ-OR₂ (I)
oder
R₁(NR'-A-CO)ₘ-NR'₂R''₂ (I')
dargestellt ist, worin:
- die Einheit -NR'-A-CO- für ein nichtpeptidisches Mimetikum eines Dipeptid- oder Tripeptidfragments steht, das eine erzwungene Struktur aufweist und eine Induktor von β-Faltung ist, worin A für eine aromatische oder nichtaromatische heterozyklische, monozyklische oder polyzyklische, gegebenenfalls kondensierte, Gruppe steht oder die Gruppe -NR'-A-CO- für eine aromatische oder nichtaromatische heterozyklische, monozyklische oder polyzyklische, gegebenenfalls kondensierte, Gruppe steht;
- R₁ für eine Acylgruppe R₃-CO- oder eine Gruppe R₃-O-CO- steht, worin R₃ für eine Benzylgruppe, eine tert-Butylgruppe oder eine 9-Fluorenylmethylgruppe steht;
- R₂ für H, eine Alkylgruppe oder eine Benzylgruppe steht;
- R'₂ und R''₂ unabhängig voneinander für H, eine Alkylgruppe oder eine Benzylgruppe stehen;
- R' für ein Wasserstoffatom steht oder R' eine monozyklische Gruppe oder eine, gegebenenfalls mit dem Atom N und der Gruppe A kondesierte, polyzyklische Gruppe bildet;
- m im Bereich von 2 bis 40 liegt.

2. Oligomer nach Anspruch 1, **dadurch gekennzeichnet, dass** die Gruppe -NR'-A-CO ein asymmetrisches Zentrum umfasst, das in R-Konfiguration oder in S-Konfiguration vorliegen kann.

3. Oligomer nach Anspruch 1, **dadurch gekennzeichnet, dass** die Struktureinheiten -NR'-A-CO- alle identisch sind.

4. Oligomer nach Anspruch 1, **dadurch gekennzeichnet, dass** die Struktureinheiten -NR'-A-CO- unterschiedlich sind.

5. Oligomer nach Anspruch 1, **dadurch gekennzeichnet, dass** die Struktureinheiten aus den folgenden Gruppen ausgewählt sind: 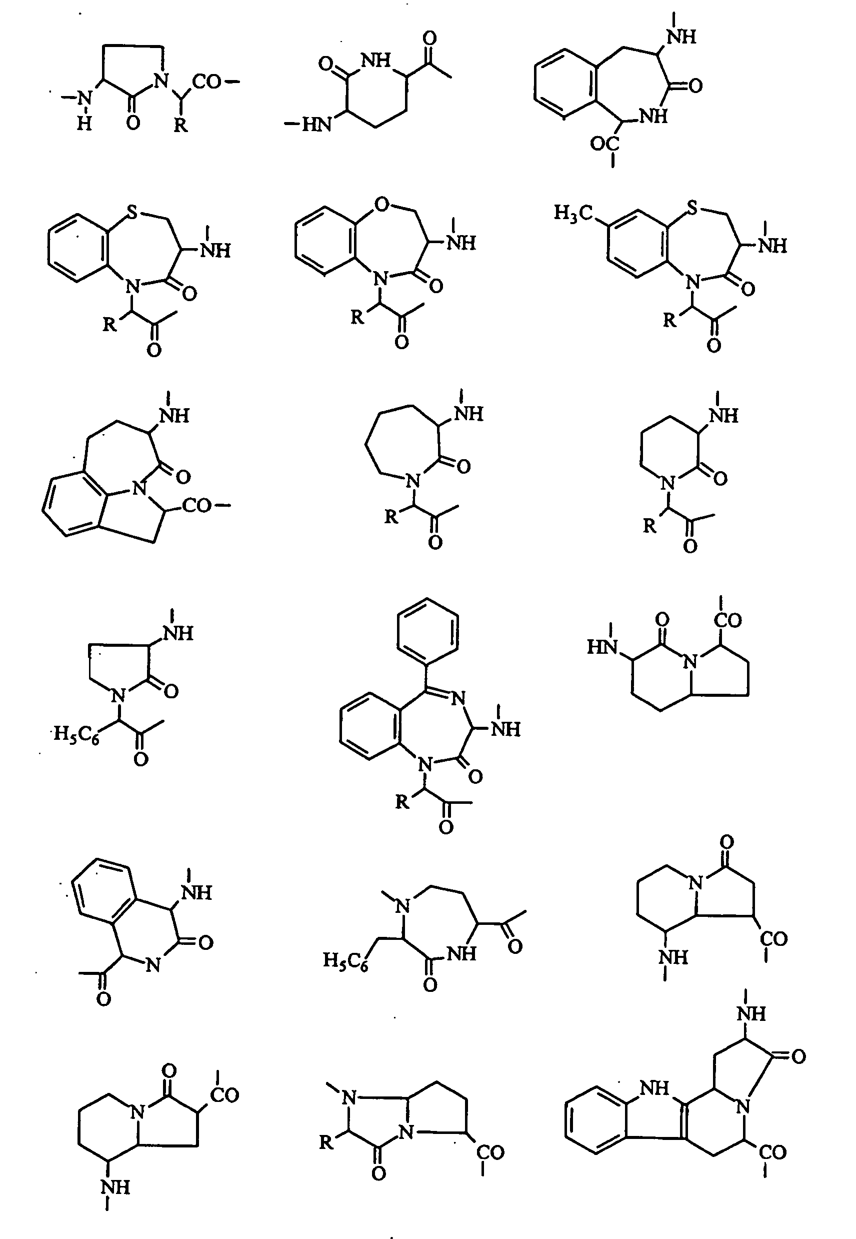 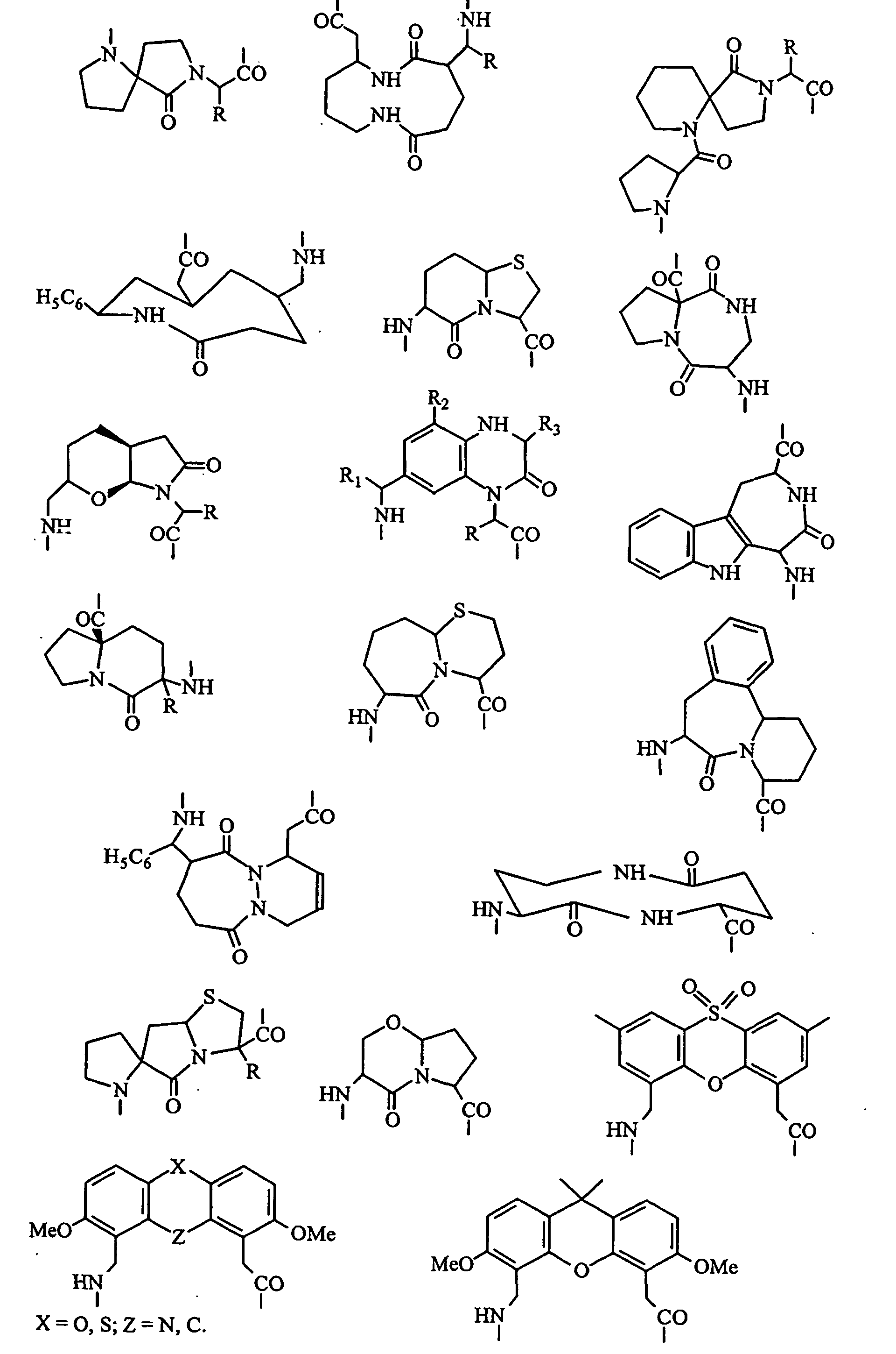 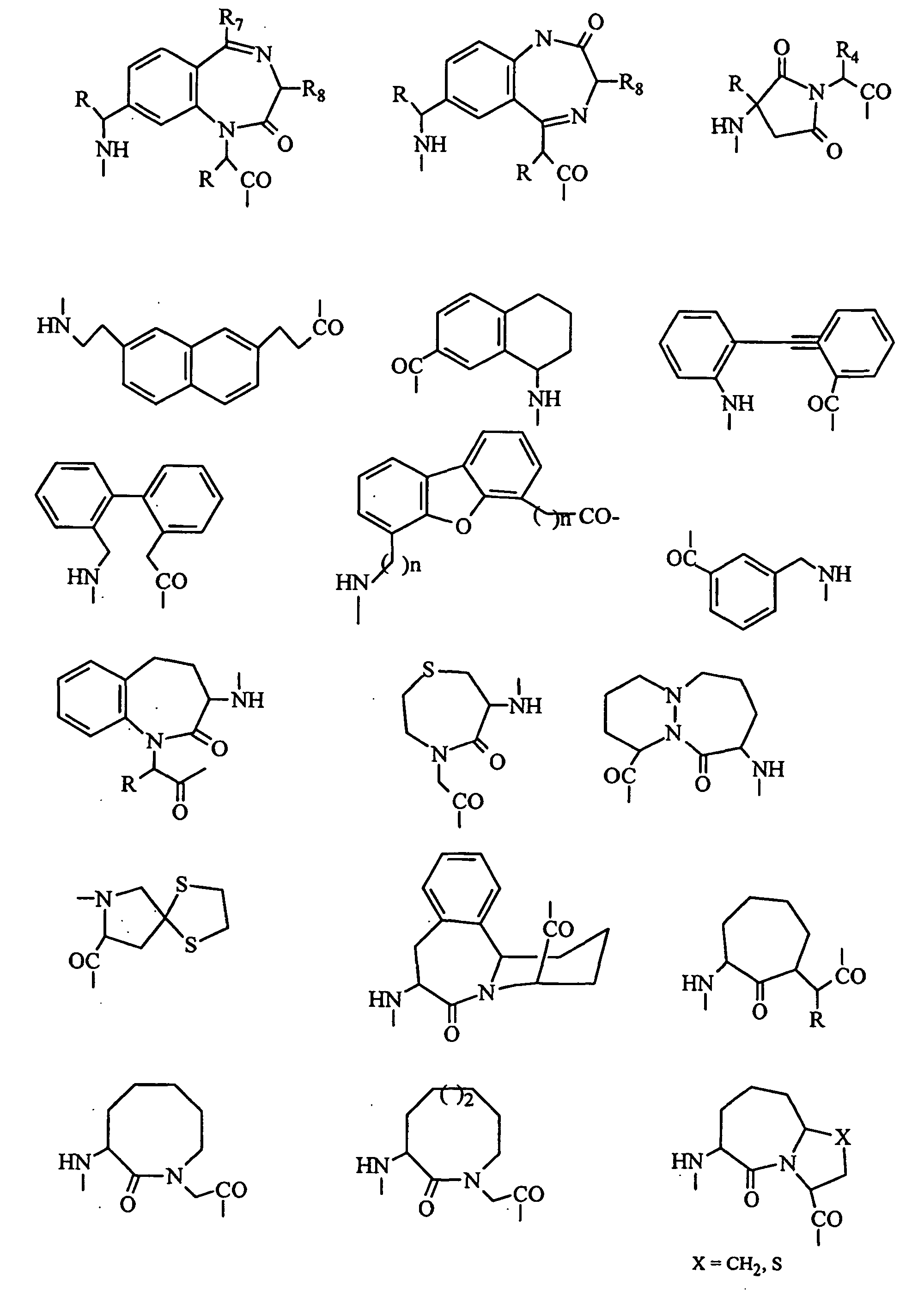 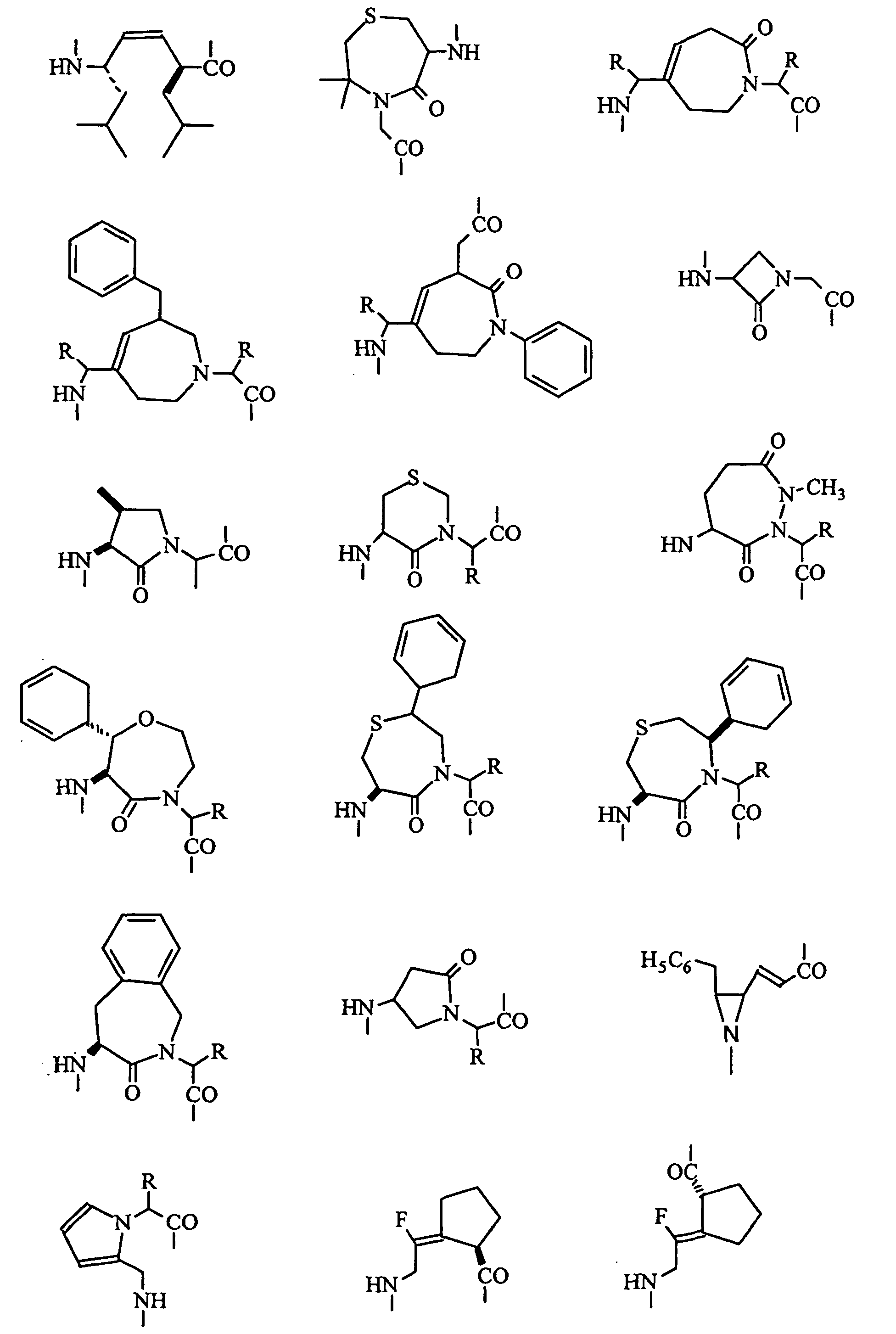 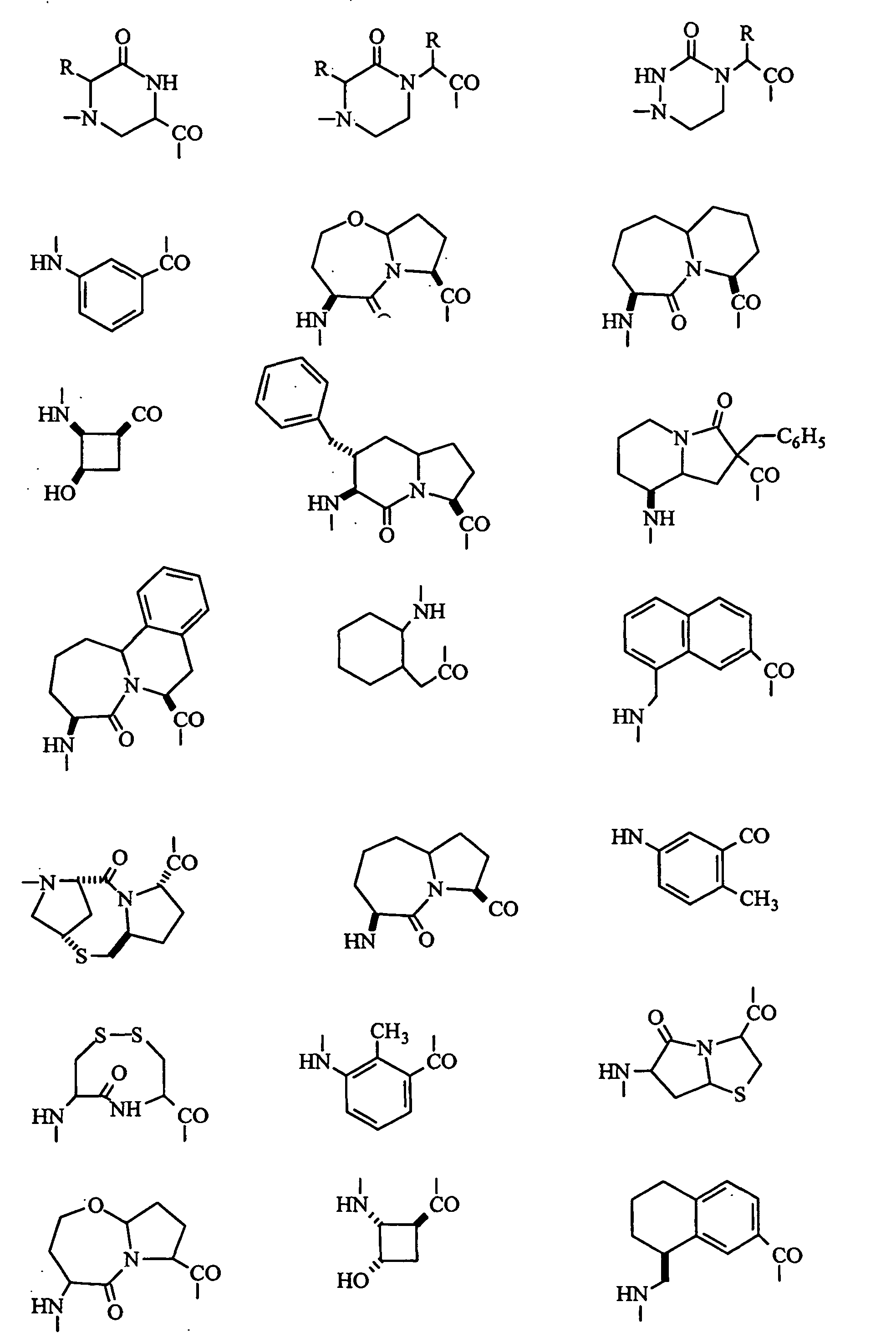 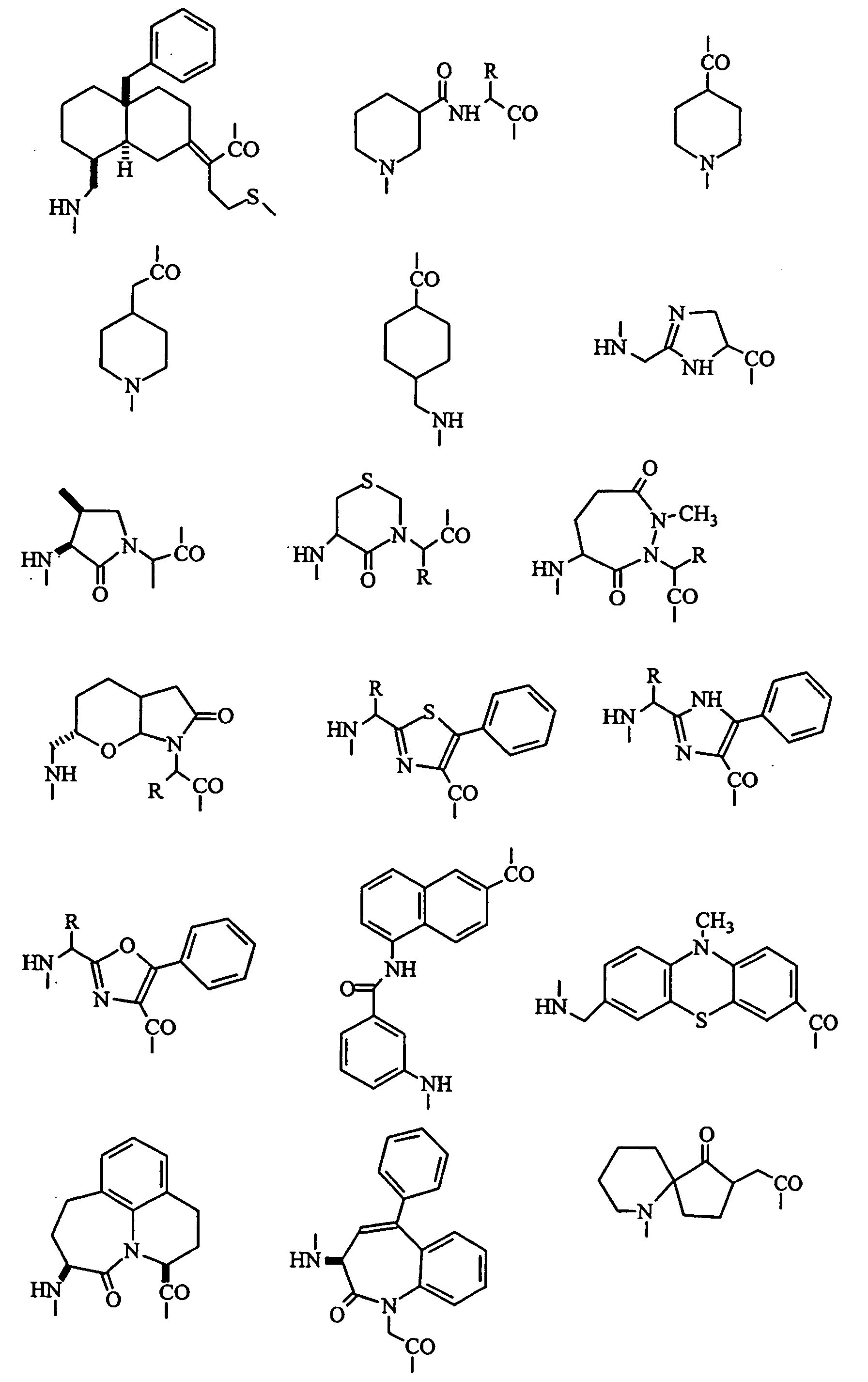 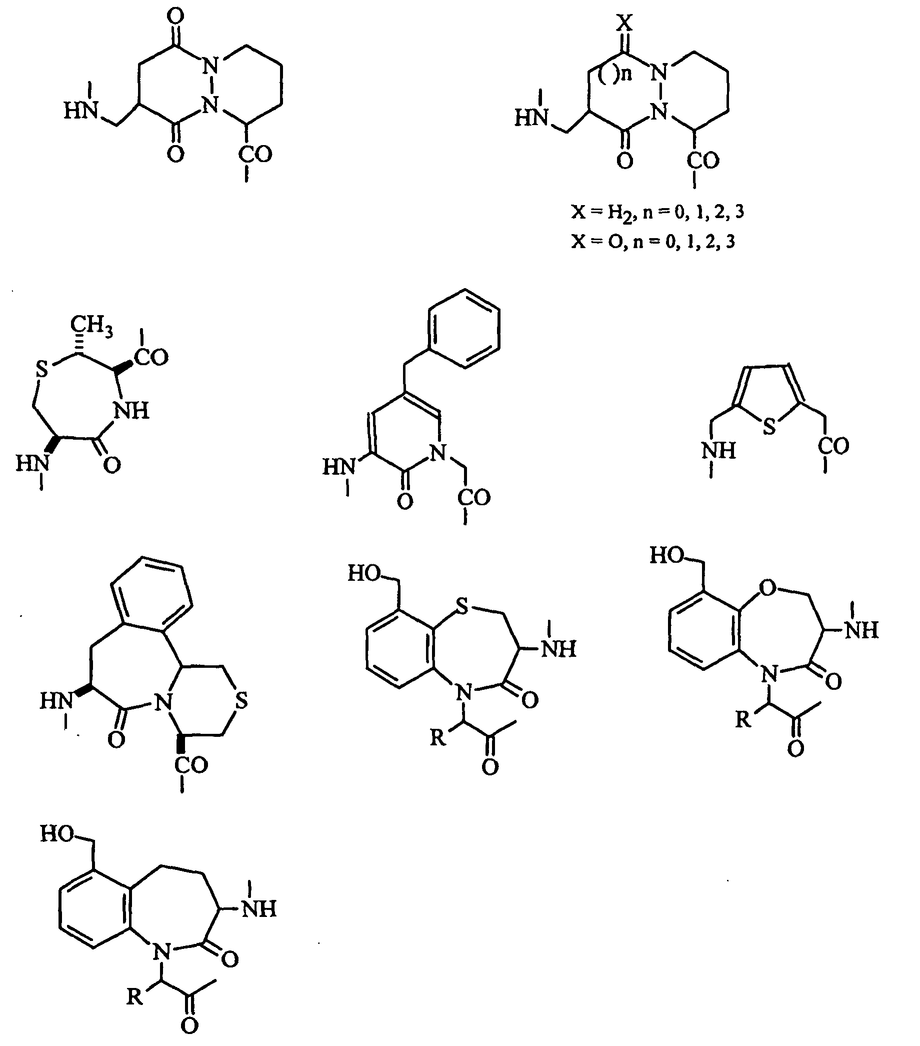 worin:
- die R, und gegebenenfalls die R₄, unabhängig voneinander aus den die Seitenketten von α-Aminosäuren bildenden Gruppen ausgewählt sind;
- R₅ und R₆ unabhängig voneinander für H, CH₃- oder C₆H₅-CH₂- stehen;
- R₇ für H oder Phenyl steht;
- R₈ für H, CH₃-, C₂H₅- oder C₆H₅-CH₂- steht;
- die Substituenten X und Z für jede sie enthaltende Verbindung spezifisch definiert sind;
- Me für eine Methylgruppe steht; und
- n für die Anzahl an Kohlenstoffatomen steht und 0, 1, 2 oder 3 ist.

6. Oligomer nach Anspruch 5, **dadurch gekennzeichnet, dass** die R, und gegebenenfalls die R₄, unabhängig voneinander aus H, CH₃-, (CH₃)₂CH-, CH₃(CH₂)₃- oder C₆H₅-CH₂- ausgewählt sind.

7. Verfahren zur Herstellung eines Oligomers nach Anspruch 1, **dadurch gekennzeichnet, dass** es darin besteht, eine Polymerisation von zumindest einer Aminosäure durchzuführen, die ein nichtpeptidisches Mimetikum eines Dipeptid- oder Tripeptidfragments darstellt, das eine erzwungene Struktur aufweist, ein Induktor von β-Faltung ist und der Formel NHR'-A-CO-OH (II) entspricht, worin R' für ein Wasserstoffatom steht oder R' eine monozyklische Gruppe oder eine, gegebenenfalls mit dem Atom N und der Gruppe A kondensierte, polyzyklische Gruppe bildet.

8. Verfahren nach Anspruch 7, **dadurch gekennzeichnet, dass** A für eine aromatische oder nichtaromatische heterozyklische, monozyklische oder, gegebenenfalls kondensierte, polyzyklische Gruppe steht.

9. Verfahren nach Anspruch 7, **dadurch gekennzeichnet, dass** die Gruppe -NR'-A-CO- für eine aromatische oder nichtaromatische heterozyklische, monozyklische oder, gegebenenfalls kondensierte, polyzyklische Gruppe steht.

10. Verfahren nach Anspruch 7, **dadurch gekennzeichnet, dass** die Gruppe -NR'-A-CO- ein asymmetrisches Zentrum umfasst.

11. Verfahren nach Anspruch 7, **dadurch gekennzeichnet, dass** die Polymerisation in Lösung durchgeführt wird.

12. Verfahren nach Anspruch 7, **dadurch gekennzeichnet, dass** die Polymerisation nach einem Festphasen-Peptidsyntheseverfahren durchgeführt wird.

13. Verfahren nach Anspruch 7, **dadurch gekennzeichnet, dass** die Verbindung (II) aus den folgenden Verbindungen ausgewählt ist, worin:
- die R, und gegebenenfalls die R₄, unabhängig voneinander aus den die Seitenketten von α-Aminosäuren bildenden Gruppen ausgewählt sind;
- R₅ und R₆ unabhängig voneinander für H, CH₃- oder C₆H₅-CH₂- stehen;
- R₇ für H oder Phenyl steht;
- R₈ für H, CH₃-, C₂H₅- oder C₆H₅-CH₂- steht;
- die Substituenten X und Z für jede sie enthaltende Verbindung spezifisch definiert sind;
- Me für eine Methylgruppe steht; und
- n für die Anzahl an Kohlenstoffatomen steht und 0, 1, 2 oder 3 ist;

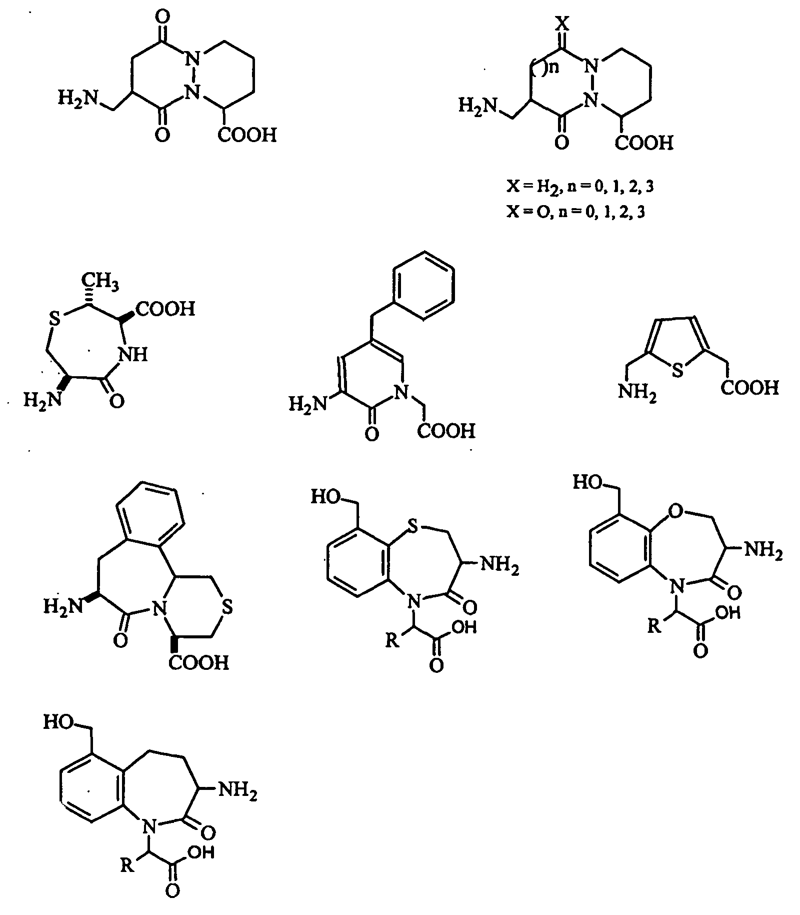

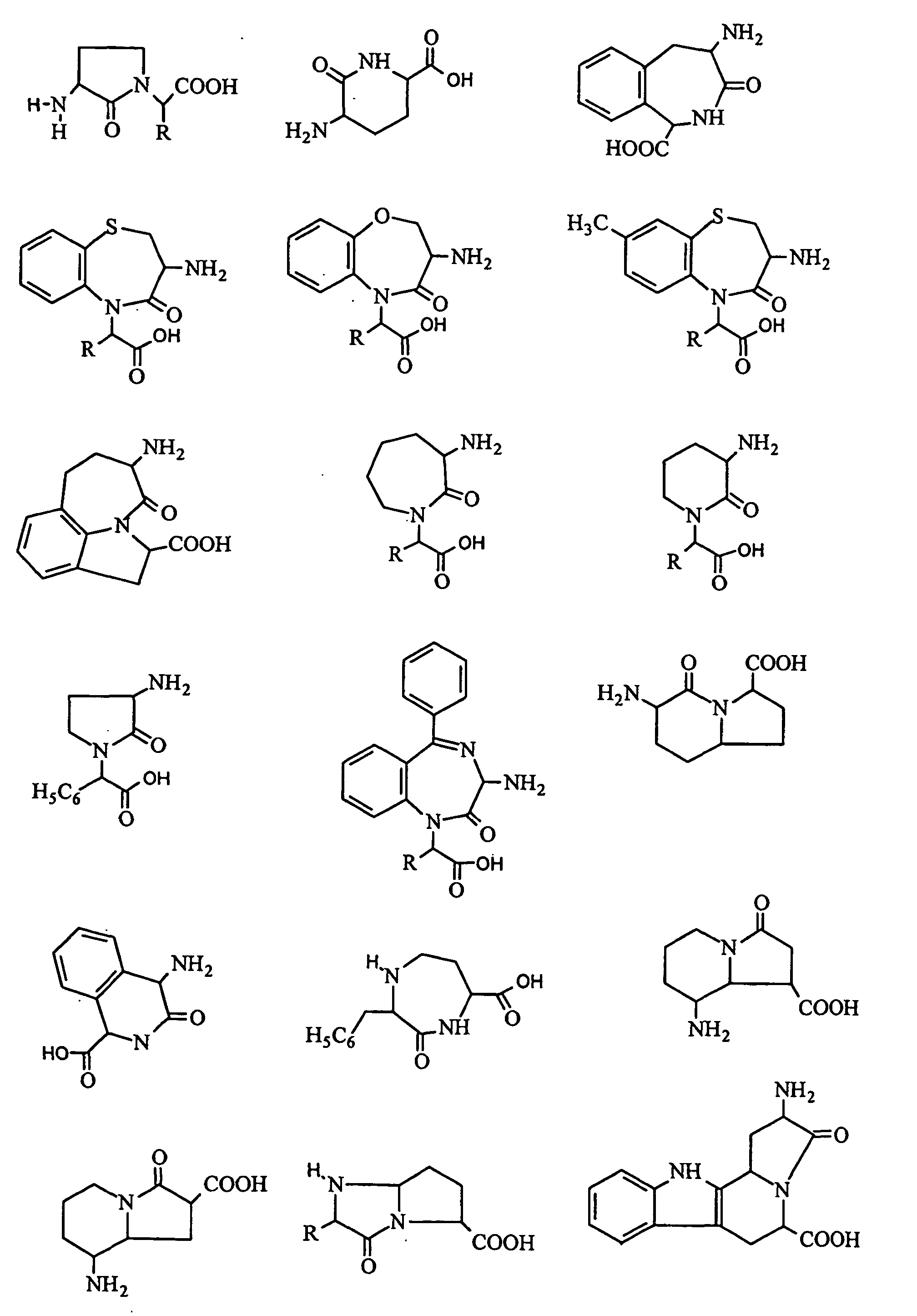

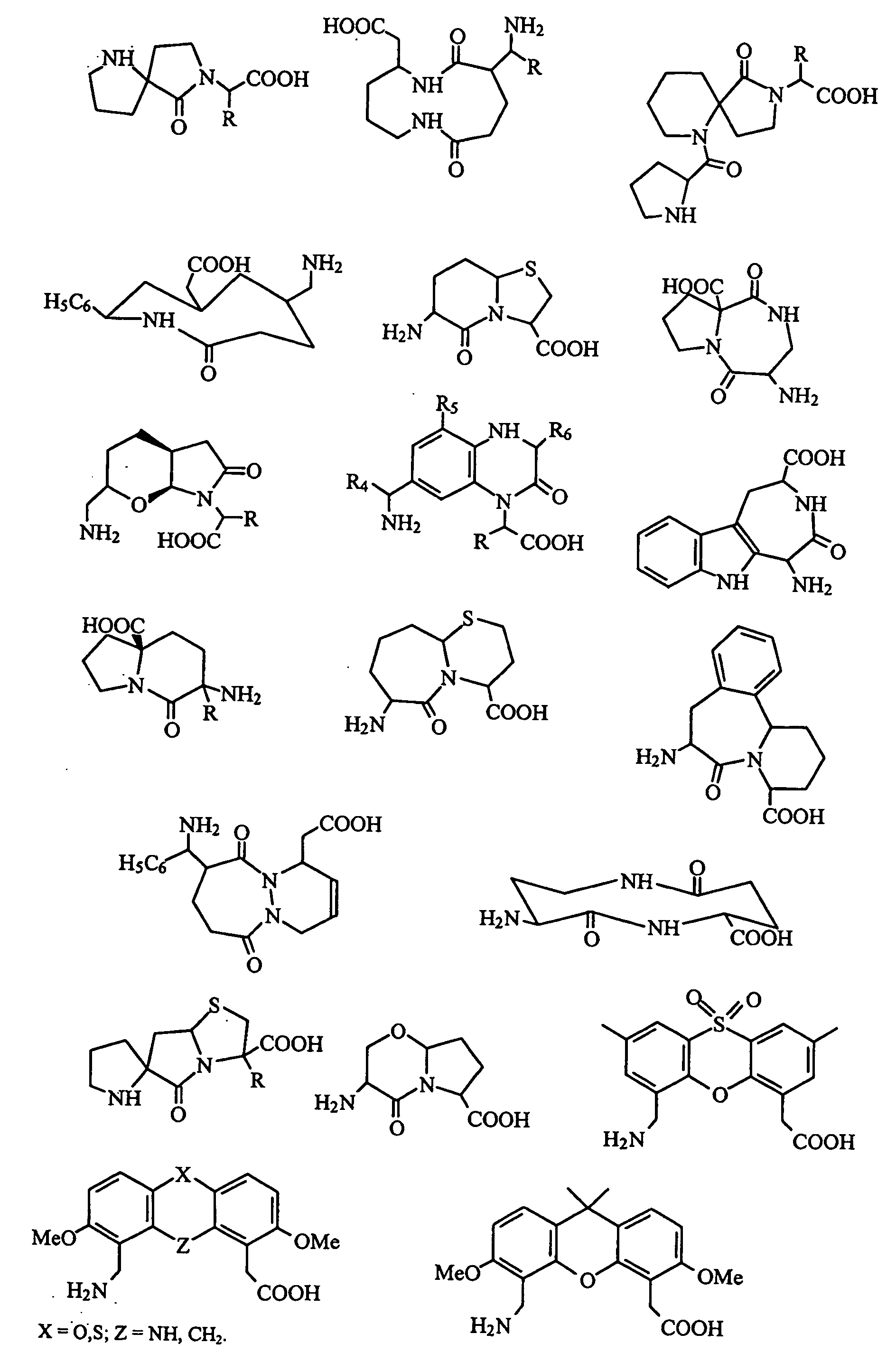

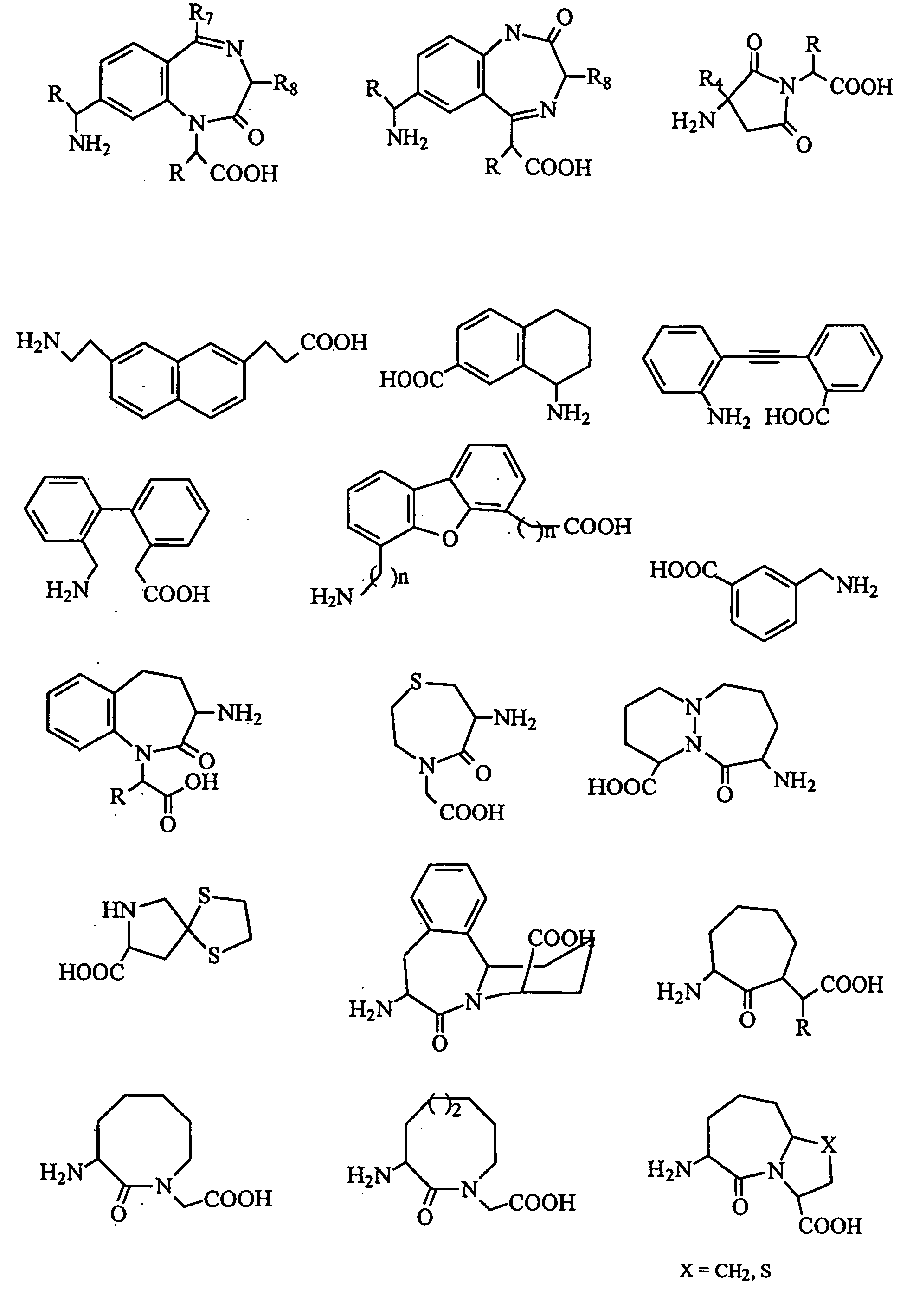

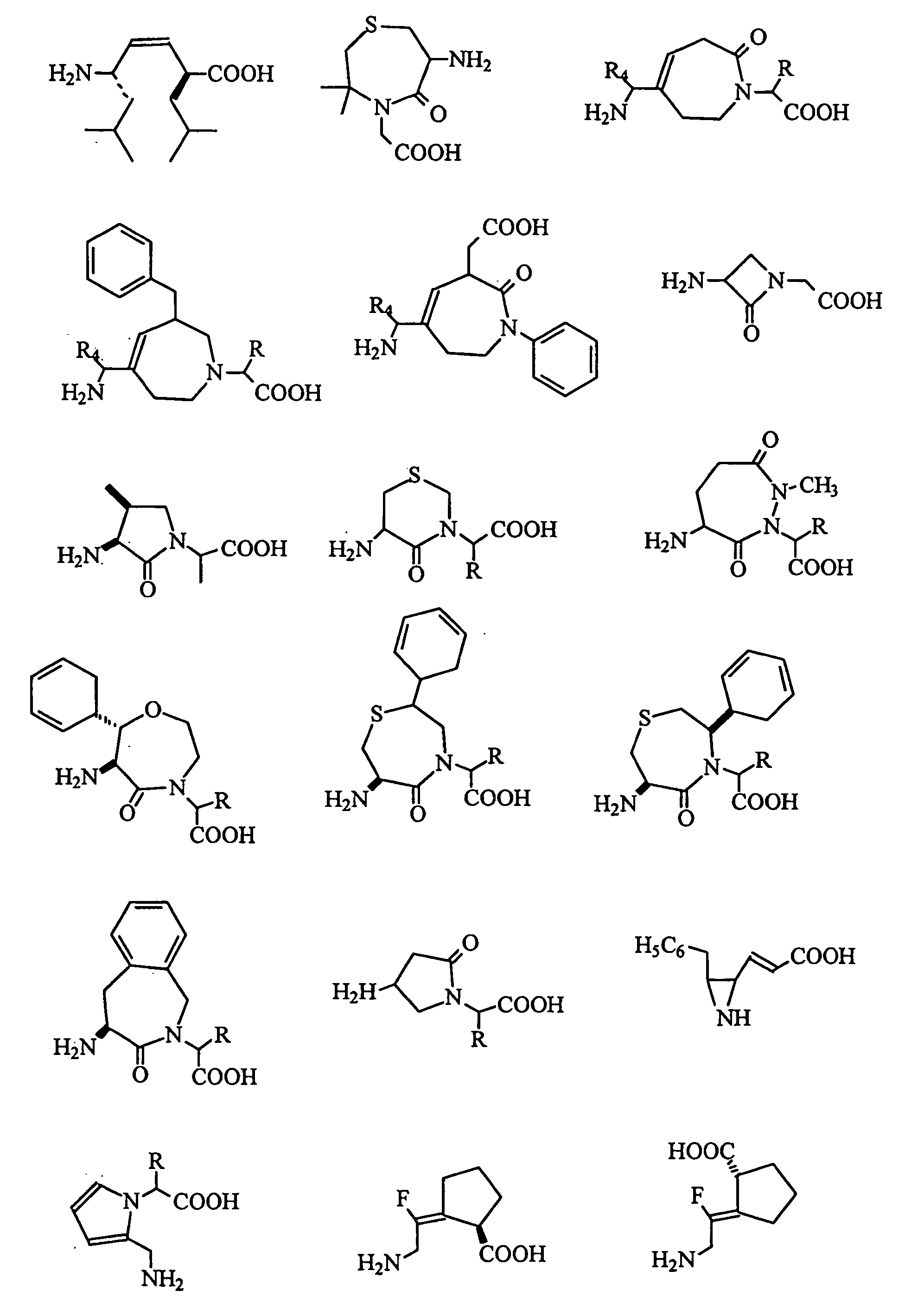

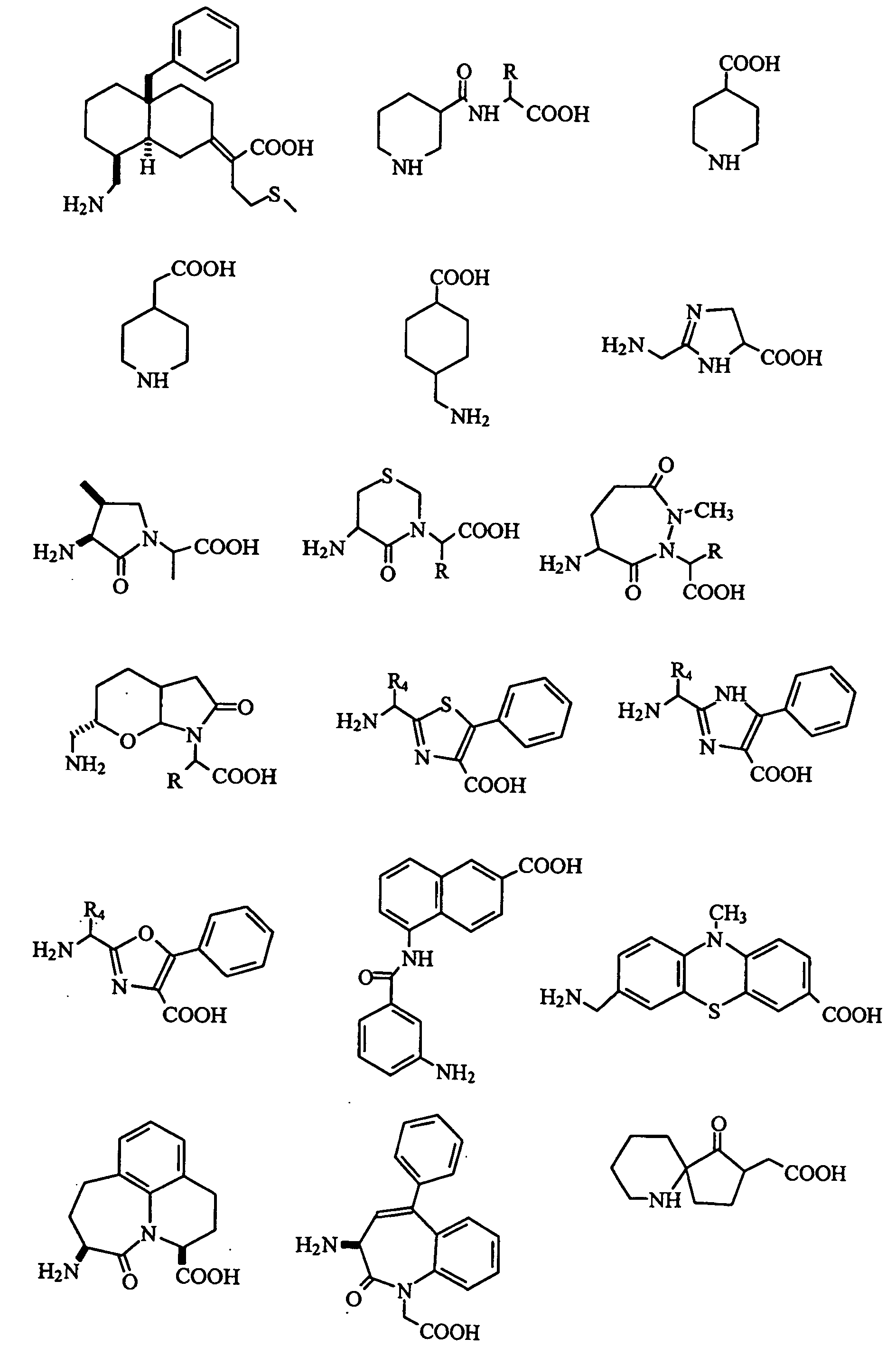

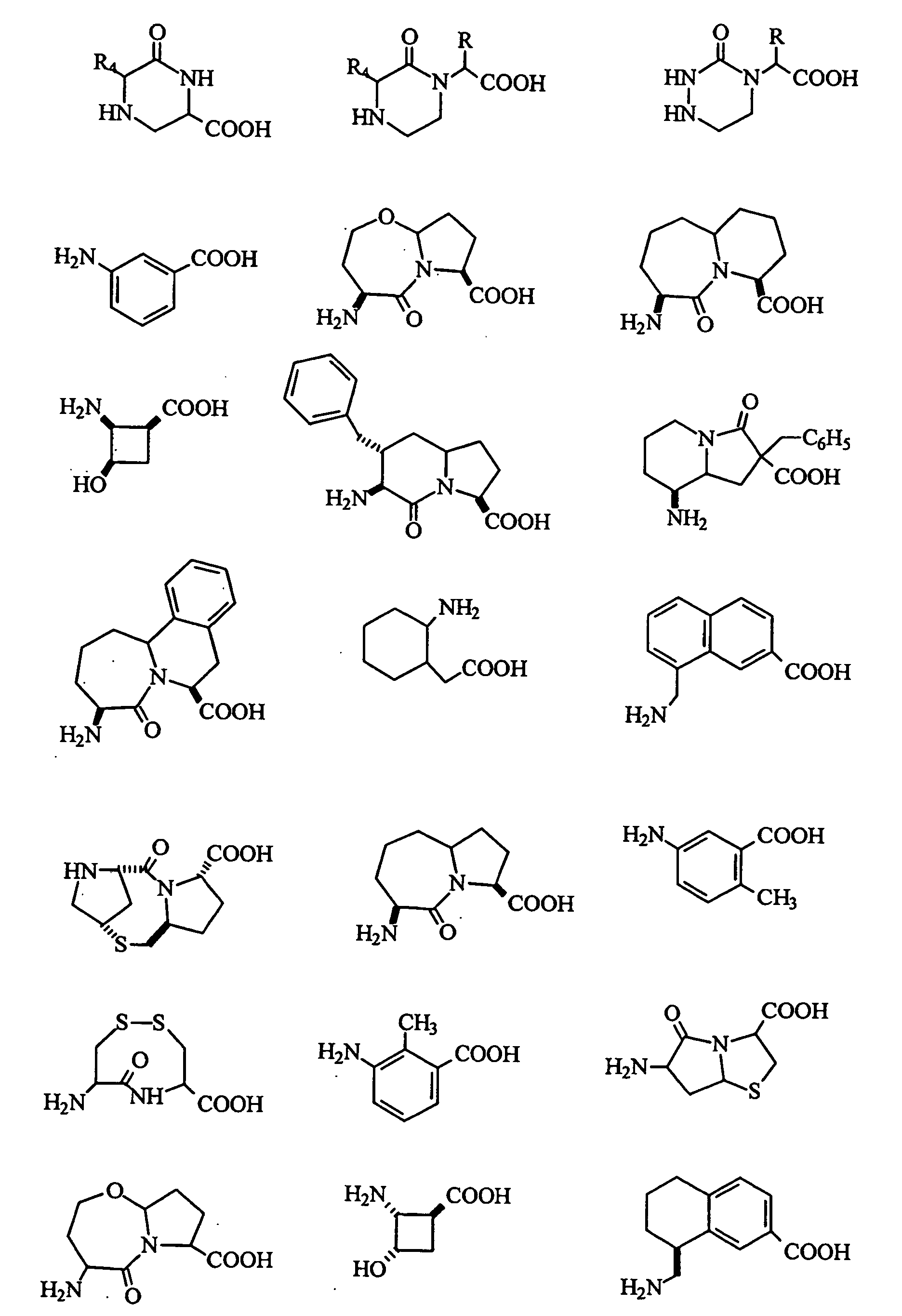

14. Verfahren nach Anspruch 12, **dadurch gekennzeichnet, dass**:
a) ein Harzträger, der Amin-Substituenten trägt, mit einer Verbindung H-NR'-A-CO-OH (II') funktionalisiert wird, die der für (II) angegebenen Definition entspricht und in der die Aminogruppe zuvor geschützt wurde;
b) das so auf den Harzträger fixierte Fragment ausgehend vom C-terminalen Ende in Richtung des N-terminalen Endes durch aufeinander folgende (n-2) Kupplungsreaktionen von Monomer (II) verlängert wird, wobei das Monomer (II) im Überschuss eingesetzt wird und verschiedene Monomere (II) in den aufeinander folgenden Kupplungsschritten eingesetzt werden können;
c) eine letzte Kupplungsreaktion eines an seiner N-terminalen Funktionalität mit einer Gruppe R₁ geschützten Monomers durchgeführt wird, worin R₁ unter jenen Bedingungen, unter denen das Oligomer vom Träger zu trennen ist, stabil ist;
d) das Oligomer vom Harzträger getrennt wird.

15. Verfahren zur Herstellung eines künstlichen Proteins oder eines künstlichen Polypeptids, das zu einem natürlichen Protein oder einem natürlichen Polypeptid analog ist, umfassend die Durchführung von Festphasen-Peptidsynthese-Kupplungsreaktionen, **dadurch gekennzeichnet, dass** in der Abfolge der Kupplungsreaktionen von α-Aminosäuren, die das natürliche Protein oder das natürliche Polypeptid bilden, eine oder mehrere α-Aminosäuresequenzen durch ein Oligomer (I) oder (I') mit einer Länge, die jener der ersetzten α-Aminosäuresequenz entspricht, ersetzt werden.

16. Künstliches Protein oder künstliches Polypeptid, das zu einem bestimmten natürlichen Protein oder einem bestimmten natürlichen Polypeptid analog ist, umfassend ein oder mehrere strukturierende Fragmente und ein oder mehrere Peptidfragmente, **dadurch gekennzeichnet, dass** das oder die Peptidfragmente mit jenen des entsprechenden natürlichen Proteins oder natürlichen Polypeptids identisch sind, und dadurch, dass das oder die strukturierenden Fragmente aus einem Fragment eines Oligomers (I) oder (I') bestehen, das eine Länge aufweist, die mit jener des strukturierenden Teils in der α-Helix des natürlichen Proteins oder des natürlichen Polypeptids im Wesentlichen identisch ist.

17. Protein, das zu hCRF (menschlichem Corticotropin freisetzendem Faktor) analog ist, **dadurch gekennzeichnet, dass** es einer der folgenden Formeln entspricht:
H-Ser-Glu-Glu-Pro-Pro-(DBT)₈-Lys-Leu-Met-Glu-Ile-Ile-NH₂
oder
H-Ser-Glu-Glu-Pro-Pro-(DBT)₉-Leu-Met-Glu-Ile-Ile-NH₂
worin DBT ein von (3S)-[Amino]-5-(carbonylmethyl)-2,3-dihydro-1,5-benzothiazepin-4(5H)-on abgeleitetes Fragment ist.

18. Protein, das zu hCRF (menschlichem Corticotropin freisetzendem Faktor) analog ist, **dadurch gekennzeichnet, dass** es folgender Formel entspricht:
H-Ser-Glu-Glu-Pro-Pro-(A₁)₂₀-Arg-Lys-Leu-Met-Glu-Ile-Ile-NH₂
worin A₁ eine von 3-(S)-Amino-1-carbonylmethylpyrrolidin-2-on abgeleitete Gruppe ist und durch die Formel 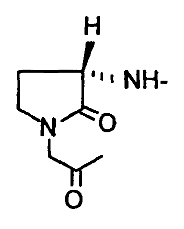 dargestellt ist.

## Claims

1. An oligomer, represented by one of the general formulae:
R₁-(NR'-A-CO)ₙ-OR₂ (I)
or
R₁-(NR'-A-CO)ₙ-NR'₂R"₂ (I')
in which:
- the unit -NR'-A-CO- represents a β-turn inducing nonpeptide constrained mimetic of a dipeptide or tripeptide fragment, wherein A represents a heterocyclic group which may or may not be aromatic, and which is a monocyclic group or an optionally condensed polycyclic group, or the group -NR'-A-CO- represents a heterocyclic group which may or may not be aromatic, and which is a monocyclic group or an optionally condensed polycyclic group ;
- R₁ represents an acyl group R₃-CO- or a group R₃-O-CO- in which R₃ represents a benzyl group, a tert-butyl group or a 9-fluorenylmethyl group;
- R₂ represents H, an alkyl group or a benzyl group;
- R'₂ and R"₂ represent, independently of one another, H, an alkyl group or a benzyl group;
- R' represents a hydrogen atom or else R' forms a monocyclic group or an optionally condensed polycyclic group with the N atom and the group A;
- n is between 2 and 40.

2. The oligomer as claimed in claim 1, **characterized in that** the group -NR'-A-CO- comprises an asymmetrical center which may have an R configuration or an S configuration.

3. The oligomer as claimed in claim 1, **characterized in that** the recurring units -NR'-A-CO- are all identical.

4. The oligomer as claimed in claim 1, **characterized in that** the recurring units -NR'-A-CO- are different.

5. The oligomer as claimed in claim 1, **characterized in that** the recurring units are chosen from the following groups: 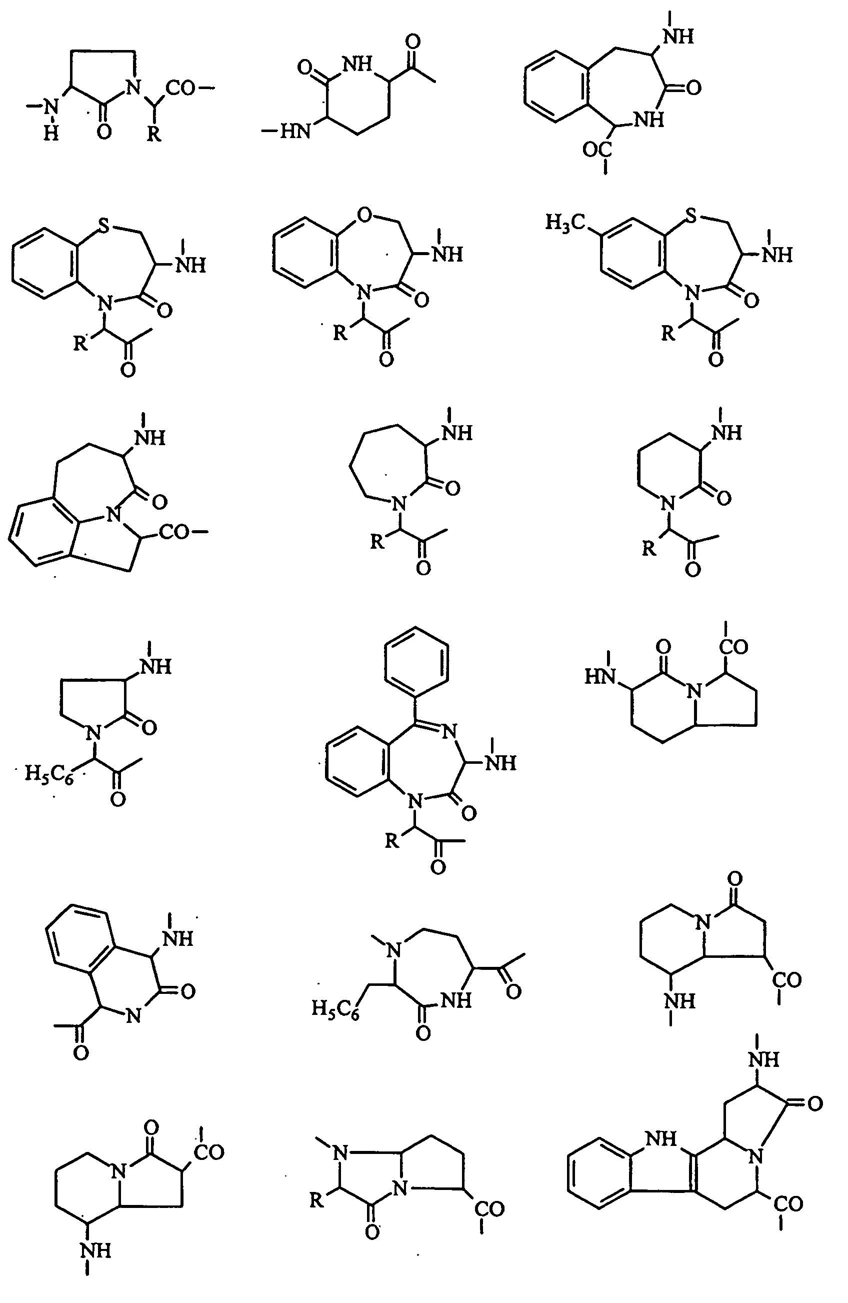 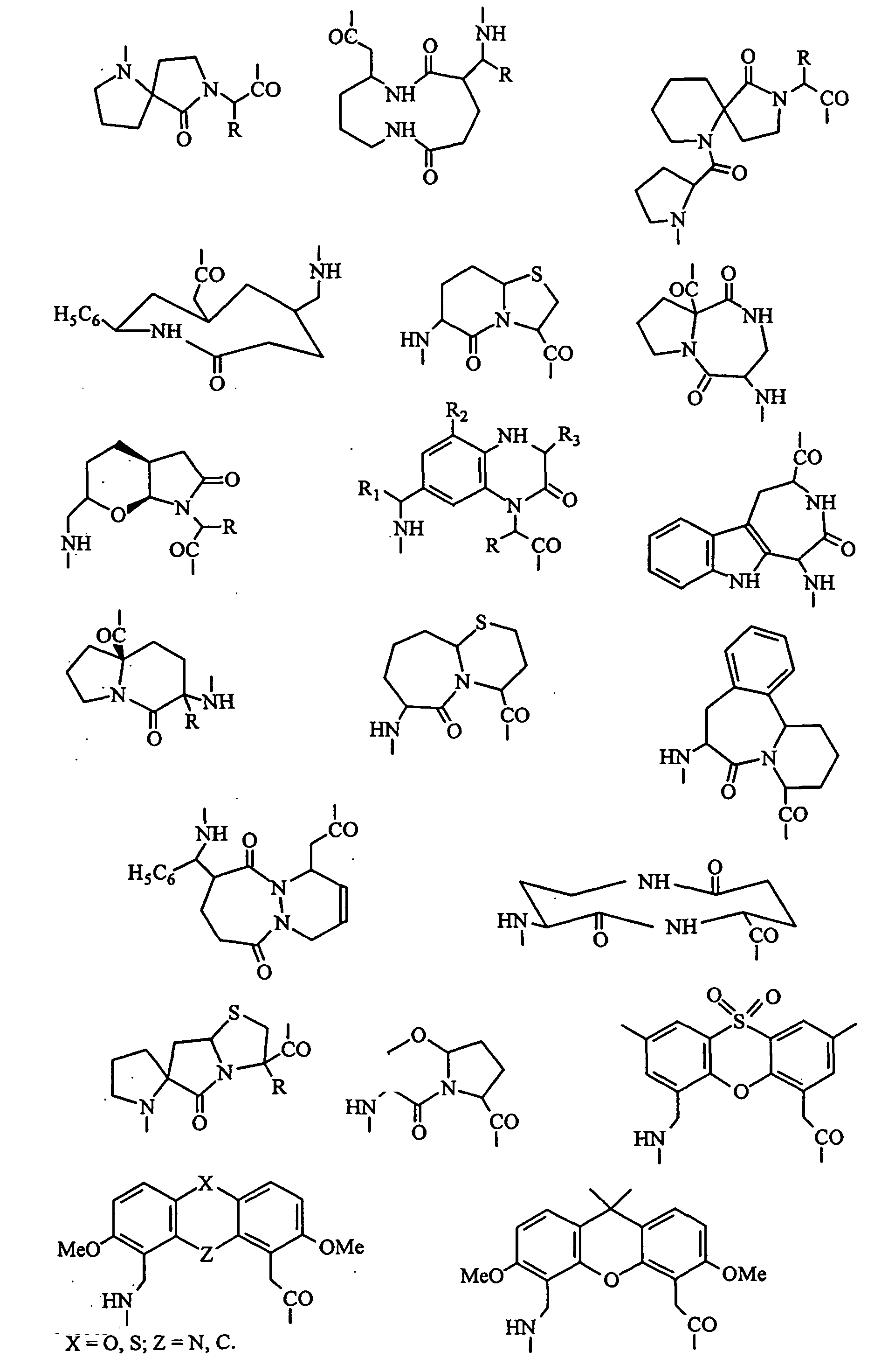 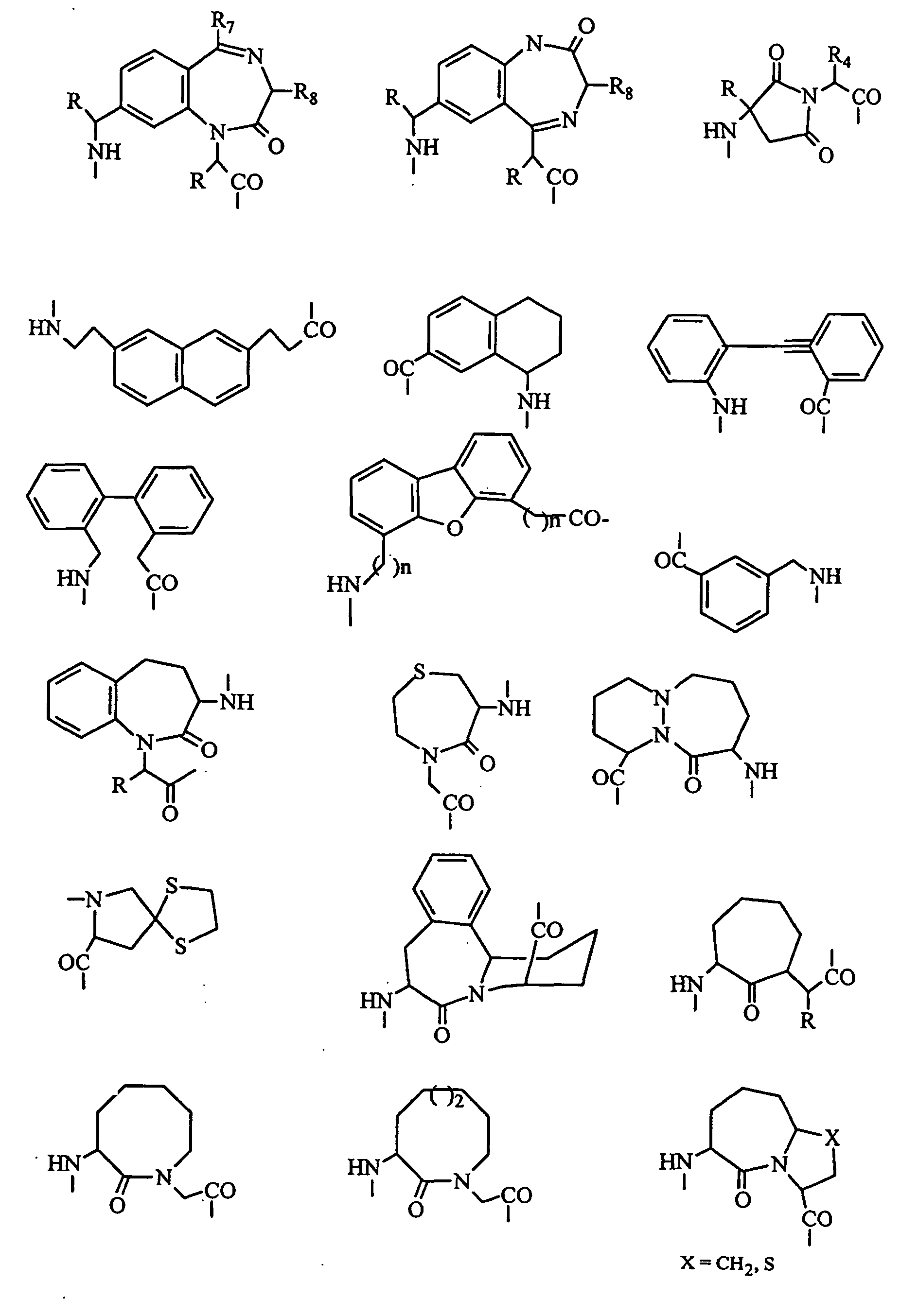 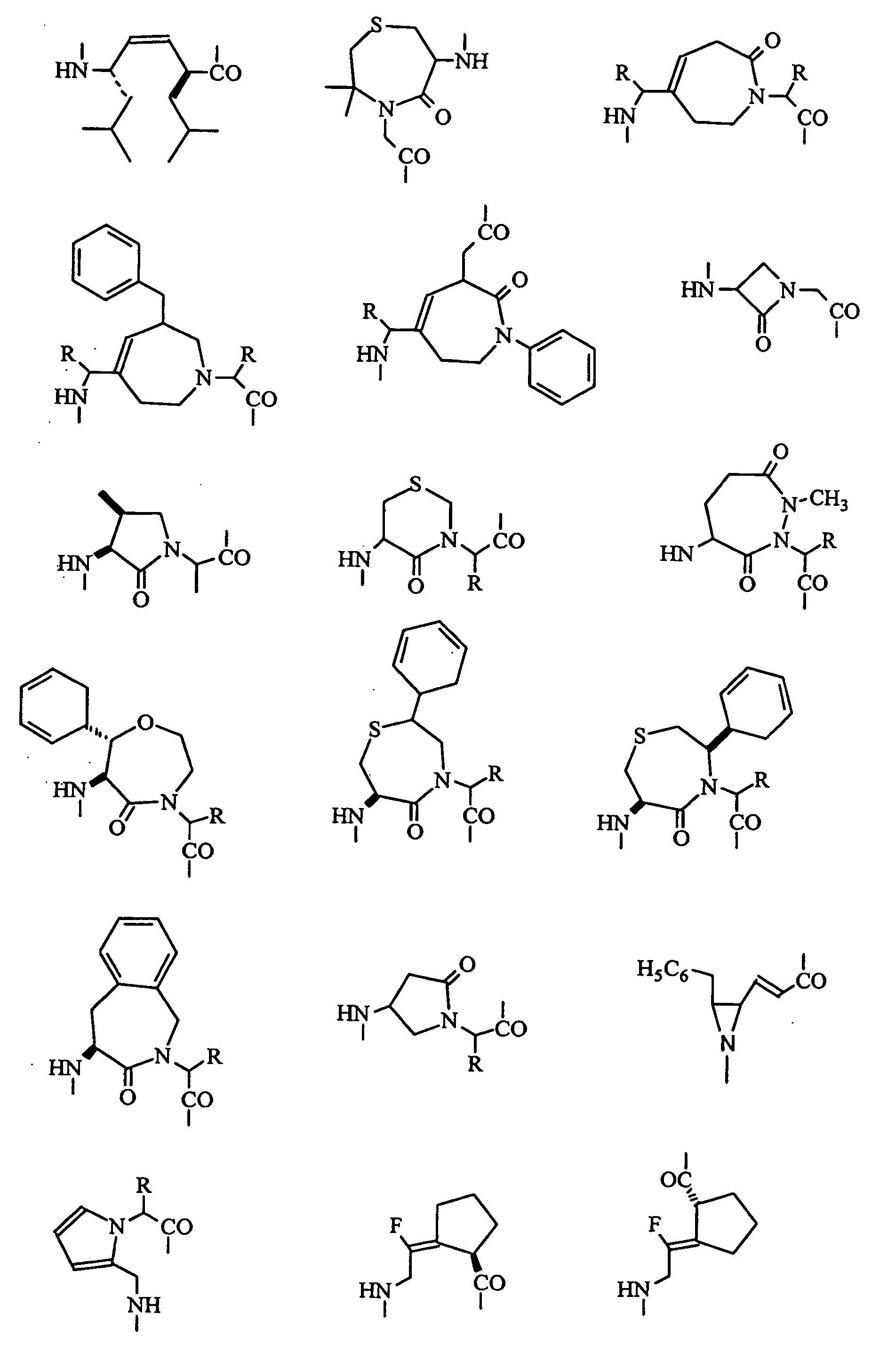 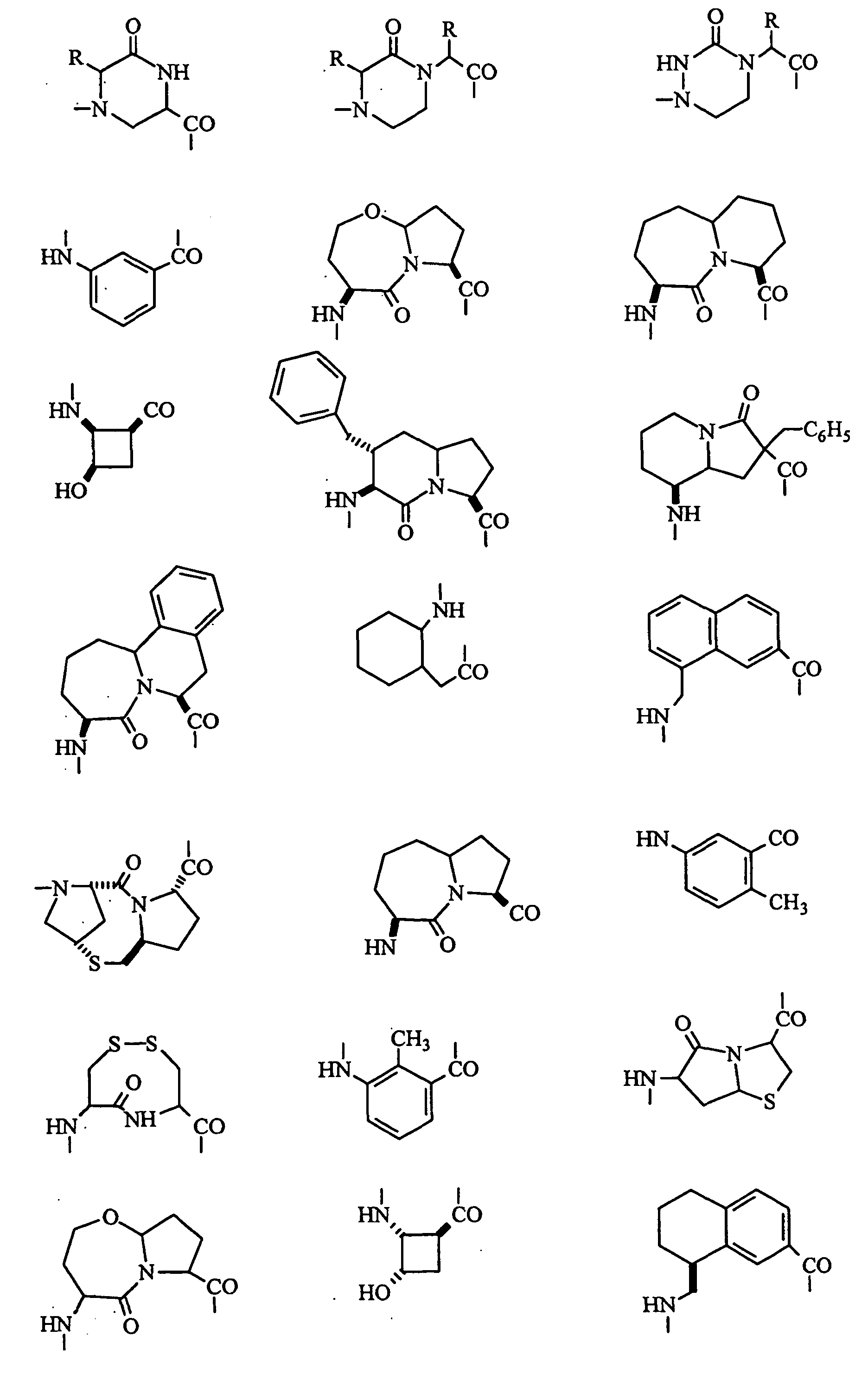 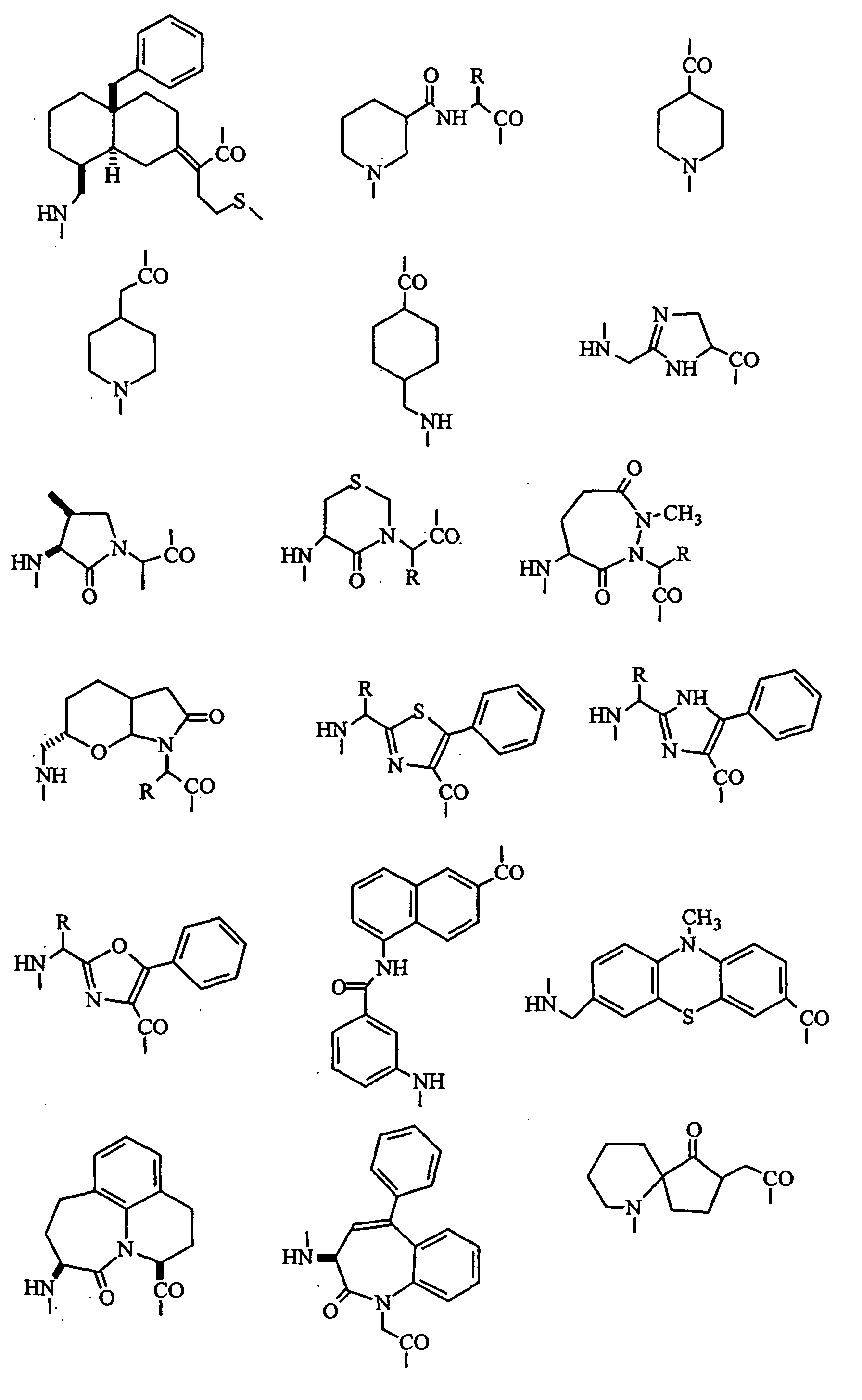 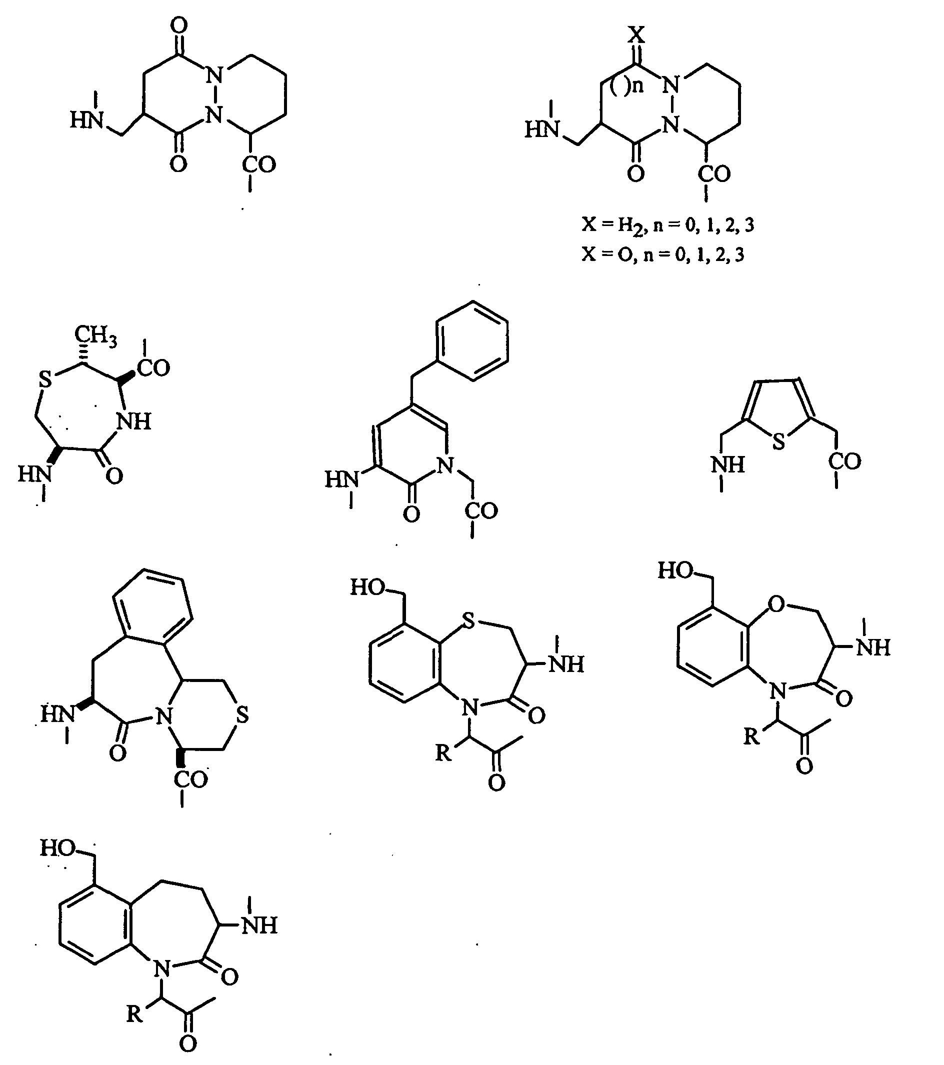 in which:
- R, and where appropriate R₄, are chosen, independently of one another, from the groups constituting the side chains of α-amino acids;
- R₅ and R₆ represent, independently of one another, H, CH₃- or C₆H₅-CH₂-;
- R₇ represents H or a phenyl;
- R₈ represents H, CH₃-, C₂H₅- or C₆H₅-CH₂-;
- the substituents X and Z are defined specifically for each compound which contains them, and
- Me represents a methyl group ; and
- n is the number of carbon atoms, equal to 0, 1 or2.

6. The oligomer as claimed in claim 5, **characterized in that** R, and where appropriate R₄ are chosen, independently of one another, from H, CH₃-, (CH₃)₂CH-, CH₃-(CH₂)₃- or C₆H₅-CH₂-.

7. A method for preparing an oligomer as claimed in claim 1, **characterized in that** it consists in carrying out a polymerization of at least one amino acid constituting a β-turn inducing nonpeptide constrained mimetic of a dipeptide or of a tripeptide and corresponding to the formula NHR'-A-CO-OH (II), in which R' represents a hydrogen atom or else R' forms a monocyclic group or an optionally condensed polycyclic group with the N atom and the group A.

8. The method as claimed in claim 7, **characterized in that** A represents a heterocyclic group which may or may not be aromatic, and which is a monocyclic group or an optionally condensed polycyclic group.

9. The method as claimed in claim 7, **characterized in that** the group -NR'-A-CO- represents a heterocyclic group which may or not be aromatic, and which is a monocyclic group or an optionally condensed polycyclic group.

10. The method as claimed in claim 7, **characterized in that** the group -NR'-A-CO- comprises an asymmetrical center.

11. The method as claimed in claim 7, **characterized in that** the polymerization is carried out in solution.

12. The method as claimed in claim 7, **characterized in that** the polymerization is carried out in solid phase, according to a peptide synthesis strategy.

13. The method as claimed in claim 7, **characterized in that** the compound (II) is chosen from the following compounds, in which:
- R, and where appropriate R₄, are chosen, independently of one another, from the groups constituting the side chains of α-amino acids;
- R₅ and R₆ represent, independently of one another, H, CH₃- or C₆H₅-CH₂-;
- R₇ represents H or a phenyl;
- R₈ represents H, CH₃-, C₂H₅- or C₆H₅-CH₂-;
- the substituents X and Z are defined specifically for each compound which contains them, and
- Me represents a methyl group ; and
- n is the number of carbon atoms, equal to 0, 1 or 2 ;

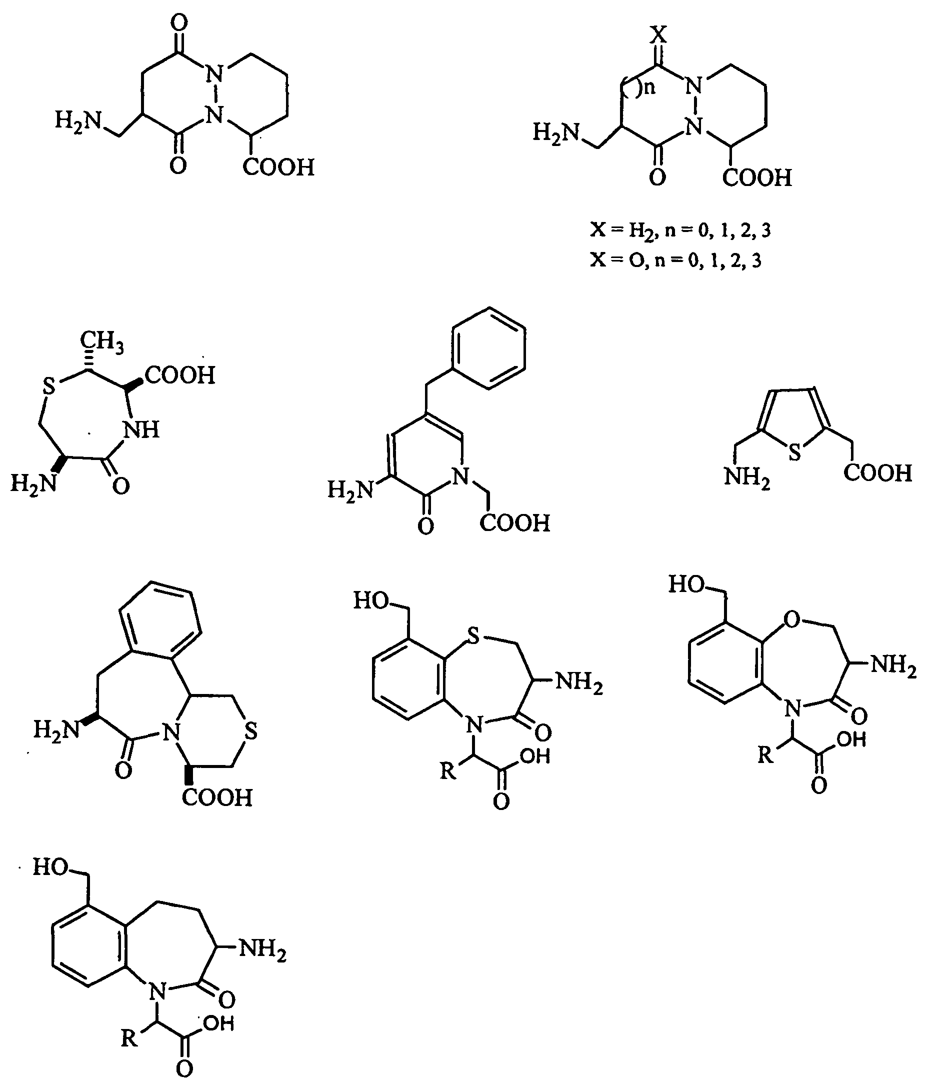

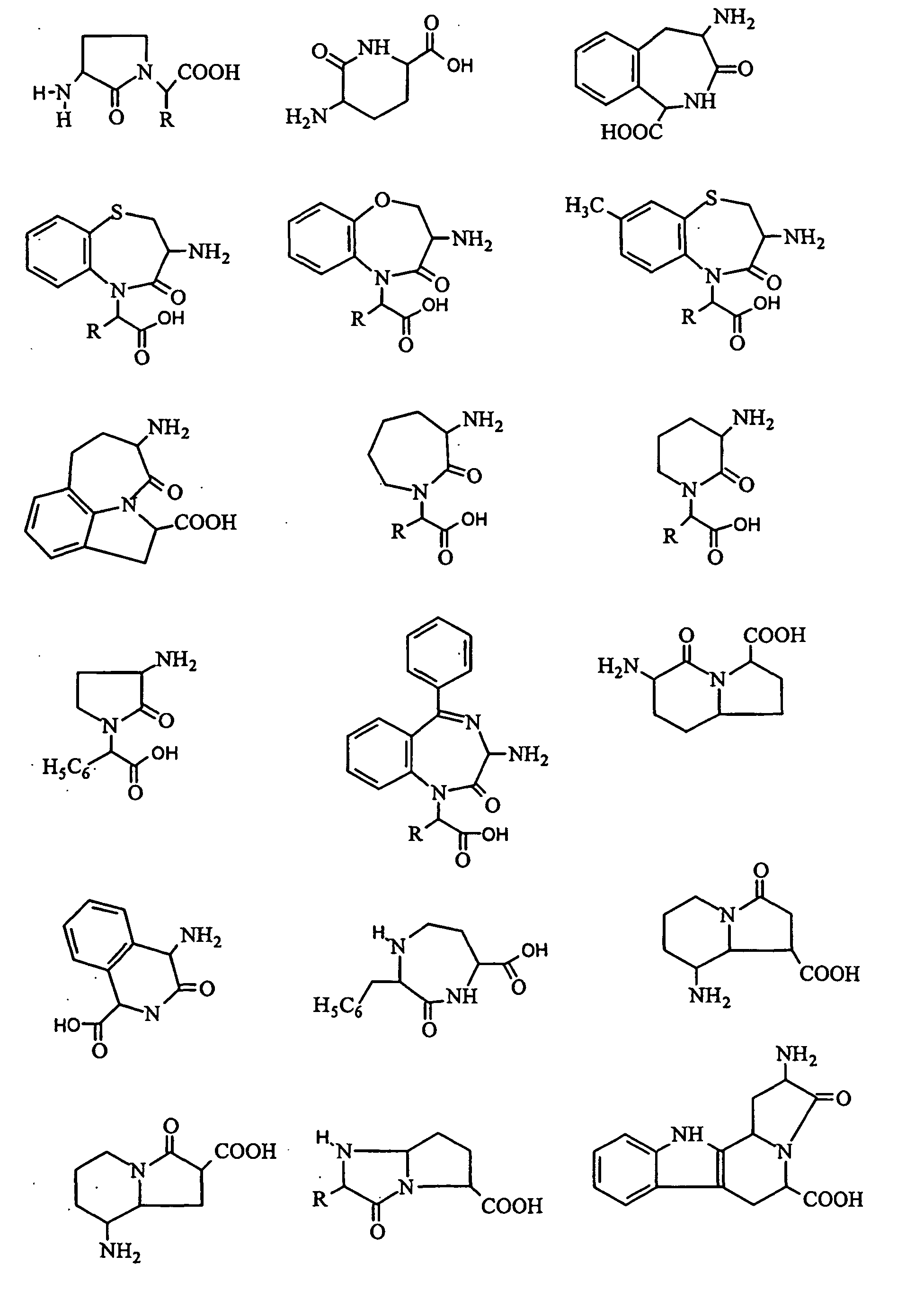

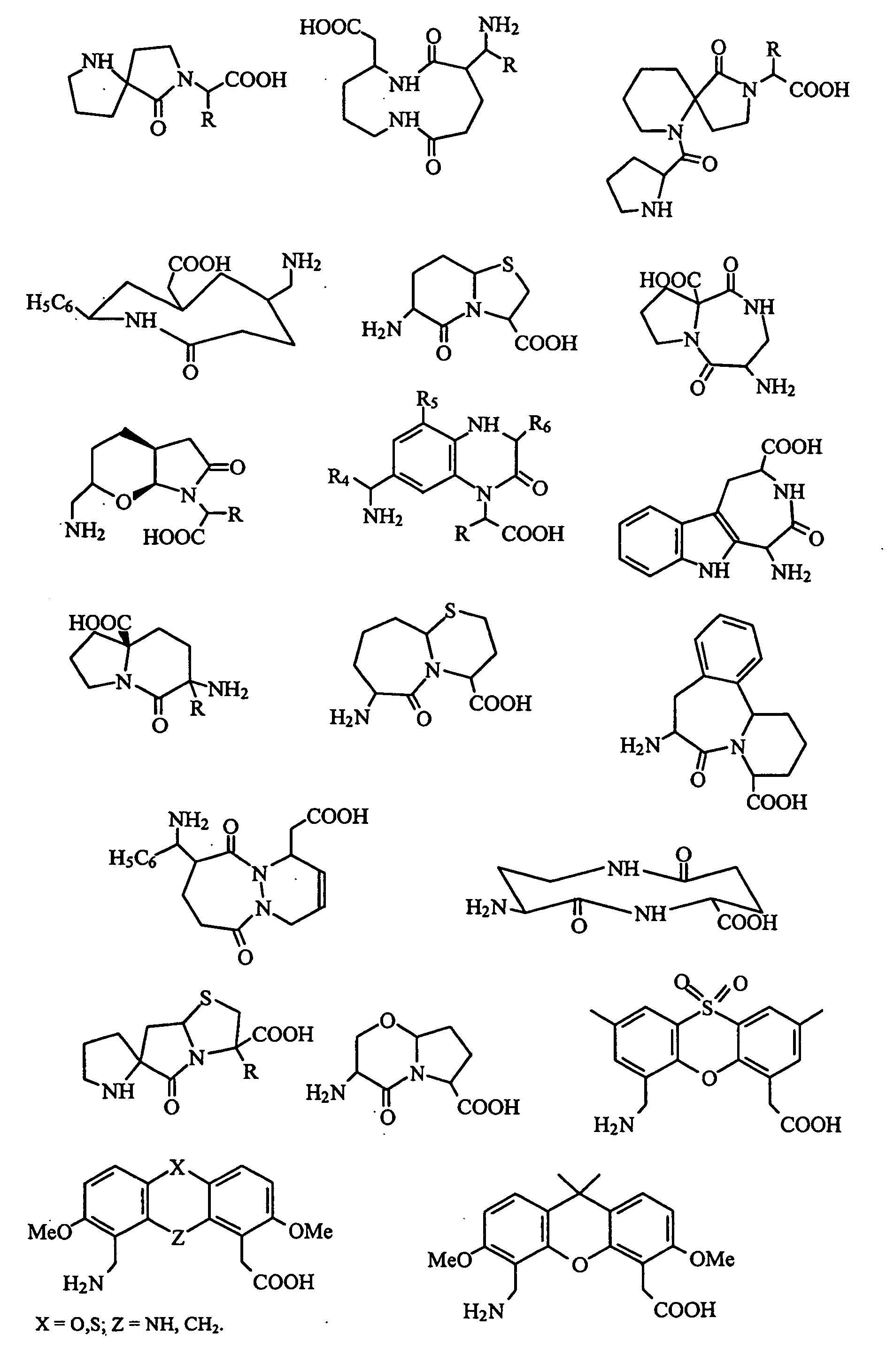

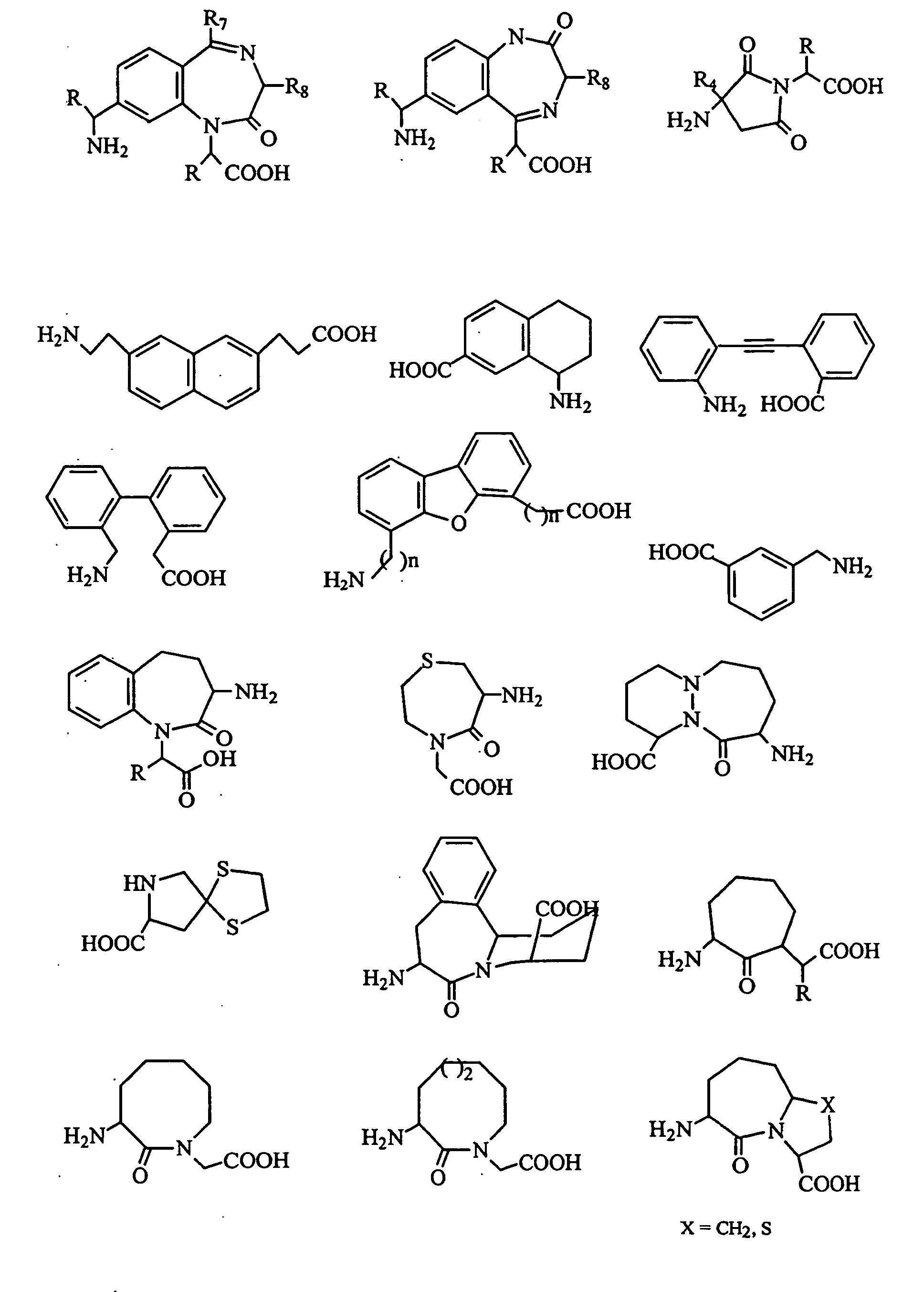

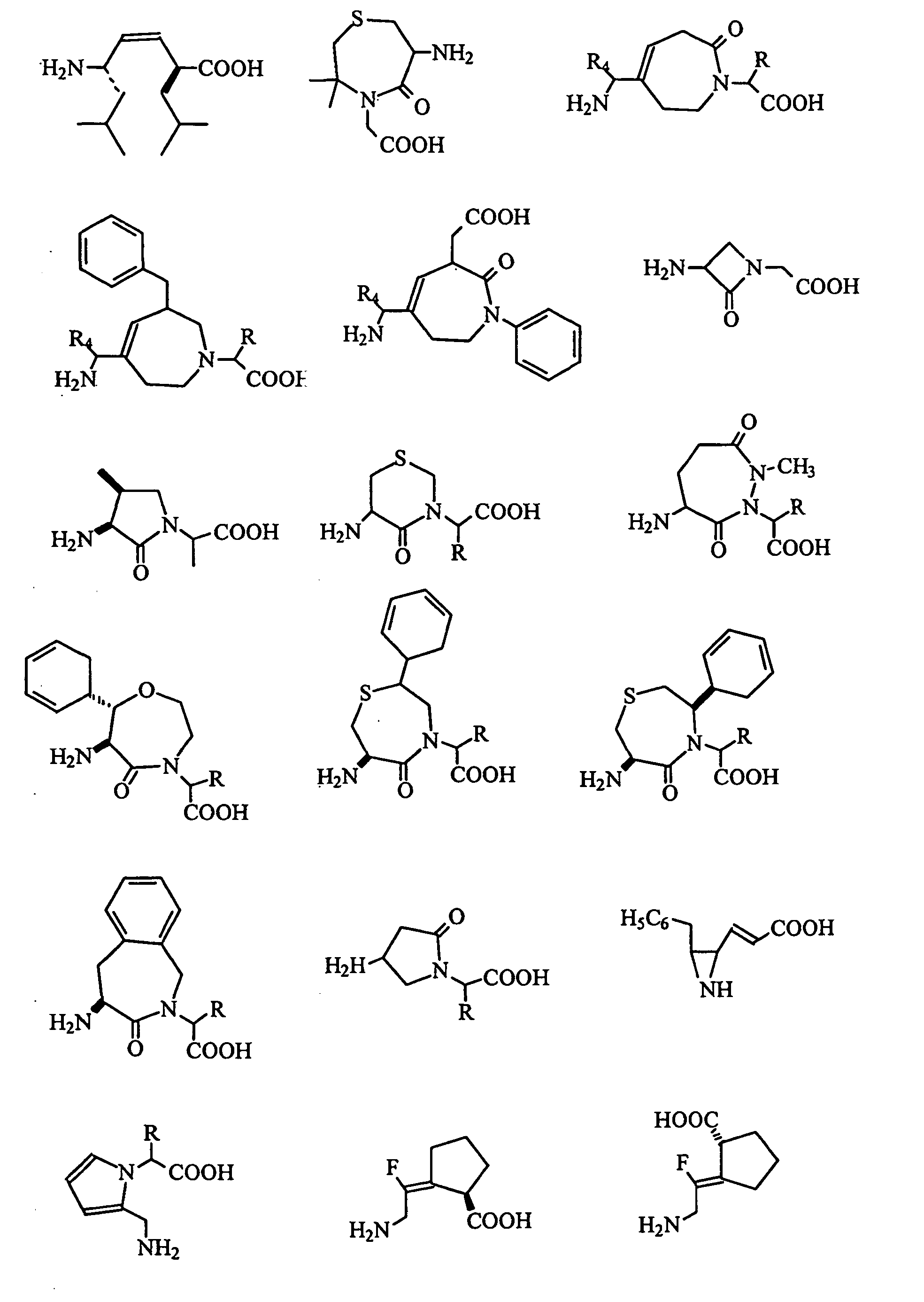

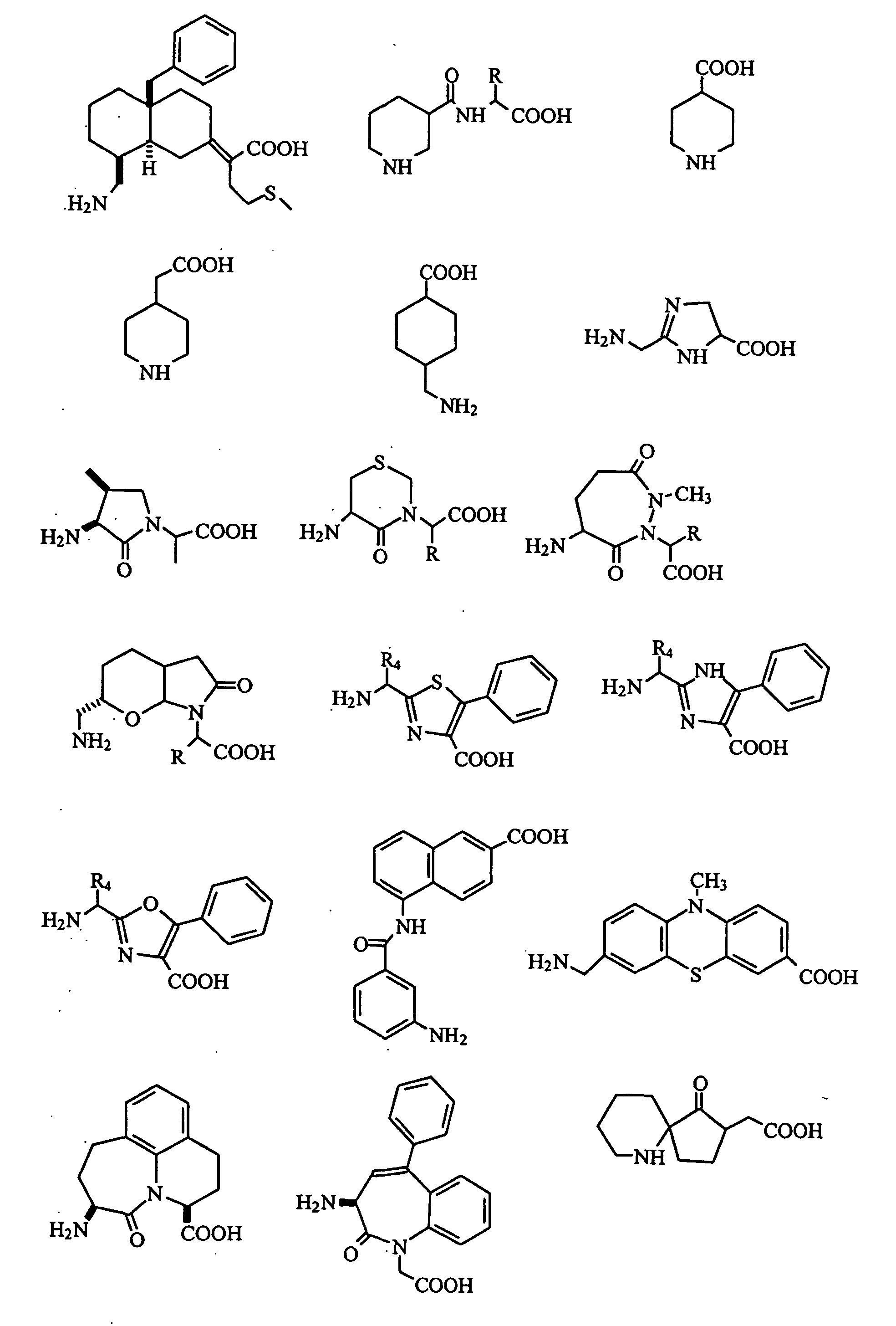

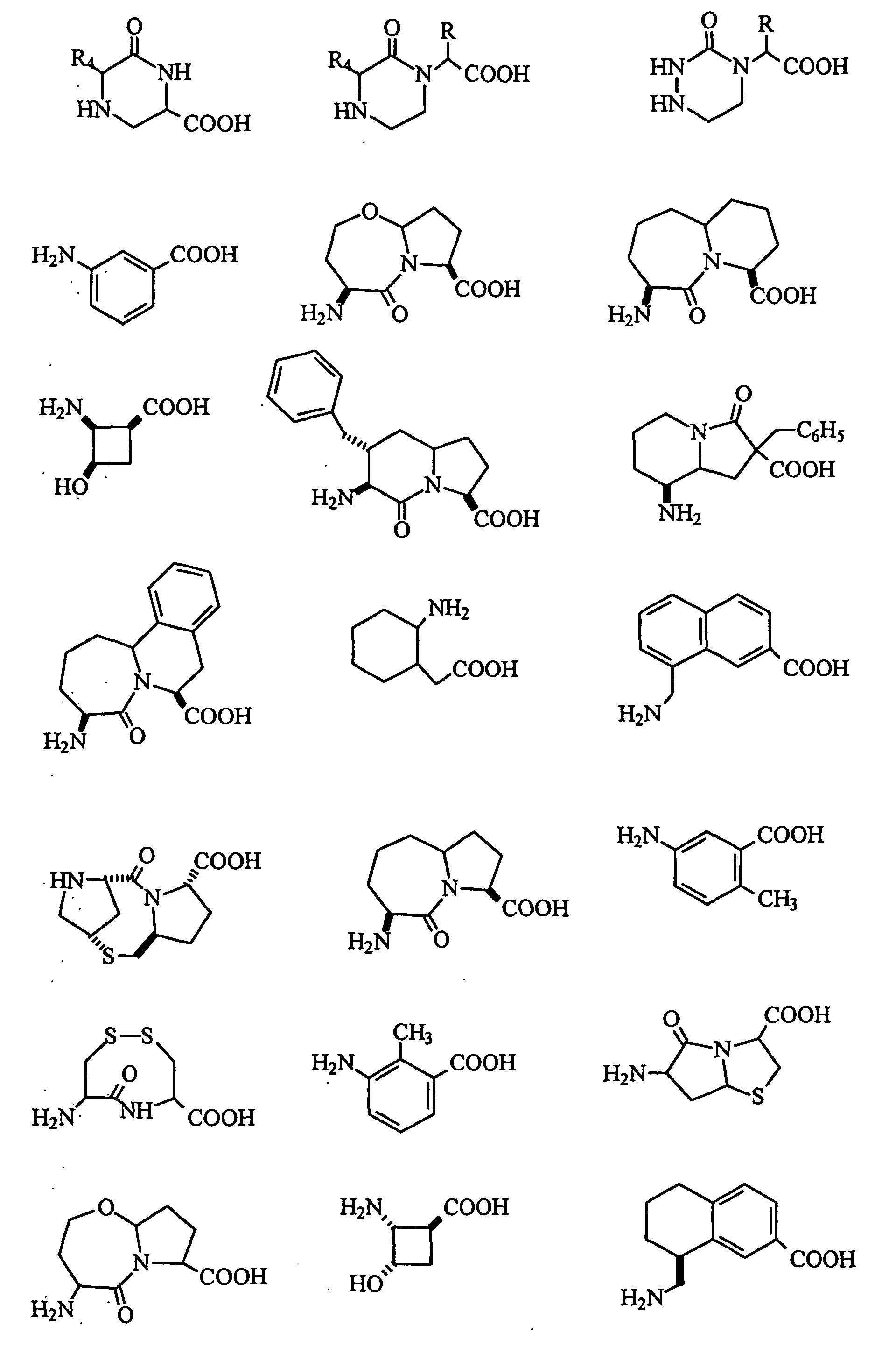

14. The method as claimed in claim 12, **characterized in that**:
a) a support resin carrying amino substituents is functionalized with a compound H-NR'-A-CO-OH (II') which corresponds to the definition given for (II) and in which the amino group has been protected beforehand;
b) the fragment thus attached to the support resin is extended from the C-terminal side to the N-terminal side by (n-2) successive reactions for coupling the monomer (II), said monomer (II) being used in excess, various monomers (II) possibly being used in the successive coupling steps;
c) a final reaction is carried out for coupling a monomer protected on its N-terminal function with a group R₁ which is stable under the conditions under which the oligomer must be separated from the support;
d) the oligomer is separated from the support resin.

15. A method for preparing an artificial protein or an artificial polypeptide which is analogous to a natural protein or a natural polypeptide, consisting in carrying out solid-phase peptide synthesis coupling reactions, **characterized in that**, in the succession of reactions for coupling the α-amino acids constituting the natural polypeptide or protein, one or more α-amino acid sequences are replaced with an oligomer (I) or (I'), the length of which is equivalent to that of the α-amino acid sequence replaced.

16. An artificial polypeptide or protein which is analogous to a given natural polypeptide or protein, comprising one or more structuring fragments and one or more peptide fragments, **characterized in that** the peptide fragment(s) is (are) identical to those of the corresponding natural polypeptide or protein, and **in that** the structuring fragment(s) consist(s) of a fragment of an oligomer (I) or (I'), the length of which is substantially identical to that of the α-helical structuring component of the natural polypeptide or protein.

17. A protein analogous to hCRF (human corticotropin releasing factor), **characterized in that** it corresponds to one of the following formulae:
H-Ser-Glu-Glu-Pro-Pro-(DBT)₈-Lys-Leu-Met-Glu-Ile-Ile-NH₂
or
H-Ser-Glu-Glu-Pro-Pro-(DBT)₉-Leu-Met-Glu-Ile-Ile-NH₂
in which DBT is a fragment derived from (3S)-[amino]-5-(carbonylmethyl)-2,3-dihydro-1,5-benzothiazepin-4(5H)-one.

18. A protein analogous to hCRF (human corticotropin releasing factor), **characterized in that** it corresponds to the following formula:
H-Ser-Glu-Glu-Pro-Pro-(A₁)₂₀-Arg-Lys-Leu-Met-Glu-Ile-Ile-NH₂
in which A₁ is a group derived from is the 3-(S)-amino-1-carbonylmethylpyrrolidin-2-one and represented by the formula
